# EUROPEAN PATENT APPLICATION

(11) **EP 4 483 905 A1**
(43) Date of publication of application: **01.01.2025**
(21) Application number: 23759947.7
(22) Date of filing: 21.02.2023
(51) Int. Cl.: A61K 47/54, A61K 51/04, A61P 35/00, C07D 401/12, C07H 15/26, C07K 5/02

(54) **RADIOLABELED FAP-ALPHA-AFFINITY COMPOUND AND USE THEREOF**

(30) Priority: 22.02.2022 JP 2022026194
(71) Applicant: OSAKA UNIVERSITY, Suita-shi Osaka 565-0871 (JP)
(72) Inventor: SHIRAKAMI, Yoshifumi, Suita-shi, Osaka 565-0871 (JP); KADONAGA, Yuichiro, Suita-shi, Osaka 565-0871 (JP); KANEDA, Kazuko, Suita-shi, Osaka 565-0871 (JP); WATABE, Tadashi, Suita-shi, Osaka 565-0871 (JP); FUKASE, Koichi, Suita-shi, Osaka 565-0871 (JP); SHIMOYAMA, Atsushi, Suita-shi, Osaka 565-0871 (JP); ASO, Ayaka, Suita-shi, Osaka 565-0871 (JP); YOSHIYA, Taku, Ibaraki-shi, Osaka 567-0085 (JP)
(74) Representative: De Clercq & Partners
(86) International application number: PCT/JP2023/006110
(87) International publication number: WO 2023/162946

(57) **Abstract**

It is an object of the present invention to provide drugs that bind specifically to FAPα, are effective in the treatment and diagnosis of tumors or cancers expressing FAPα, for example, in the treatment and diagnosis of solid cancers such as pancreatic cancer, sarcoma, esophageal cancer, lung cancer, breast cancer, prostate cancer, head and neck cancer, ovarian cancer, colon cancer, neuroendocrine tumor, thyroid cancer, uterine cancer, liver cancer and the like (particularly, pancreatic cancer), and have a lower risk of prolonged side effects.

The present invention provides a conjugate comprising a radioactive moiety comprising an aryl group substituted with a radionuclide selected from ²¹¹At, ²¹⁰At, ¹³¹I, ¹²⁵I, ¹²⁴I, ¹²³I, ⁷⁷Br and ⁷⁶Br, and a bioactive moiety having an affinity for fibroblast activation protein α (FAPα).

## Description

### [Technical Field]

The present invention relates to radiolabeled FAPα-affinity compounds useful as therapeutic and/or diagnostic agents for tumors or cancers, and methods for producing the same.

### [Background Art]

In the lesions of pancreatic cancer, cancer cells are thickly covered with connective tissue called the stroma, making it difficult for administered anti-cancer drugs to reach the cancer cells because they are blocked by the stroma. As a result, the five-year survival rate for pancreatic cancer is extremely low, at less than 10%. The stroma is a supportive tissue that supplies nutrients to cancer cells and supports their structure, and the stroma is also developed in many solid cancers other than pancreatic cancer. Therefore, there is a need to develop treatments for stroma-rich cancers.

Cancer-associated fibroblasts (CAFs), which constitute the stroma, highly express fibroblast activation protein α (FAPα). Therefore, drugs that can bind specifically to FAPα are expected to be effective in the diagnosis of solid tumors, and, if they can further destroy the stroma and tumors, they are also expected to be effective in the treatment of solid tumors.

Patent Literature 1 discloses a drug containing a complex moiety formed from a chelating agent such as 1,4,7,10-tetraazacyclododecane-1,4,7,10-tetraacetic acid (DOTA) and the like and a radionuclide such as ⁶⁸Ga (half-life 67.6 hr), ⁹⁰Y (half-life 64.0 hr), ²⁰³Pb (half-life 51.9 hr) and the like, as a drug targeting FAPα, and describes that the drug can be used for cancer imaging diagnosis. Drugs into which ¹⁸F is introduced by chemical bonding have also been reported.

Patent Literature 2 discloses a drug containing a complex moiety formed from α-(2-carboxyethyl)-1,4,7,10-tetraazacyclododecane-1,4,7,10-tetraacetic acid (DOTA-GA) and ¹¹¹In (half-life 67.3 hr), as a drug targeting FAPα, and describes that the drug can be used for cancer imaging diagnosis.

Non-Patent Literature 1 discloses a drug containing a complex moiety formed from a chelating agent such as 1,4,7,10-tetraazacyclododecane-1,4,7,10-tetraacetic acid (DOTA) and the like and ⁶⁴Cu or ²²⁵Ac, as a drug targeting FAPα, and demonstrates that the drug is effective in the treatment and imaging diagnosis of pancreatic cancer in human pancreatic cancer xenograft mice.

Since the α-ray emitting nuclide ²¹¹At has a shorter half-life than the same α-ray emitting nuclide ²²⁵Ac (²¹¹At: 7.2 hours, ²²⁵Ac: 10 days), drugs labeled with ²¹¹At have a short duration of action and can be used as outpatient treatment. In addition, since ²¹¹At is a short-lived nuclide, drugs labeled with ²¹¹At have the advantage of having a lower risk of prolonged side effects, and are expected to be useful as new anti-cancer drugs.

### [Citation List]

### [Patent Literature]

[Patent Literature 1]
   WO 2019/154886
[Patent Literature 2]
   WO 2019/083990

### [Non-Patent Literature]

[Non-Patent Literature 1]
J Nucl Med. 2020;61:563-569

### [Summary of Invention]

### [Technical Problem]

It is an object of the present invention to provide drugs that bind specifically to FAPα, are effective in the treatment and diagnosis of tumors or cancers expressing FAPα, for example, in the treatment and diagnosis of solid cancers such as pancreatic cancer, sarcoma, esophageal cancer, lung cancer, breast cancer, prostate cancer, head and neck cancer, ovarian cancer, colon cancer, neuroendocrine tumor, thyroid cancer, uterine cancer, liver cancer and the like (particularly, pancreatic cancer), and have a lower risk of prolonged side effects.

### [Solution to Problem]

The present inventors have conducted intensive studies in an attempt to solve the above-mentioned problems and found that a novel compound represented by the following Formula (I-1) or (I-2), which is radiolabeled with a short-lived nuclide such as ²¹¹At and the like, binds specifically to FAPα, and is effective in the treatment and diagnosis of tumors or cancers expressing FAPα, for example, in the treatment and diagnosis of solid cancers such as pancreatic cancer, sarcoma, esophageal cancer, lung cancer, breast cancer, prostate cancer, head and neck cancer, ovarian cancer, colon cancer, neuroendocrine tumor, thyroid cancer, uterine cancer, liver cancer and the like (particularly, pancreatic cancer), which resulted in the completion of the present invention.

Accordingly, the present invention provides the following.

[1] A conjugate comprising
   a radioactive moiety comprising an aryl group substituted with a radionuclide selected from ²¹¹At, ²¹⁰At, ¹³¹I, ¹²⁵I, ¹²⁴I, ¹²³I, ⁷⁷Br and ⁷⁶Br, and
   a bioactive moiety having an affinity for fibroblast activation protein α (FAPα).
[2] The conjugate of the aforementioned [1], wherein the bioactive moiety having an affinity for fibroblast activation protein α comprises a structure represented by the following formula: 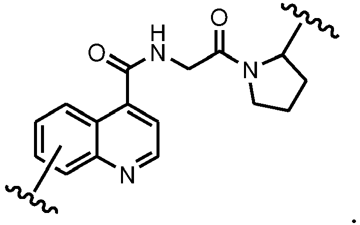
[3] The conjugate of the aforementioned [1] or [2], wherein the radioactive moiety comprises an aryl-C₁₋₃ alkyl group, wherein the aryl is substituted with a radionuclide selected from ²¹¹At, ²¹⁰At, ¹³¹I, ¹²⁵I, ²²⁴I, ¹²³I, ⁷⁷Br and ⁷⁶Br.
[4] A compound radiolabeled represented by Formula (I-1) or a pharmaceutically acceptable salt thereof (hereinafter also referred to as Compound (I-1)): 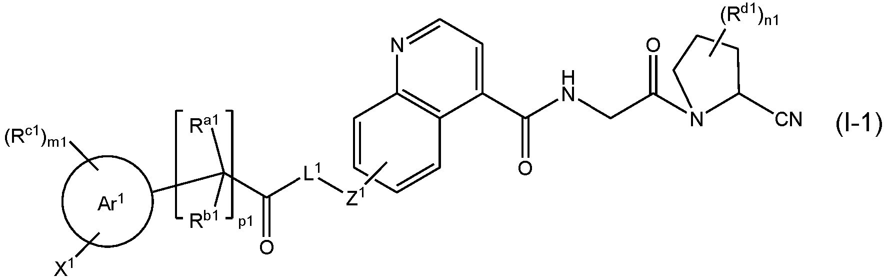 wherein
   X² is a radionuclide selected from ²¹¹At, ²¹⁰At, ¹³¹I, ¹²⁵I, ¹²⁴I, ¹²³I, ⁷⁷Br and ⁷⁶Br;
   Ar¹ is a C₆₋₁₄ aryl group;
   R^{a1} and R^{b1} in the number of p1 are each independently a hydrogen atom or a C₁₋₆ alkyl group;
   R^{c1} in the number of m1 are each independently a C₁₋₆ alkyl group or a hydroxy group;
   R^{d1} in the number of n1 are each independently a halogen atom;
   Z¹ is an oxygen atom, a sulfur atom or NR^{f1} wherein R^{f1} is a hydrogen atom or a C₁₋₃ alkyl group;
   L¹ is
      (1) a linker represented by *-L^{d1}-L^{c1}-L^{b1}-L^{a1}-** wherein
         * indicates a binding site to CO,
         ** indicates a binding site to Z¹,
         L^{a1} is
            (i) a C₁₋₆ alkylene group, or
            (ii) -CH₂-(CH₂-O-CH₂)_{q1}-CH₂- wherein q1 is an integer of 0 to 5, L^{b1} is a bond or -CO-,
         L^{c1} is
            (i) NR^{g1} wherein R^{g1} is a hydrogen atom or a C₁₋₃ alkyl group,
            (ii) a divalent cyclic amino group,
            (iii) an oxygen atom, or
            (iv) a sulfur atom, and
         L^{d1} is
            (i) a bond,
            (ii) *-(NH-A^{a1}-CO)ᵣ₁-*** wherein NH-A^{a1}-CO in the number of r1 are each independently an amino acid residue, r1 is an integer of 1 to 3, * indicates a binding site to CO, and *** indicates a binding site to L^{c1}, or
            (iii) *-NH-B^{a1}-O-B^{b1}-CO-*** wherein -NH-B^{a1}-O- is a divalent residue derived from an aminosaccharide or a derivative thereof, B^{b1} is a C₁₋₆ alkylene group, * indicates a binding site to CO, and *** indicates a binding site to L^{c1}, or
      (2) a linker represented by *-(NH-A^{b1}-CO)ₛ₁-** wherein
         * indicates a binding site to CO,
         ** indicates a binding site to Z¹,
         NH-A^{b1}-CO in the number of s1 are each independently an amino acid residue, and
         s1 is an integer of 1 to 3;
   p1 is an integer of 1 to 3;
   m1 is an integer of 0 to 3; and
   n1 is an integer of 0 to 3.
[5] The compound or pharmaceutically acceptable salt thereof of the aforementioned [4], wherein
   Z¹ is an oxygen atom or a sulfur atom, and
   L¹ is a linker represented by *-L^{d1}-L^{c1}-L^{b1}-L^{a1}-** wherein each symbol is as defined above.
[6] The compound or pharmaceutically acceptable salt thereof of the aforementioned [4], wherein
   Z¹ is NR^{f1} wherein the symbol is as defined above, and
   L¹ is a linker represented by *-(NH-A^{b1}-CO)ₛ₁-** wherein the symbols are as defined above.
[7] The compound or pharmaceutically acceptable salt thereof of the aforementioned [5], wherein L¹ is a linker represented by *-L^{d1}- L^{c1}- L^{b1}- L^{a1}-**
   wherein
   * indicates a binding site to CO,
   ** indicates a binding site to Z¹,
   L^{a1} is a C₁₋₆ alkylene group,
   L^{b1} is a bond or -CO-,
   L^{c1} is a divalent cyclic amino group, and
   L^{d1} is
      (i) a bond,
      (ii) *-(NH-A^{a1}CO)ᵣ₁-*** wherein each symbol is as defined above, or
      (iii) *-NH-B^{a1}-O-B^{b1}-CO-*** wherein each symbol is as defined above.
[8] The compound or pharmaceutically acceptable salt thereof of any one of the aforementioned [4], [5] and [7], wherein the divalent cyclic amino group in L^{c1} is a divalent 3- to 8-membered cyclic diamino group.
[9] The compound or pharmaceutically acceptable salt thereof of the aforementioned [5], wherein L¹ is a linker represented by *-L^{d1}-L^{c1}-L^{b1}-L^{a1}-** wherein
   * indicates a binding site to CO,
   ** indicates a binding site to Z¹,
   L^{a1} is -CH₂-(CH₂-O-CH₂)_{q1}-CH₂- wherein the symbol is as defined above,
   L^{b1} is a bond,
   L^{c1} is NR^{g1} wherein the symbol is as defined above, an oxygen atom or a sulfur atom, and
   L^{d1} is a bond.
[10] The compound or pharmaceutically acceptable salt thereof of the aforementioned [4] or [6], wherein s1 is 1.
[11] A compound radiolabeled represented by Formula (I-2) or a pharmaceutically acceptable salt thereof (hereinafter also referred to as Compound (I-2)): 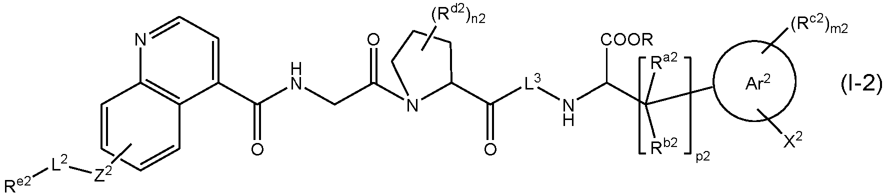 wherein
   X² is a radionuclide selected from ²¹¹At, ²¹⁰At, ¹³¹I, ¹²⁵I, ¹²⁴I, ¹²³I, ⁷⁷Br and ⁷⁶Br;
   Ar² is a C₆₋₁₄ aryl group;
   R^{a2} and R^{b2} in the number of p2 are each independently a hydrogen atom or a C₂₋₆ alkyl group;
   R^{c2} in the number of m2 are each independently a C₂₋₆ alkyl group or a hydroxy group;
   R^{d2} in the number of n2 are each independently a halogen atom;
   Z² is an oxygen atom, a sulfur atom or NR^{f2} wherein R^{f2} is a hydrogen atom or a C₁₋₃ alkyl group;
   L² is
      (1) a linker represented by *-L^{c2}-L^{b2}-L^{a2}-**
         wherein
         * indicates a binding site to R^{e2},
         ** indicates a binding site to Z²,
         L^{a2} is
            (i) a C₁₋₆ alkylene group, or
            (ii) -CH₂- (CH₂-O-CH₂)_{q2}-CH₂- wherein q2 is an integer of 0 to 5, L^{b2} is a bond or -CO-, and
         L^{c2} is
            (i) NR^{g2} wherein R^{g2} is a hydrogen atom or a C₁₋₃ alkyl group,
            (ii) a divalent cyclic amino group,
            (iii) an oxygen atom, or
            (iv) a sulfur atom, or
      (2) a linker represented by *-(NH-A^{a2}-CO) ᵣ₂-**
         wherein
         * indicates a binding site to R^{e2},
         ** indicates a binding site to Z²,
         NH-A^{a2}-CO in the number of r2 are each independently an amino acid residue, and
         r2 is an integer of 1 to 3;
   R^{e2} is a C₁₋₆ alkyl-carbonyl group; or
   the group R^{e2}-L²-Z²- is a hydrogen atom;
   L³ is a linker represented by ***-(NH-A^{b2}-CO)ₛ₂-****
      wherein
      *** indicates a binding site to CO,
      **** indicates a binding site to NH,
      NH-A^{b2}-CO in the number of s2 are each independently an amino acid residue, and
      s2 is an integer of 0 to 3;
   R is a hydrogen atom or a C₁₋₃ alkyl group;
   p2 is an integer of 0 to 3;
   m2 is an integer of 0 to 3; and
   n2 is an integer of 0 to 3.
[12] The compound or pharmaceutically acceptable salt thereof of the aforementioned [11], wherein
   Z² is an oxygen atom or a sulfur atom, and
   L² is a linker represented by *-L^{c2}-L^{b2}-L^{a2}-** wherein each symbol is as defined above.
[13] The compound or pharmaceutically acceptable salt thereof of the aforementioned [11], wherein
   Z² is NR^{f2} wherein the symbol is as defined above, and
   L² is a linker represented by *-(NH-A^{a2}-CO)ᵣ₂-** wherein each symbol is as defined above.
[14] The compound or pharmaceutically acceptable salt thereof of any one of the aforementioned [11] to [13], wherein p2 is an integer of 1 to 3.
[15] The compound or pharmaceutically acceptable salt thereof of any one of the aforementioned [11] to [14], wherein s2 is 0.
[16] A pharmaceutical composition comprising a compound or pharmaceutically acceptable salt thereof as defined in any one of the aforementioned [11] to [15], and a pharmaceutically acceptable carrier.
[17] A therapeutic agent for a tumor or cancer expressing fibroblast activation protein α (FAPα), comprising a compound or pharmaceutically acceptable salt thereof as defined in any one of the aforementioned [11] to [15].
[18] The therapeutic agent of the aforementioned [17], wherein the tumor or cancer expressing fibroblast activation protein α is pancreatic cancer, sarcoma, esophageal cancer, lung cancer, breast cancer, prostate cancer, head and neck cancer, ovarian cancer, colon cancer, neuroendocrine tumor, thyroid cancer, uterine cancer or liver cancer.
[19] A compound or pharmaceutically acceptable salt thereof as defined in any one of Claims 1 to 15, for use in the treatment of a tumor or cancer expressing fibroblast activation protein α (FAPα).
[20] The compound or pharmaceutically acceptable salt thereof of the aforementioned [19], wherein the tumor or cancer expressing fibroblast activation protein α is pancreatic cancer, sarcoma, esophageal cancer, lung cancer, breast cancer, prostate cancer, head and neck cancer, ovarian cancer, colon cancer, neuroendocrine tumor, thyroid cancer, uterine cancer or liver cancer.
[21] A method for treating a tumor or cancer expressing fibroblast activation protein α (FAPα) in a mammal, comprising administering to the mammal a therapeutically effective amount of a compound or pharmaceutically acceptable salt thereof as defined in any one of Claims 1 to 15.
[22] The method of the aforementioned [21], wherein the tumor or cancer expressing fibroblast activation protein α is pancreatic cancer, sarcoma, esophageal cancer, lung cancer, breast cancer, prostate cancer, head and neck cancer, ovarian cancer, colon cancer, neuroendocrine tumor, thyroid cancer, uterine cancer or liver cancer.
[23] Use of a compound or pharmaceutically acceptable salt thereof as defined in any one of Claims 1 to 15, for the manufacture of a therapeutic agent for a tumor or cancer expressing fibroblast activation protein α (FAPα).
[24] The use of the aforementioned [23], wherein the tumor or cancer expressing fibroblast activation protein α is pancreatic cancer, sarcoma, esophageal cancer, lung cancer, breast cancer, prostate cancer, head and neck cancer, ovarian cancer, colon cancer, neuroendocrine tumor, thyroid cancer, uterine cancer or liver cancer.
[25] A compound represented by Formula (II-1) or a salt thereof (hereinafter also referred to as Compound (II-1)): 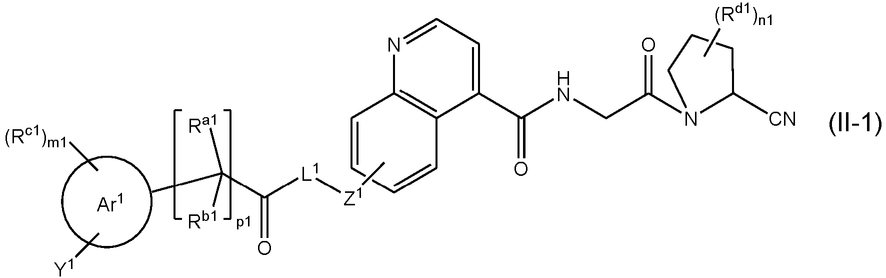 wherein
   Y¹ is a boryl group (-B(OH)₂) or its ester group;
   Ar¹ is a C₆₋₁₄ aryl group;
   R^{a1} and R^{b1} in the number of p1 are each independently a hydrogen atom or a C₁₋₆ alkyl group;
   R^{c1} in the number of m1 are each independently a C₁₋₆ alkyl group or a hydroxy group;
   R^{d1} in the number of n1 are each independently a halogen atom;
   Z¹ is an oxygen atom, a sulfur atom or NR^{f1} wherein R^{f1} is a hydrogen atom or a C₁₋₃ alkyl group;
   L¹ is
      (1) a linker represented by *-L^{d1}-L^{c1}-L^{b1}-L^{a1}-**
         wherein
         * indicates a binding site to CO,
         ** indicates a binding site to Z¹,
         L^{a1} is
            (i) a C₁₋₆ alkylene group, or
            (ii) -CH₂-(CH₂-O-CH₂)_{q1}-CH₂- wherein q1 is an integer of 0 to 5, L^{b1} is a bond or -CO-,
         L^{c1} is
            (i) NR^{g1} wherein R^{g1} is a hydrogen atom or a C₁₋₃ alkyl group,
            (ii) a divalent cyclic amino group,
            (iii) an oxygen atom, or
            (iv) a sulfur atom, and
         L^{d1} is
            (i) a bond,
            (ii) *-(NH-A^{a1}-CO)ᵣ₁-*** wherein NH-A^{a1}-CO in the number of r1 are each independently an amino acid residue, r1 is an integer of 1 to 3, * indicates a binding site to CO, and *** indicates a binding site to L^{c1}, or
            (iii) *-NH-B^{a1}-O-B^{b1}-CO-*** wherein -NH-B^{a1}-O- is a divalent residue derived from an aminosaccharide or a derivative thereof, B^{b1} is a C₁₋₆ alkylene group, * indicates a binding site to CO, and *** indicates a binding site to L^{c1}, or
      (2) a linker represented by *- (NH-A^{b1}-CO)ₛ₁-**
         wherein
         * indicates a binding site to CO,
         ** indicates a binding site to Z¹,
         NH-A^{b1}-CO in the number of s1 are each independently an amino acid residue, and
         s1 is an integer of 1 to 3;
   p1 is an integer of 1 to 3;
   m1 is an integer of 0 to 3; and
   n1 is an integer of 0 to 3.
[26] A compound radiolabeled represented by Formula (II-2) or a pharmaceutically acceptable salt thereof (hereinafter also referred to as Compound (II-2)): 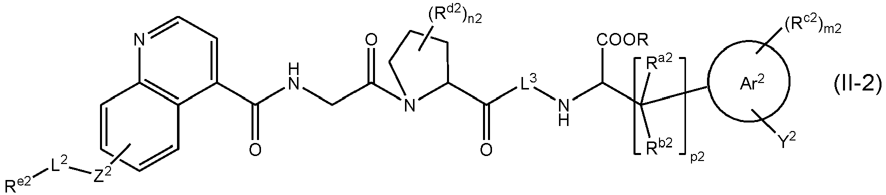 wherein
   Y² is a boryl group (-B(OH)₂) or its ester group;
   Ar² is a C₆₋₁₄ aryl group;
   R^{a2} and R^{b2} in the number of p2 are each independently a hydrogen atom or a C₁₋₆ alkyl group;
   R^{c2} in the number of m2 are each independently a C₁₋₆ alkyl group or a hydroxy group;
   R^{d2} in the number of n2 are each independently a halogen atom; Z² is an oxygen atom, a sulfur atom or NR^{f2} wherein R^{f2} is a hydrogen atom or a C₁₋₃ alkyl group;
   L² is
      (1) a linker represented by *-L^{c2}-L^{b2}-L^{a2}-**
         wherein
         * indicates a binding site to R^{e2},
         ** indicates a binding site to Z²,
         L^{a2} is
            (i) a C₁₋₆ alkylene group, or
            (ii) -CH₂-(CH₂-O-CH₂)_{q2}-CH₂- wherein q2 is an integer of 0 to 5, L^{b2} is a bond or -CO-, and
         L^{c2} is
            (i) NR^{g2} wherein R^{g2} is a hydrogen atom or a C₁₋₃ alkyl group,
            (ii) a divalent cyclic amino group,
            (iii) an oxygen atom, or
            (iv) a sulfur atom, or
      (2) a linker represented by *-(NH-A^{a2}-CO)ᵣ₂-**
         wherein
         * indicates a binding site to R^{e2},
         ** indicates a binding site to Z²,
         NH-A^{a2}-CO in the number of r2 are each independently an amino acid residue, and
         r2 is an integer of 1 to 3;
   R^{e2} is a C₁₋₆ alkyl-carbonyl group; or
   the group R^{e2}-L²-Z²- is a hydrogen atom;
   L³ is a linker represented by ***-(NH-A^{b2}-CO)ₛ₂-****
      wherein
      *** indicates a binding site to CO,
      **** indicates a binding site to NH,
      NH-A^{b2}-CO in the number of s2 are each independently an amino acid residue, and
      s2 is an integer of 0 to 3;
   R is a hydrogen atom or a C₁₋₃ alkyl group;
   p2 is an integer of 0 to 3;
   m2 is an integer of 0 to 3; and
   n2 is an integer of 0 to 3.
[27] A method for producing a compound radiolabeled represented by Formula (I-1) or a pharmaceutically acceptable salt thereof, comprising the following step; 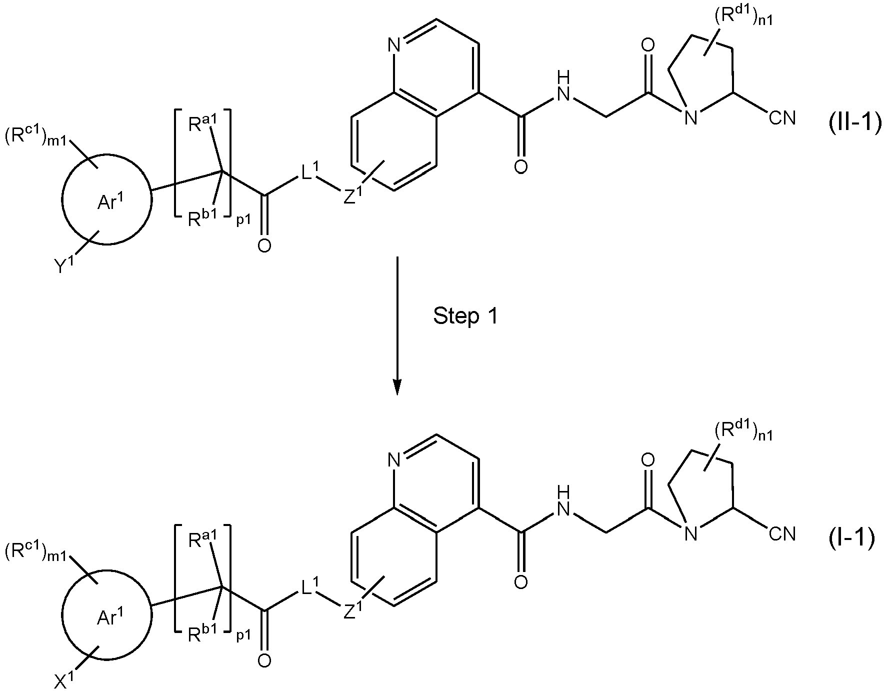 wherein
   X¹ is a radionuclide selected from ²¹¹At, ²¹⁰At, ¹³¹I, ¹²⁵I, ¹²⁴I, ¹²³I, ⁷⁷Br and ⁷⁶Br;
   Y¹ is a boryl group (-B(OH)₂) or its ester group;
   Ar¹ is a C₆₋₁₄ aryl group;
   R^{a1} and R^{b1} in the number of p1 are each independently a hydrogen atom or a C₁₋₆ alkyl group;
   R^{c1} in the number of m1 are each independently a C₁₋₆ alkyl group or a hydroxy group;
   R^{d1} in the number of n1 are each independently a halogen atom;
   Z¹ is an oxygen atom, a sulfur atom or NR^{f1} wherein R^{f1} is a hydrogen atom or a C₁₋₃ alkyl group;
   L¹ is
      (1) a linker represented by *-L^{d1}-L^{c1}-L^{b1}-L^{a1}-**
         wherein
         * indicates a binding site to CO,
         ** indicates a binding site to Z¹,
         L^{a1} is
            (i) a C₁₋₆ alkylene group, or
            (ii) -CH₂-(CH₂-O-CH₂)_{q1}-CH₂- wherein q1 is an integer of 0 to 5, L^{b1} is a bond or -CO-,
         L^{c1} is
            (i) NR^{g1} wherein R^{g1} is a hydrogen atom or a C₁₋₃ alkyl group,
            (ii) a divalent cyclic amino group,
            (iii) an oxygen atom, or
            (iv) a sulfur atom, and
         L^{d1} is
            (i) a bond,
            (ii) *-(NH-A^{a1}-CO)ᵣ₁-*** wherein NH-A^{a1}-CO in the number of r1 are each independently an amino acid residue, r1 is an integer of 1 to 3, * indicates a binding site to CO, and *** indicates a binding site to L^{c1}, or
            (iii) *-NH-B^{a1}-O-B^{b1}-CO-*** wherein -NH-B^{a1}-O- is a divalent residue derived from an aminosaccharide or a derivative thereof, B^{b1} is a C₁₋₆ alkylene group, * indicates a binding site to CO, and *** indicates a binding site to L^{c1}, or
      (2) a linker represented by *-(NH-A^{b1}-CO)ₛ₁-**
         wherein
         * indicates a binding site to CO,
         ** indicates a binding site to Z¹,
         NH-A^{b1}-CO in the number of s1 are each independently an amino acid residue, and
         s1 is an integer of 1 to 3;
   p1 is an integer of 1 to 3;
   m1 is an integer of 0 to 3; and
   n1 is an integer of 0 to 3,
   Step 1: a step of reacting a compound represented by Formula (II-1) or a salt thereof with a radionuclide selected from ²¹¹At, ²¹⁰At, ¹³¹I, ¹²⁵I, ¹²⁴I, ¹²³I, ⁷⁷Br and ⁷⁶Br in the presence of a reagent selected from an alkali metal iodide, an alkali metal bromide, N-bromosuccinimide, N-chlorosuccinimide, N-iodosuccinimide and hydrogen peroxide, in water to obtain a compound radiolabeled represented by Formula (I-1) or a pharmaceutically acceptable salt thereof.
[28] A method for producing a compound radiolabeled represented by Formula (I-2) or a pharmaceutically acceptable salt thereof, comprising the following step; 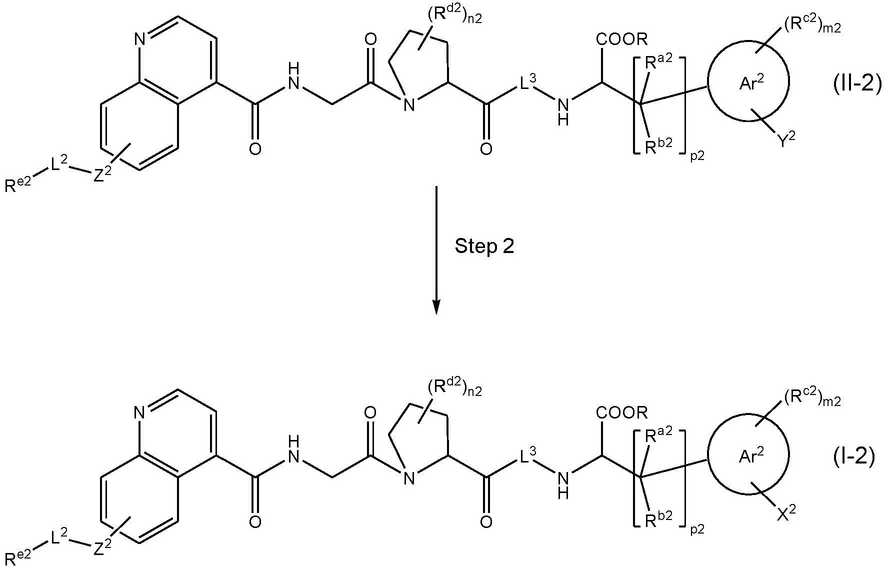 wherein
   X² is a radionuclide selected from ²¹¹At, ²¹⁰At, ¹³¹I, ¹²⁵I, ¹²⁴I, ¹²³I, ⁷⁷Br and ⁷⁶Br;
   Y² is a boryl group (-B(OH)₂) or its ester group;
   Ar² is a C₆₋₁₄ aryl group;
   R^{a2} and R^{b2} in the number of p2 are each independently a hydrogen atom or a C₂₋₆ alkyl group;
   R^{c2} in the number of m2 are each independently a C₁₋₆ alkyl group or a hydroxy group;
   R^{d2} in the number of n2 are each independently a halogen atom; Z² is an oxygen atom, a sulfur atom or NR^{f2} wherein R^{f2} is a hydrogen atom or a C₁₋₃ alkyl group;
   L² is
      (1) a linker represented by *-L^{c2}-L^{b2}-L^{a2}-**
         wherein
         * indicates a binding site to R^{e2},
         ** indicates a binding site to Z²,
         L^{a2} is
            (i) a C₁₋₆ alkylene group, or
            (ii) -CH₂-(CH₂-O-CH₂)_{q2}-CH₂- wherein q2 is an integer of 0 to 5, L^{b2} is a bond or -CO-, and
         L^{c2} is
            (i) NR^{g2} wherein R^{g2} is a hydrogen atom or a C₁₋₃ alkyl group,
            (ii) a divalent cyclic amino group,
            (iii) an oxygen atom, or
            (iv) a sulfur atom, or
      (2) a linker represented by *-(NH-A^{a2}-CO)ᵣ₂-**
         wherein
         * indicates a binding site to R^{e2},
         ** indicates a binding site to Z²,
         NH-A^{a2}-CO in the number of r2 are each independently an amino acid residue, and
         r2 is an integer of 1 to 3;
   R^{e2} is a C₁₋₆ alkyl-carbonyl group; or
   the group R^{e2}-L²-Z²- is a hydrogen atom;
   L³ is a linker represented by ***-(NH-A^{b2}-CO)ₛ₂-****
      wherein
      *** indicates a binding site to CO,
      **** indicates a binding site to NH,
      NH-A^{b2}-CO in the number of s2 are each independently an amino acid residue, and
      s2 is an integer of 0 to 3;
   R is a hydrogen atom or a C₁₋₃ alkyl group;
   p2 is an integer of 0 to 3;
   m2 is an integer of 0 to 3; and
   n2 is an integer of 0 to 3,
   Step 2: a step of reacting a compound represented by Formula (II-2) or a salt thereof with a radionuclide selected from ²¹¹At, ²¹⁰At, ¹³¹I, ¹²⁵I, ¹²⁴I, ¹²³I, ⁷⁷Br and ⁷⁶Br in the presence of a reagent selected from an alkali metal iodide, an alkali metal bromide, N-bromosuccinimide, N-chlorosuccinimide, N-iodosuccinimide and hydrogen peroxide, in water to obtain a compound radiolabeled represented by Formula (I-2) or a pharmaceutically acceptable salt thereof.

### [Advantageous Effects of Invention]

According to the present invention, it is possible to provide radiolabeled compounds that bind specifically to FAPα, are effective in the treatment and diagnosis of tumors or cancers expressing FAPα, for example, in the treatment and diagnosis of solid cancers such as pancreatic cancer, sarcoma, esophageal cancer, lung cancer, breast cancer, prostate cancer, head and neck cancer, ovarian cancer, colon cancer, neuroendocrine tumor, thyroid cancer, uterine cancer, liver cancer and the like (particularly, pancreatic cancer), and have a lower risk of prolonged side effects.

### [Brief Description of Drawings]

[Fig. 1]

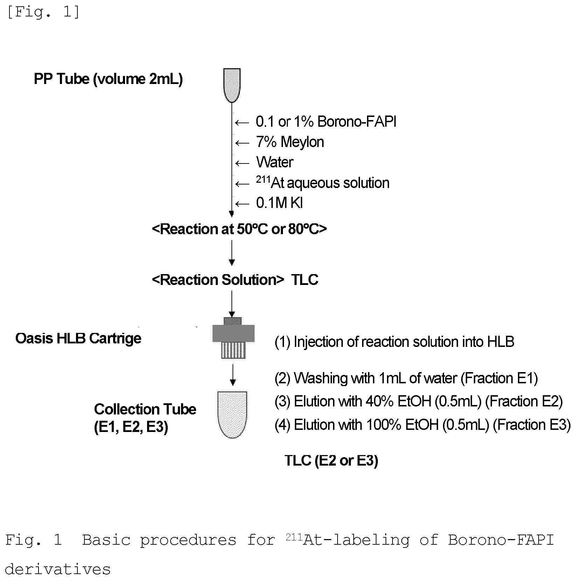

   Fig. 1 outlines basic procedures for radiolabeling of the boronic acid compounds in Examples 1-14.
[Fig. 2]

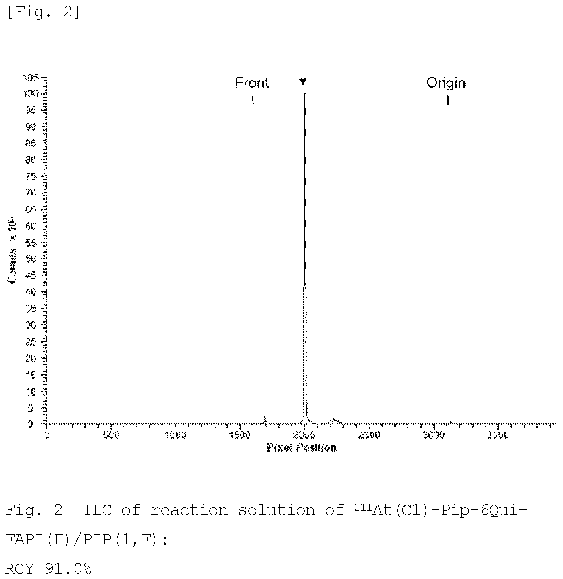

   Fig. 2 shows the analysis results of the reaction solution in Example 1 by thin-layer chromatograph method (TLC).
[Fig. 3]

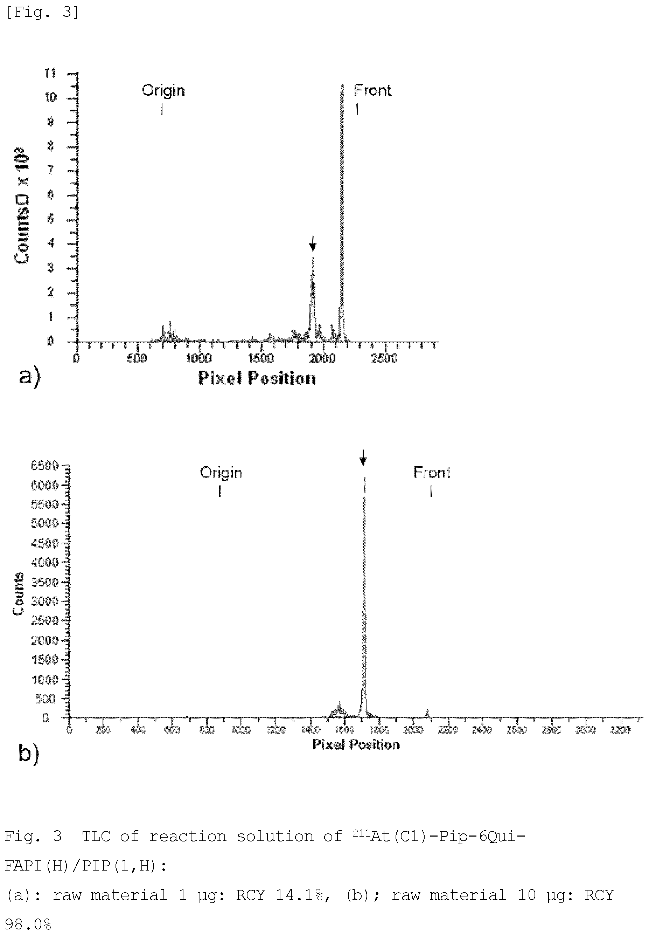

   Fig. 3 shows the analysis results of the reaction solution in Example 2 by thin-layer chromatograph method (TLC). (a) shows the analysis results for 1 µg of the raw material, and (b) shows the analysis results for 10 µg of the raw material.
[Fig. 4]

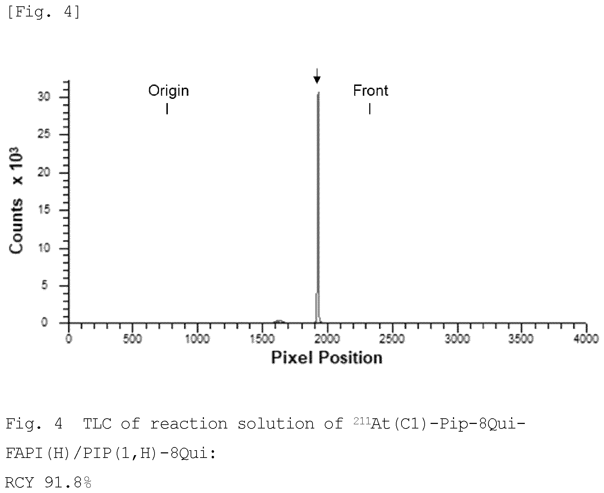

   Fig. 4 shows the analysis results of the reaction solution in Example 3 by thin-layer chromatograph method (TLC).
[Fig. 5]

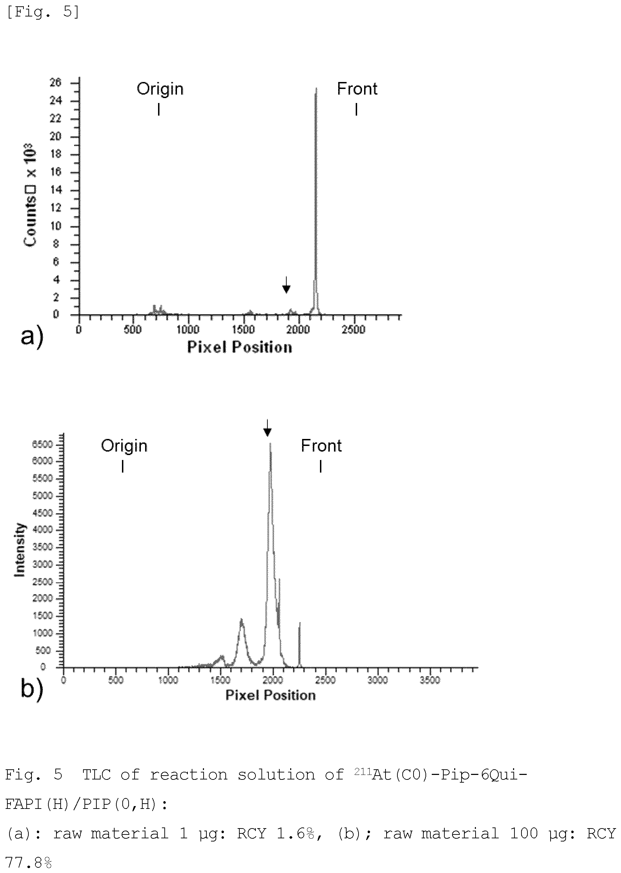

   Fig. 5 shows the analysis results of the reaction solution in Example 4 by thin-layer chromatograph method (TLC). (a) shows the analysis results for 1 µg of the raw material, and (b) shows the analysis results for 100 µg of the raw material.
[Fig. 6]

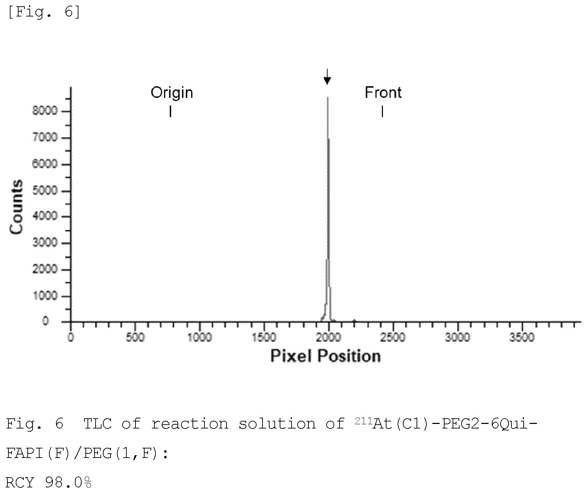

   Fig. 6 shows the analysis results of the reaction solution in Example 5 by thin-layer chromatograph method (TLC).
[Fig. 7]

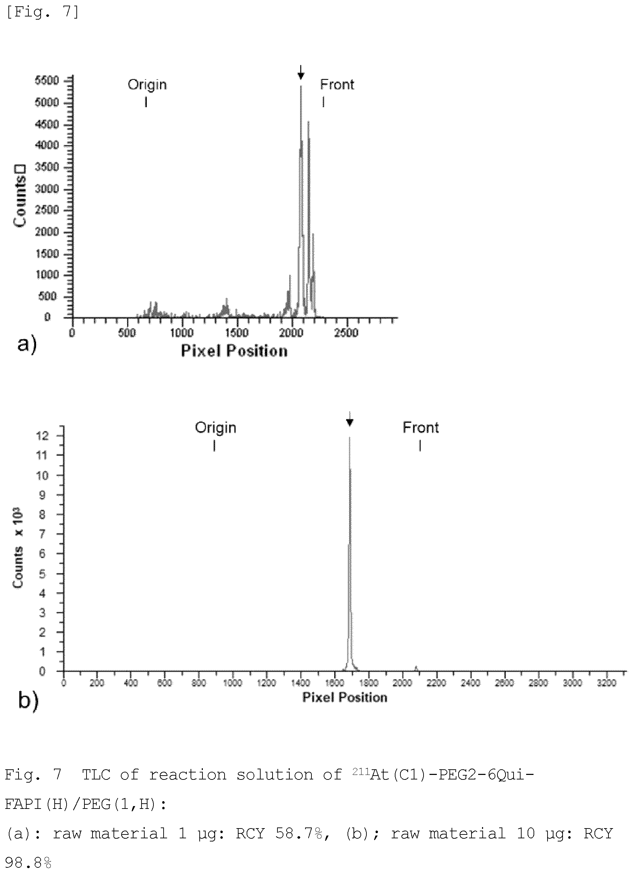

   Fig. 7 shows the analysis results of the reaction solution in Example 6 by thin-layer chromatograph method (TLC). (a) shows the analysis results for 1 µg of the raw material, and (b) shows the analysis results for 10 µg of the raw material.
[Fig. 8]

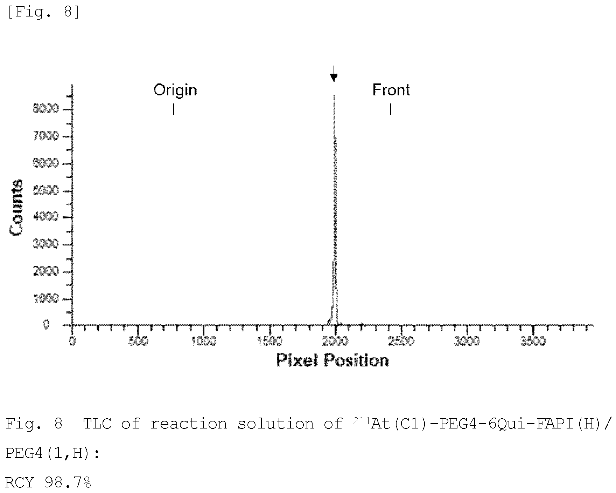

   Fig. 8 shows the analysis results of the reaction solution in Example 7 by thin-layer chromatograph method (TLC).
[Fig. 9]

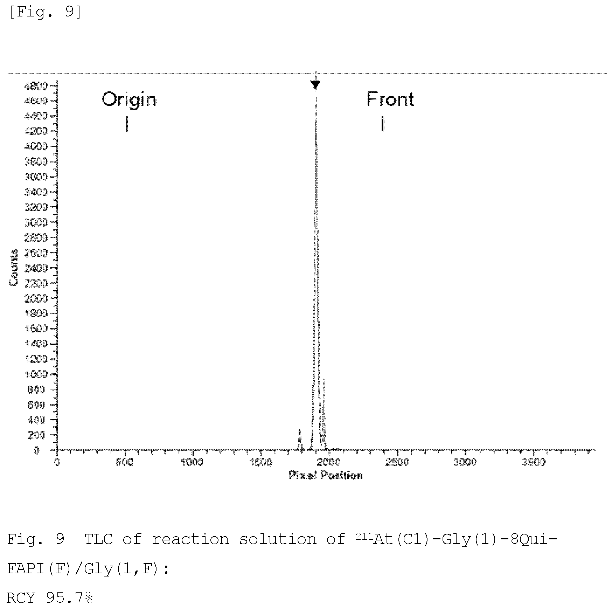

   Fig. 9 shows the analysis results of the reaction solution in Example 8 by thin-layer chromatograph method (TLC).
[Fig. 10]

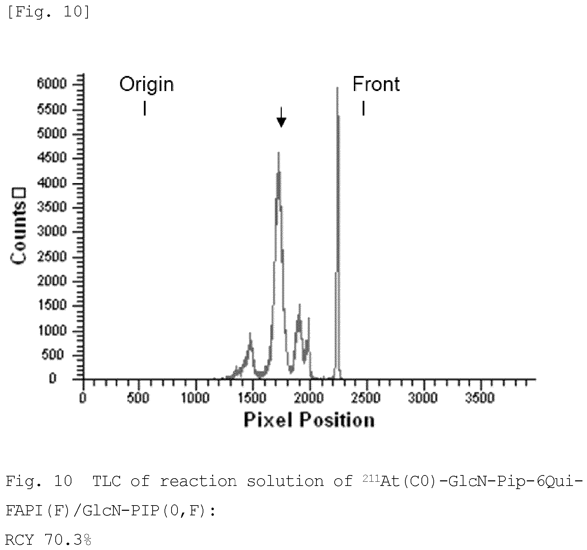

   Fig. 10 shows the analysis results of the reaction solution in Example 9 by thin-layer chromatograph method (TLC).
[Fig. 11]

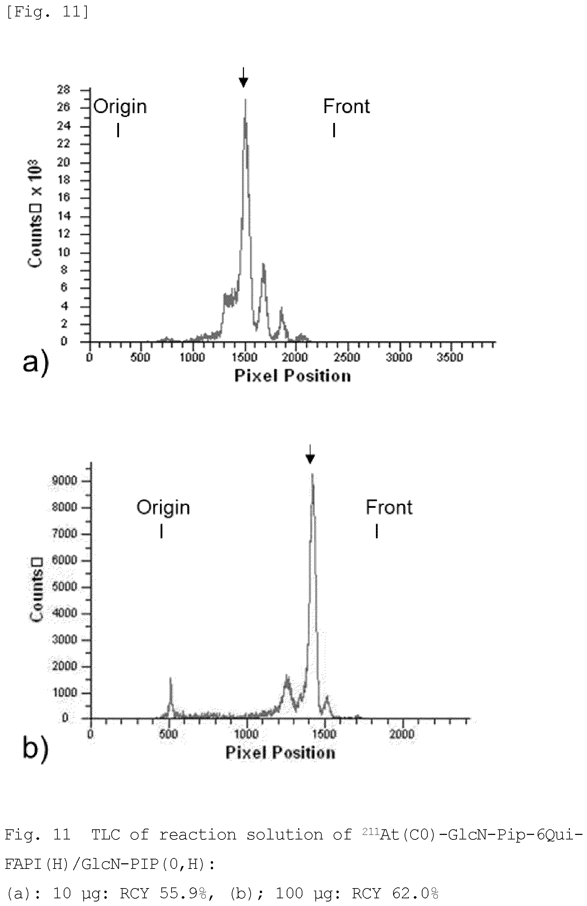

   Fig. 11 shows the analysis results of the reaction solution in Example 10 by thin-layer chromatograph method (TLC). (a) shows the analysis results for 10 µg of the raw material, and (b) shows the analysis results for 100 µg of the raw material.
[Fig. 12]

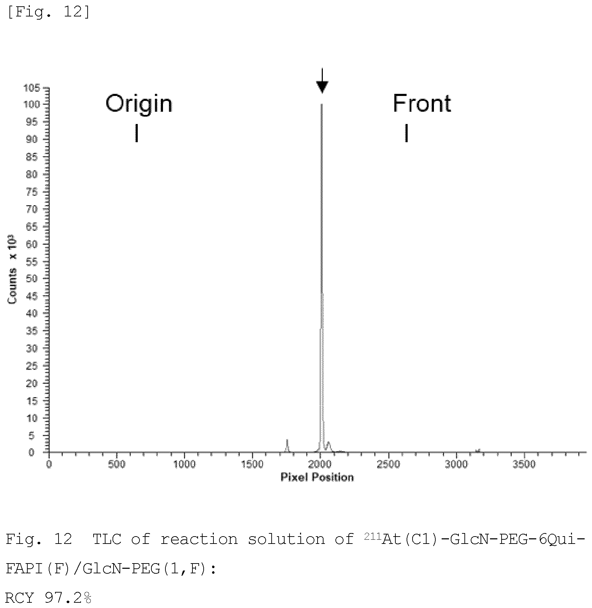

   Fig. 12 shows the analysis results of the reaction solution in Example 11 by thin-layer chromatograph method (TLC).
[Fig. 13]

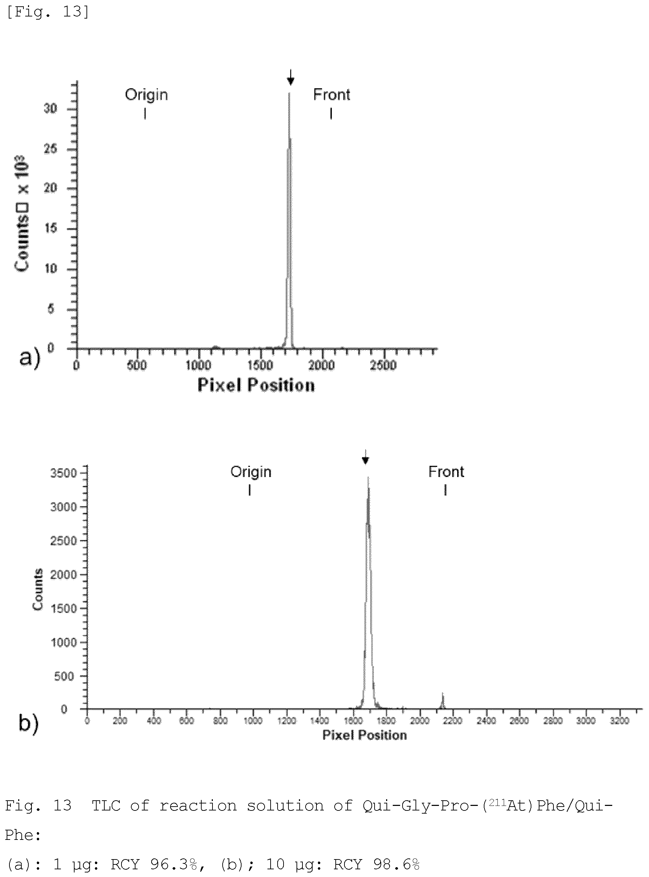

   Fig. 13 shows the analysis results of the reaction solution in Example 12 by thin-layer chromatograph method (TLC). (a) shows the analysis results for 1 µg of the raw material, and (b) shows the analysis results for 10 µg of the raw material.
[Fig. 14]

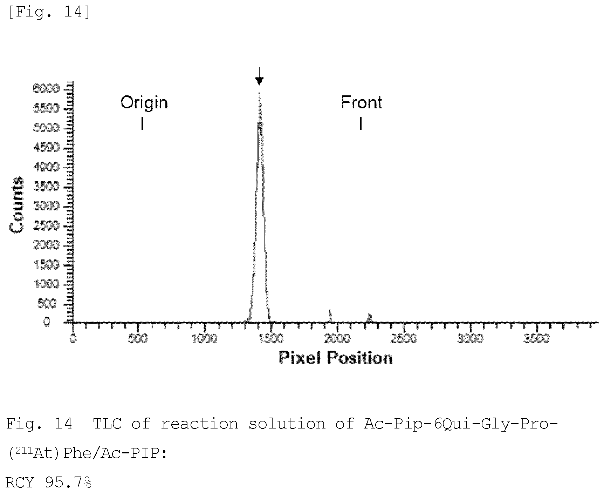

   Fig. 14 shows the analysis results of the reaction solution in Example 13 by thin-layer chromatograph method (TLC).
[Fig. 15]

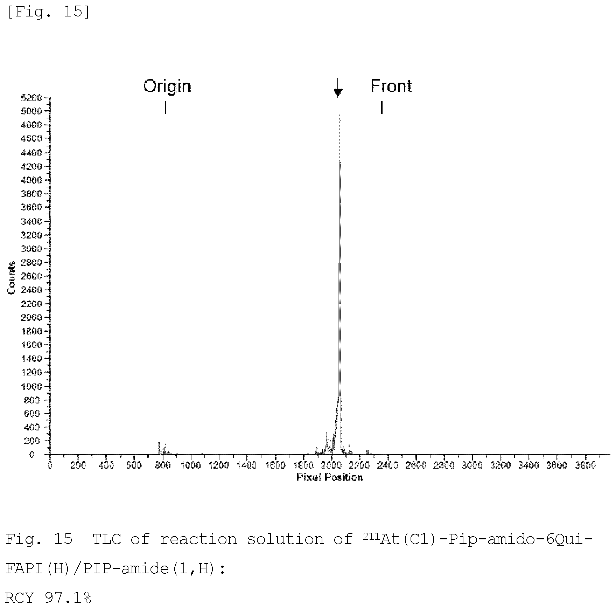

   Fig. 15 shows the analysis results of the reaction solution in Example 14 by thin-layer chromatograph method (TLC).
[Fig. 16]

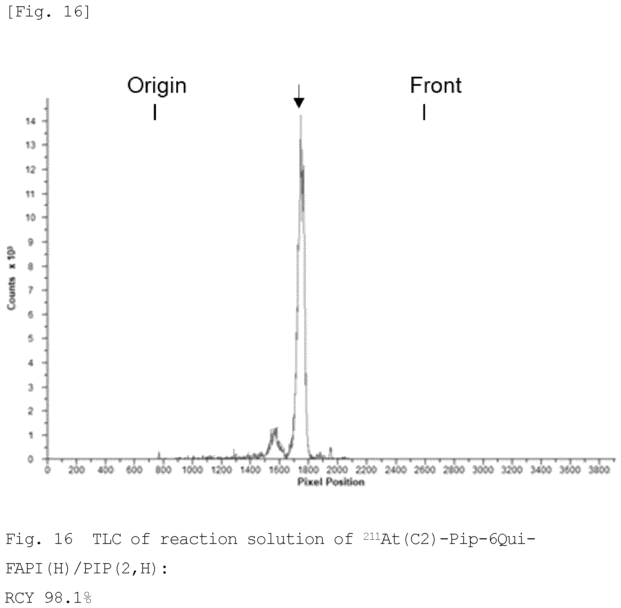

   Fig. 16 shows the analysis results of the reaction solution in Example 15 by thin-layer chromatograph method (TLC).
[Fig. 17]

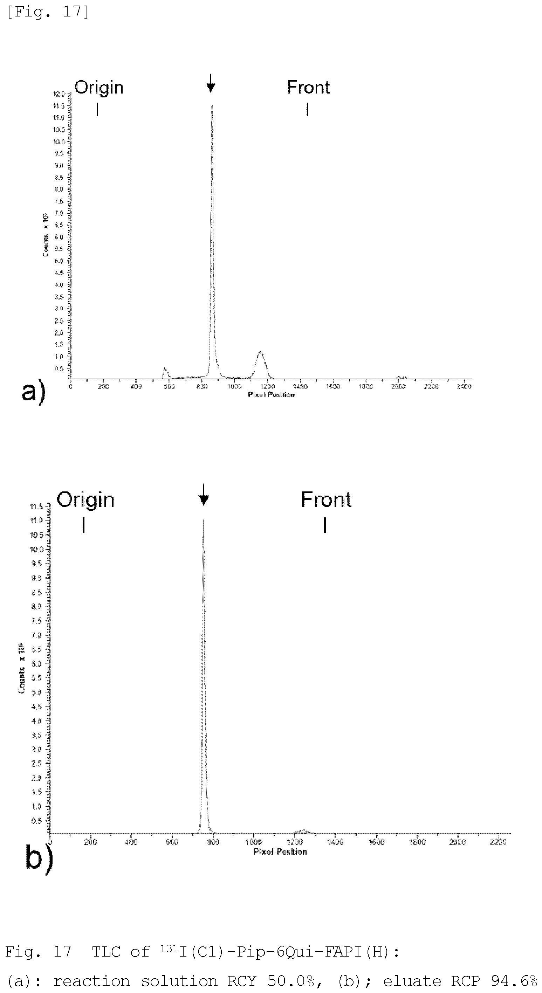

   Fig. 17 shows the analysis results of the reaction solution and eluate in Example 16 by thin-layer chromatograph method (TLC). (a) shows the analysis results of the reaction solution, and (b) shows the analysis results of the eluate.
[Fig. 18]

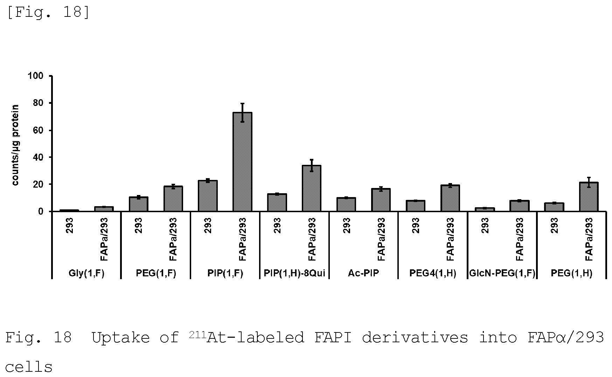

   Fig. 18 is a graph showing the uptake of ²¹¹At-labeled FAPI derivatives into FAPα/293 cells.
[Fig. 19]

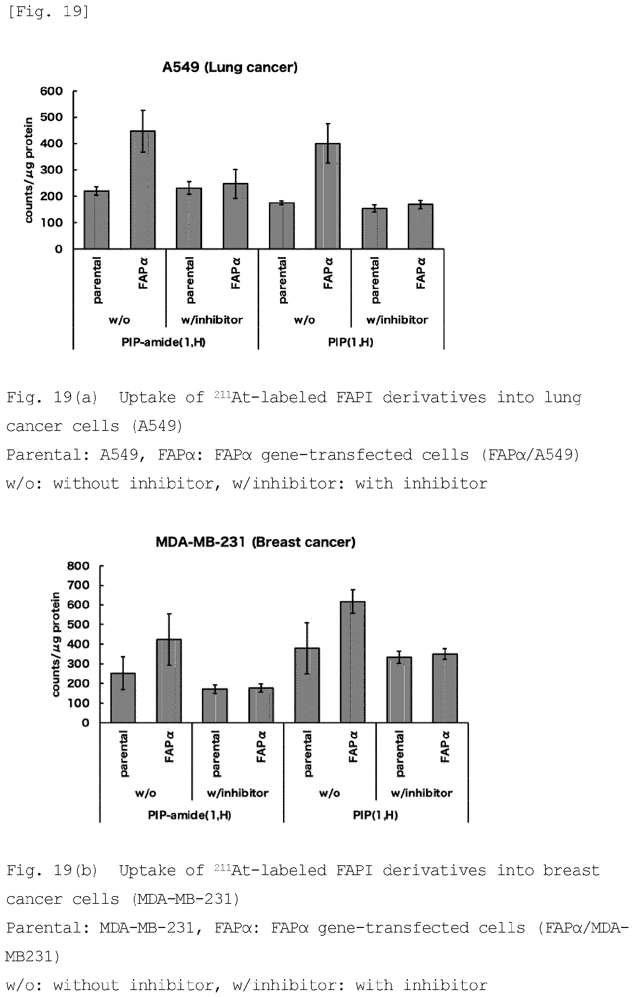

   Fig. 19(a) is a graph showing the uptake of ²¹¹At-labeled FAPI derivatives into lung cancer cells (A549), and Fig. 19(b) is a graph showing the uptake of ²¹¹At-labeled FAPI derivatives into breast cancer cells (MDA-MB-231).
[Fig. 20]

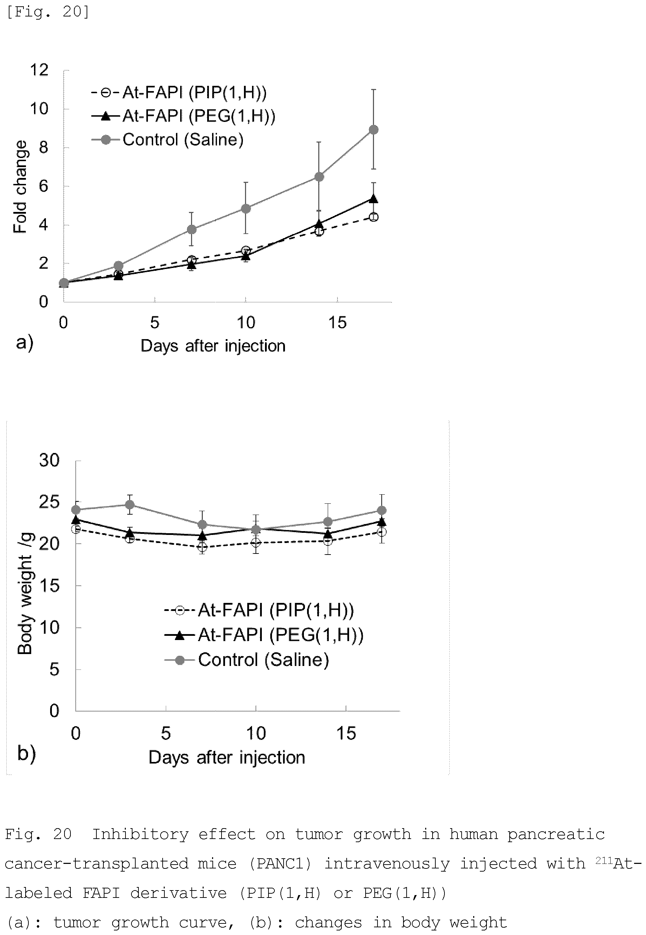

   Fig. 20 is a graph showing the inhibitory effect of ²¹¹At-labeled FAPI derivatives on tumor growth in human pancreatic cancer-transplanted mice. (a) is a graph showing tumor growth in mice, and (b) is a graph showing changes in body weight.

### [Description of Embodiments]

The present invention will be explained in detail below.

In the present specification, examples of the "halogen atom" include a fluorine atom, a chlorine atom, a bromine atom and iodine atom.

In the present specification, the "C₁₋₃ alkyl group" means a linear or branched alkyl group having 1 to 3 carbon atoms, and examples thereof include methyl, ethyl, propyl and isopropyl.

In the present specification, the "C₁₋₆ alkyl group" means a linear or branched alkyl group having 1 to 6 carbon atoms, and examples thereof include methyl, ethyl, propyl, isopropyl, butyl, isobutyl, sec-butyl, tert-butyl, pentyl, isopentyl, neo-pentyl, 1-ethylpropyl, hexyl, isohexyl, 1,1-dimethylbutyl, 2,2-dimethylbutyl, 3,3-dimethylbutyl, 2-ethylbutyl and the like. Preferred is "C₁₋₃ alkyl group".

In the present specification, the "aryl group" means a cyclic hydrocarbon group having aromaticity, and examples thereof include phenyl, 1-naphthyl, 2-naphthyl, 1-anthryl, 2-anthryl, 9-anthryl and the like. Preferred is a C₆₋₁₄ aryl group, and more preferred is a phenyl group.

In the present specification, the "C₆₋₁₄ aryl group" means a cyclic hydrocarbon group having aromaticity and having 6 to 14 carbon atoms, and examples thereof include phenyl, 1-naphthyl, 2-naphthyl, 1-anthryl, 2-anthryl, 9-anthryl and the like. Preferred is a phenyl group.

In the present specification, the "C₆₋₁₄ aryl-C₁₋₃ alkyl group" means the aforementioned "C₁₋₃ alkyl group" substituted by aforementioned "C₆₋₁₄ aryl group", and examples thereof include benzyl, 1-phenylethyl, 2-phenylethyl, 3-phenylpropyl, (1-naphthyl)methyl, (2-naphthyl)methyl, 2-(1-naphthyl)ethyl and the like. Preferred is a phenyl-C₁₋₃ alkyl group.

In the present specification, the "phenyl-C₁₋₃ alkyl group" means the aforementioned "C₁₋₃ alkyl group" substituted by phenyl, and examples thereof include benzyl, 1-phenylethyl, 2-phenylethyl, 3-phenylpropyl and the like.

In the present specification, the "C₁₋₆ alkyl-carbonyl group" means a group represented by formula R'C(O)- wherein R' is a C₁₋₆ alkyl group, and examples thereof include acetyl, propanoyl, butanoyl, 2-methylpropanoyl, pentanoyl, 3-methylbutanoyl, 2-methylbutanoyl, 2,2-dimethylpropanoyl, hexanoyl, heptanoyl and the like.

In the present specification, the "C₁₋₆ alkylene group" means a linear or branched alkylene group having 1 to 6 carbon atoms, and examples thereof include -CH₂-, -(CH₂)₂-, -(CH₂)₃-, -(CH2)₄-, - (CH₂)₅-, -(CH₂)₆-, -CH(CH₃)-, -C(CH₃)₂-, -CH(C₂H₅)-, -CH(C₃H₇)-, - CH(CH(CH₃)₂)-, -(CH(CH₃))₂-, -CH₂-CH(CH₃)-, -CH(CH₃)-CH₂-, -CH₂-CH₂-C(CH₃)₂-, -C(CH₃)₂-CH₂-CH₂-, -CH₂-CH₂-CH₂-C(CH₃)₂-, -C(CH₃)₂-CH₂-CH₂-CH₂- and the like. Preferred is a C₂₋₄ alkylene group.

In the present specification, the "C₂₋₄ alkylene group" means a linear or branched alkylene group having 2 to 4 carbon atoms, and examples thereof include -(CH₂)₂-, -(CH₂)₃-, -(CH₂)₄-, -CH(CH₃)-, -C(CH₃)₂-, -CH(C₂H₅)-, -CH(C₃H₇)-, -CH(CH(CH₃)₂)-, -(CH(CH₃))₂-, -CH₂-CH(CH₃)-, -CH(CH₃)-CH₂- and the like.

In the present specification, the "divalent cyclic amino group" means a divalent group obtained by removing one H from the amino group (-NH-) and one H from the other moiety of a cyclic amine, specifically has a structure represented by the following. 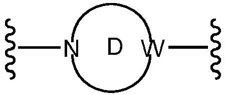 wherein
Ring D is a 3- to 8-membered saturated or unsaturated cyclic amine optionally containing, in addition to one nitrogen atom, hetero atom(s) selected from an oxygen atom, a sulfur atom or a nitrogen atom, and optionally having substituent(s), and
W is a carbon atom, CR^{h} wherein R^{h} is a hydrogen atom or a C₁₋₃ alkyl group, or a nitrogen atom.

W is preferably a nitrogen atom. That is, the "divalent cyclic amino group" is preferably a divalent cyclic diamino group.

Specific examples of the divalent cyclic diamino group include a divalent 3- to 8-membered (preferably 5- to 8-membered, more preferably 6-membered) cyclic diamino group such as piperazine-1,4-diyl, dihydropyrazine-1,4-diyl, tetrahydropyrazine-1,4-diyl, tetrahydropyrimidine-1,3-diyl, hexahydropyrimidine-1,3-diyl, dihydropyridazine-1,2-diyl, tetrahydropyridazine-1,2-diyl, hexahydropyridazine-1,2-diyl, 1,2-diazepane-1,2-diyl, 1,3-diazepane-1,3-diyl, 1,4-diazepane-1,4-diyl, dihydro-1,2-diazepine-1,2-diyl, tetrahydro-1,2-diazepine-1,2-diyl, hexahydro-1,2-diazepine-1,2-diyl, dihydro-1,3-diazepine-1,3-diyl, tetrahydro-1,3-diazepine-1,3-diyl, hexahydro-1,3-diazepine-1,3-diyl, tetrahydro-1,4-diazepine-1,4-diyl, hexahydro-1,4-diazepine-1,4-diyl, 1,2-diazocane-1,2-diyl, 1,3-diazocane-1,3-diyl, 1,4-diazocane-1,4-diyl, 1,5-diazocane-1,5-diyl, pyrazoline-1,2-diyl, pyrazolidine-1,2-diyl, imidazoline-1,3-diyl, imidazolidine-1,3-diyl and the like. Among them, preferred is a divalent 3- to 8-membered (preferably 5- to 8-membered, more preferably 6-membered) cyclic saturated diamino group, and particularly preferred is piperazine-1,4-diyl.

In the present specification, the "amino acid residue" means a divalent group obtained by removing H from the amino group and OH from the carboxy group of an amino acid.

The amino acid of the "amino acid residue" is not particularly limited as long as it has an amino group and a carboxy group, and it may be a natural type (L-type) or an unnatural type (D-type), or may be an artificial amino acid. Further, the amino acid may be an α-amino acid, a β-amino acid, a γ-amino acid or the like. The "amino acid residue" may be a residue derived from a cyclic amino acid, as shown below. The "amino acid residue" may be a residue of an amino acid as shown below. 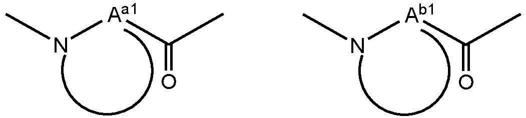 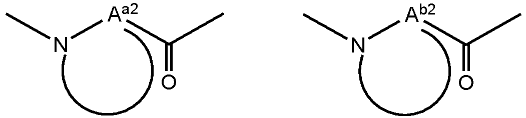 wherein each symbol is as defined above.

Examples of the α-amino acid include glycine, alanine, valine, leucine, isoleucine, serine, threonine, cysteine, methionine, glutamic acid, aspartic acid, lysine, arginine, histidine, glutamine, asparagine, phenylalanine, tyrosine, α-methyltyrosine, tryptophan, ornithine, thyroxine, proline, 3,4-dihydroxyphenylalanine, 3-(1-naphthyl)alanine, 3-(2-naphthyl)alanine, α-aminobutyric acid, norvaline, norleucine, homonorleucine, 1,2,4-triazole-3-alanine, 2-aminoadipic acid, propargylglycine, allylglycine, α-cyclobutylmethylglycine, 6-azidonorleucine, 4-azidophenylalanine, 4-fluoroglutamic acid, 4-iodophenylalanine and the like;
examples of the β-amino acid include β-alanine, 3-aminoadipic acid and the like; and
examples of the γ-amino acid include γ-aminobutyric acid and the like.

When the aforementioned amino acid has a functional group in its side chain, the functional group may be protected/modified. Examples of such amino acid include δ-Boc-lysine, δ-Z-lysine, δ-Fmoc-lysine, β-Bn-aspartic acid, γ-Bn-glutamic acid and the like. Divalent groups obtained by removing H from the amino group and OH from the carboxy group of such amino acids are also included in the "amino acid residue".

The aforementioned amino acid may have a substituent in its side chain. Examples of such amino acid include 4-azidophenylalanine, 3-azidophenylalanine, 3-azidotyrosine and 2-azidotyrosine, and α-methylated amino acids thereof, and the like. Divalent groups obtained by removing H from the amino group and OH from the carboxy group of such amino acids are also included in the "amino acid residue".

The configuration of the aforementioned amino acid is not particularly limited, and may be any of D-form, L-form and DL-form (i.e., any of R-form, S-form and R/S-form).

In the present specification, the "divalent residue derived from an aminosaccharide or a derivative thereof" means a divalent group obtained by removing H from the amino group and H from the hydroxy group of an aminosaccharide or a derivative thereof.

The aminosaccharide of the "divalent residue derived from an aminosaccharide or a derivative thereof" refers to a saccharide in which at least one of its hydroxy group is replaced with an amino group, and examples thereof include monosaccharides such as glucosamine, galactosamine and the like, and disaccharides or higher saccharides containing these monosaccharides, and the aminosaccharides are not particularly limited as long as they are saccharides having a hydroxyl group and an amino group. The aminosaccharide derivatives include those in which the hydroxyl group (not involved in the bond) of the above aminosaccharide is protected/modified.

The configuration of the above aminosaccharide or derivative thereof is not particularly limited.

In the present specification, the "boryl group (-B(OH)₂)" is also referred to as a dihydroxyboryl group.

In the present specification, examples of the "ester group of a boryl group" include the following groups. 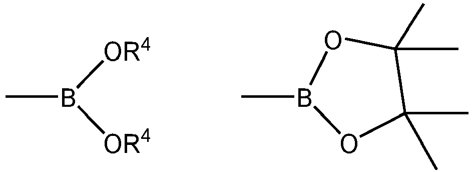 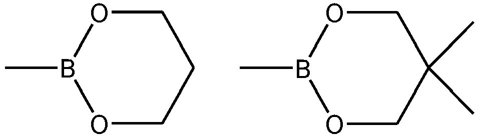 wherein R⁴ is a C₁₋₆ alkyl group.

In the present specification, the "protected amino acid residue" means an amino acid residue in which, when the amino acid residue has a functional group, the functional group is protected. When it has an amino group, it is protected with an amino-protecting group such as a tert-butoxycarbonyl group (Boc group), and when it has a carboxyl group, it is protected with a carboxy-protecting group such as a tert-butyl group. These protecting groups are appropriately selected depending on the type of other protecting groups and resin for solid phase synthesis, the synthesis strategy, and the like.

In the present specification, examples of the "hydroxy-protecting group" include a benzyl group, a p-methoxybenzyl group, a methoxymethyl group, a trimethylsilyl group, a triethylsilyl group, a trityl group, a tert-butyl group, a tertbutyldimethylsilyl group (TBS group), a tetrahydropyranyl group, a benzylidene group (formation of benzylideneacetal), an isopropylidene group (formation of dimethylacetal), an acetyl group, a benzoyl group and the like.

In the present specification, examples of the "amino-protecting group" include a 9-fluorenylmethyloxycarbonyl group (Fmoc group), a tert-butoxycarbonyl group (Boc group), a benzyloxycarbonyl group (Cbz group), a trichloroethoxycarbonyl group (Troc group) and the like.

In the present specification, examples of the "carboxy-protecting group" include a tert-butyl group, a benzyl group, a C₁₋₂ alkyl group (a methyl group, an ethyl group) and the like.

In the present specification, examples of the "mercapto-protecting group" include a benzyl group, a p-methoxybenzyl group, a methoxymethyl group, a trimethylsilyl group, a triethylsilyl group, a trityl group, a tert-butyl group, a tertbutyldimethylsilyl group (TBS group), a tetrahydropyranyl group, an isopropylidene group (formation of dimethylthioacetal), an acetyl group, a benzoyl group and the like.

The conjugate of the present invention comprises
a radioactive moiety comprising an aryl group substituted with a radionuclide selected from ²¹¹At, ²¹⁰At, ¹³¹I, ¹²⁵I, ¹²⁴I, ¹²³I, ⁷⁷Br and ⁷⁶Br, and
a bioactive moiety having an affinity for fibroblast activation protein α (FAPα).

In the above-mentioned radioactive moiety, the aryl group is preferably a C₆₋₁₄ aryl group, more preferably a phenyl group.

The aryl group is substituted with a radionuclide selected from ²¹¹At (α-ray emitting nuclide), ²¹⁰At (α-ray emitting nuclide), ¹³¹I (β-ray emitting nuclide), ¹²⁵I (X-ray emitting nuclide), ¹²⁴I (positron emitting nuclide), ¹²³I (γ-ray emitting nuclide), ⁷⁷Br (auger electron emitting nuclide) and ⁷⁶Br (positron emitting nuclide). The substitution position is not particularly limited. For example, when the aryl group is a phenyl group, the 3-position or the 4-position is preferred.

The radioactive moiety comprising such an aryl group preferably comprises an aryl-C₀₋₃ alkyl group (preferably an aryl-C₁₋₃ alkyl group, more preferably a phenyl-C₁₋₃ alkyl group), wherein the aryl is substituted with a radionuclide selected from ²¹¹At, ²¹⁰At, ¹³¹I, ¹²⁵I, ¹²⁴I, ¹²³I, ⁷⁷Br and ⁷⁶Br.

Specifically, a radioactive moiety having a structure represented by the following formula: 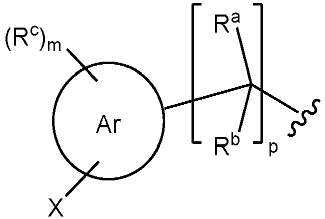 wherein
X is a radionuclide selected from ²¹¹At, ²¹⁰At, ¹³¹I, ¹²⁵I, ¹²⁴I, ¹²³I, ⁷⁷Br and ⁷⁶Br;
Ar is a C₆₋₁₄ aryl group;
R^{a} and R^{b} in the number of p are each independently a hydrogen atom or a C₁₋₆ alkyl group;
R^{c} in the number of m are each independently a substituent (for example, a C₁₋₆ alkyl group or a hydroxy group);
p is an integer of 0 to 3 (preferably an integer of 1 to 3); and m is an integer of 0 to 3,
is preferred.

The above-mentioned bioactive moiety is not limited as long as it has an affinity for fibroblast activation protein α, and examples thereof include moieties containing part or all of the compounds disclosed in WO 2019/154866, WO 2019/083990 and the like. Specifically, a moiety having a structure represented by the following formula: 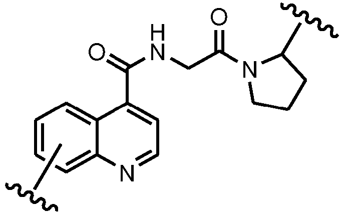 is preferred.

In one embodiment, the above-mentioned structure is preferably a structure represented by formula:

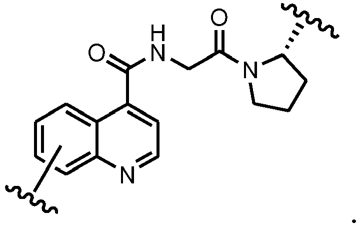

One embodiment of the conjugate of the present invention includes Radiolabeled Compound (I-1). 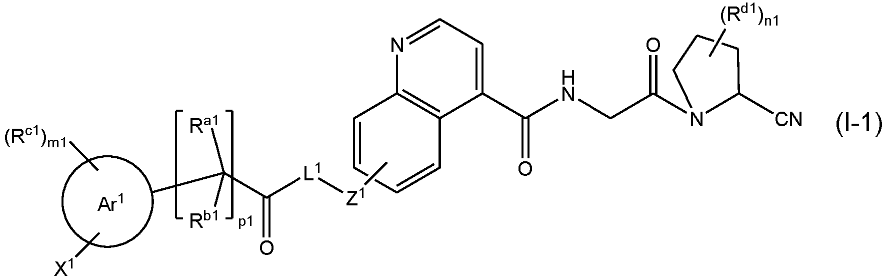 wherein each symbol in the formula is as defined above.

X¹ is a radionuclide selected from ²¹¹At (α-ray emitting nuclide), ²¹⁰At (α-ray emitting nuclide), ¹³¹I (β-ray emitting nuclide), ¹²⁵I (X-ray emitting nuclide), ¹²⁴I (positron emitting nuclide), ¹²³I (γ-ray emitting nuclide), ⁷⁷Br (auger electron emitting nuclide) and ⁷⁶Br (positron emitting nuclide).

The half-lives of these radionuclides are 7.2 hours for ²¹¹At, 8.3 hours for ²¹⁰At, 8.04 days for ¹³¹I, 59.4 days for ¹²⁵I, 4.2 days for ¹²⁴I, 13.2 hours for ¹²³I, 57 hours for ⁷⁷Br, and 16 hours for ⁷⁶Br.

The bonding position of X¹ on Ar¹ is not particularly limited. For example, when Ar¹ is a phenyl group, the 3-position or the 4-position is preferred.

Ar¹ is a C₆₋₁₄ aryl group.

In one embodiment, Ar¹ is preferably a phenyl group.

R^{c1} in the number of m1 are each independently a C₁₋₆ alkyl group (e.g., methyl) or a hydroxy group.

m1 is an integer of 0 to 3.

In one embodiment, m1 is preferably 0.

R^{a1} and R^{b1} in the number of p1 are each independently a hydrogen atom or a C₁₋₆ alkyl group.

In one embodiment, R^{a1} and R^{b1} in the number of p1 are preferably each a hydrogen atom.

p1 is an integer of 1 to 3.

In one embodiment, p1 is preferably 1 or 2 in terms of reaction yield and stability.

R^{d1} in the number of n1 are each independently a halogen atom.

In one embodiment, R^{d1} in the number of n1 are preferably each a fluorine atom.

n1 is an integer of 0 to 3.

In one embodiment, n1 is preferably an integer of 0 to 2.

Z¹ is an oxygen atom, a sulfur atom or NR^{f1} wherein the symbol is as defined above.

L¹ is
(1) a linker represented by *-L^{d1}-L^{c1}-L^{b1}-L^{a1}-**
   wherein each symbol is as defined above, or
(2) a linker represented by *-(NH-A^{b1}-CO)ₛ₁-**
   wherein each symbol is as defined above.

Preferred combinations of Z¹ and L¹ include the following embodiments (A1) and (B1).
(A1) An embodiment in which
   Z¹ is an oxygen atom or a sulfur atom (preferably an oxygen atom), and
   L¹ is a linker represented by *-L^{d1}-L^{c1}-L^{b1}-L^{a1}-** wherein each symbol is as defined above.
(B1) An embodiment in which
   Z¹ is NR^{f1} wherein the symbol is as defined above, and
   L¹ is a linker represented by *-(NH-A^{b1}-CO)ₛ₁-** wherein the symbol is as defined above.

In embodiment (A1), preferred embodiments of L¹ include the following embodiments (A1-1) and (A1-2).

(A1-1) An embodiment in which L¹ is a linker represented by *-L^{d1}-L^{c1}-L^{b1}-L^{a1}-**
   wherein
   * indicates a binding site to CO,
   ** indicates a binding site to Z¹,
   L^{a1} is a C₁₋₆ alkylene group,
   L^{b1} is a bond or -CO-,
   L^{c1} is a divalent cyclic amino group, and
   L^{d1} is
      (i) a bond,
      (ii) *-(NH-A^{a1}-CO)ᵣ₁-*** wherein each symbol is as defined above, or
      (iii) *-NH-B^{a1}-O-B^{b1}-CO-*** wherein each symbol is as defined above.
(A1-2) An embodiment in which L¹ is a linker represented by *-L^{d1}-L^{c1}-L^{b1}-L^{a1}-**
   wherein
   * indicates a binding site to CO,
   ** indicates a binding site to Z¹,
   L^{a1} is -CH₂-(CH₂-O-CH₂)_{q1}-CH₂- wherein the symbol is as defined above,
   L^{b1} is a bond,
   L^{c1} is NR^{g1} wherein the symbol is as defined above, an oxygen atom or a sulfur atom, and
   L^{d1} is
      (i) a bond, or
      (ii) *-NH-B^{a1}-O-B^{b1}-CO-*** wherein each symbol is as defined above.

In embodiment (A1-1), L^{a1} is a C₁₋₆ alkylene group.

In one embodiment, L^{a1} is preferably a C₂₋₄ alkylene group.

In embodiment (A1-1), L^{c1} is a divalent cyclic amino group.

In one embodiment, L^{c1} is preferably a divalent 3- to 8-membered (preferably 5- to 8-membered, more preferably 6-membered) cyclic diamino group.

In one embodiment, L^{c1} is more preferably a divalent 3- to 8-membered (preferably 5- to 8-membered, more preferably 6-membered) saturated cyclic diamino group, particularly preferably piperazine-1,4-diyl.

The "divalent cyclic amino group" is bonded to L^{b1} and L^{d1} in Formula (I-1), and the following structure 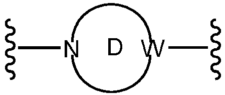 may be either an embodiment in which the nitrogen atom is bonded to L^{b1}, and W is bonded to L^{d1}, or an embodiment in which the nitrogen atom is bonded to L^{d1}, and W is bonded to L^{b1}, preferably an embodiment in which the nitrogen atom is bonded to L^{d1}, and W is bonded to L^{b1}.

In embodiment (A1-1), preferred embodiments include the following embodiments (A1-1-1) to (A1-1-5).

(A1-1-1) An embodiment in which L¹ is a linker represented by *-L^{d1}- L^{c1}- L^{b1}- L^{a1}-**
   wherein
   * indicates a binding site to CO,
   ** indicates a binding site to Z¹,
   L^{a1} is a C₁₋₆ alkylene group (preferably a C₂₋₄ alkylene group),
   L^{b1} is a bond,
   L^{c1} is a divalent cyclic amino group (preferably a divalent 3- to 8-membered (preferably 5- to 8-membered, more preferably 6-membered) cyclic diamino group, more preferably a divalent 3- to 8-membered (preferably 5- to 8-membered, more preferably 6-membered) saturated cyclic diamino group, particularly preferably piperazine-1,4-diyl), and
   L^{d1} is a bond.
(A1-1-2) An embodiment in which L¹ is a linker represented by *-L^{d1}- L^{c1}- L^{b1}- L^{a1}-**
   wherein
   * indicates a binding site to CO,
   ** indicates a binding site to Z¹,
   L^{a1} is a C₁₋₆ alkylene group (preferably a C₂₋₄ alkylene group),
   L^{b1} is a bond,
   L^{c1} is a divalent cyclic amino group (preferably a divalent 3- to 8-membered (preferably 5- to 8-membered, more preferably 6-membered) cyclic diamino group, more preferably a divalent 3- to 8-membered (preferably 5- to 8-membered, more preferably 6-membered) saturated cyclic diamino group, particularly preferably piperazine-1,4-diyl), and
   L^{d1} is *-NH-B^{a1}-O-B^{b1}-CO-*** wherein each symbol is as defined above.

In this embodiment, -NH-B^{a1}-O- is a divalent residue derived from an aminosaccharide or a derivative thereof.

In one embodiment, -NH-B^{a1}-O- is preferably a divalent residue derived from a monosaccharide such as glucosamine, galactosamine and the like, more preferably a divalent residue derived from glucosamine.

B^{b1} is a C₁₋₆ alkylene group.

In one embodiment, B^{b1} is preferably a C₂₋₄ alkylene group.

(A1-1-3) An embodiment in which L¹ is a linker represented by *-L^{d1}-L^{c1}-L^{b1}-L^{a1}-**
   wherein
   * indicates a binding site to CO,
   ** indicates a binding site to Z¹,
   L^{a1} is a C₁₋₆ alkylene group (preferably a C₂₋₄ alkylene group),
   L^{b1} is -CO-,
   L^{c1} is a divalent cyclic amino group (preferably a divalent 3- to 8-membered (preferably 5- to 8-membered, more preferably 6-membered) cyclic diamino group, more preferably a divalent 3- to 8-membered (preferably 5- to 8-membered, more preferably 6-membered) saturated cyclic diamino group, particularly preferably piperazine-1,4-diyl), and
   L^{d1} is a bond.
(A1-1-4) An embodiment in which L¹ is a linker represented by *-L^{d1}- L^{c1}- L^{b1}- L^{a1}-**
   wherein
   * indicates a binding site to CO,
   ** indicates a binding site to Z¹,
   L^{a1} is a C₁₋₆ alkylene group (preferably a C₂₋₄ alkylene group),
   L^{b1} is a bond,
   L^{c1} is a divalent cyclic amino group (preferably a divalent 3- to 8-membered (preferably 5- to 8-membered, more preferably 6-membered) cyclic diamino group, more preferably a divalent 3- to 8-membered (preferably 5- to 8-membered, more preferably 6-membered) saturated cyclic diamino group, particularly preferably piperazine-1,4-diyl), and
   L^{d1} is *-(NH-A^{a1}-CO)ᵣ₁-*** wherein each symbol is as defined above.
(A1-1-5) An embodiment in which L¹ is a linker represented by *-L^{d1}-L^{c1}-L^{b1}-L^{a1}-**
   wherein
   * indicates a binding site to CO,
   ** indicates a binding site to Z¹,
   L^{a1} is a C₁₋₆ alkylene group (preferably a C₂₋₄ alkylene group),
   L^{b1} is -CO-,
   L^{c1} is a divalent cyclic amino group (preferably a divalent 3- to 8-membered (preferably 5- to 8-membered, more preferably 6-membered) cyclic diamino group, more preferably a divalent 3- to 8-membered (preferably 5- to 8-membered, more preferably 6-membered) saturated cyclic diamino group, particularly preferably piperazine-1,4-diyl), and
   L^{d1} is *-(NH-A^{a1}CO)ᵣ₁-*** wherein each symbol is as defined above.

In embodiments (A1-1-4) and (A1-1-5), NH-A^{a1}-CO in the number of r1 are each independently an amino acid residue.

In one embodiment, NH-A^{a1}-CO in the number of r1 are preferably each a 4-azidophenylalanine residue.

r1 is an integer of 1 to 3.

In one embodiment, r1 is preferably 1.

In one embodiment, L^{d1} is preferably a 4-azidophenylalanine residue.

In embodiment (A1-2), L^{a1} is -CH₂-(CH₂-O-CH₂)_{q1}-CH₂- wherein q1 is an integer of 0 to 5.

In one embodiment, q1 is preferably an integer of 1 to 3.

In embodiment (A1-2), L^{c1} is NR^{g1} wherein R^{g1} is a hydrogen atom or a C₁₋₃ alkyl group, an oxygen atom or a sulfur atom.

In one embodiment, R^{g1} is preferably a hydrogen atom.

In one embodiment, L^{c1} is preferably NR^{g1} wherein the symbol is as defined above, more preferably NH.

In embodiment (A1-2), preferred embodiments include the following embodiments (A1-2-1) and (A1-2-2).

(A1-2-1) An embodiment in which L¹ is a linker represented by *-L^{d1}-L^{c1}-L^{b1}-L^{a1}-**
   wherein
   * indicates a binding site to CO,
   ** indicates a binding site to Z¹,
   L^{a1} is -CH₂-(CH₂-O-CH₂)_{q1}-CH₂- wherein the symbol is as defined above,
   L^{b1} is a bond,
   L^{c1} is NR^{g1} wherein the symbol is as defined above, and
   L^{d1} is a bond.
(A1-2-2) An embodiment in which L¹ is a linker represented by *-L^{d1}- L^{c1}- L^{b1}- L^{a1}-**
   wherein
   * indicates a binding site to CO,
   ** indicates a binding site to Z¹,
   L^{a1} is -CH₂-(CH₂-O-CH₂)_{q1}-CH₂- wherein the symbol is as defined above,
   L^{b1} is a bond,
   L^{c1} is NR^{g1} wherein the symbol is as defined above, and
   L^{d1} is *-NH-B^{a1}-O-B^{b1}-CO-*** wherein each symbol is as defined above.

In this embodiment, -NH-B^{a1}-O- is a divalent residue derived from an aminosaccharide or a derivative thereof.

In one embodiment, -NH-B^{a1}-O- is preferably a divalent residue derived from a monosaccharide such as glucosamine, galactosamine and the like, more preferably a divalent residue derived from glucosamine.

B^{b1} is a C₁₋₆ alkylene group.

In one embodiment, B^{b1} is preferably a C₂₋₄ alkylene group.

In embodiment (B1), Z¹ is NR^{f1} wherein R^{f1} is a hydrogen atom or a C₁₋₃ alkyl group.

In one embodiment, R^{f1} is preferably a hydrogen atom.

In one embodiment, Z¹ is preferably NH.

In embodiment (B1), L¹ is *-(NH-A^{b1}-CO)ₛ₁-** wherein NH-A^{b1}-CO in the number of s1 are each independently an amino acid residue, and s1 is an integer of 1 to 3.

In one embodiment, NH-A^{b1}-CO in the number of s1 is preferably a glycine residue.

In one embodiment, s1 is preferably 1.

In one embodiment, L¹ is preferably a glycine residue.

The configuration of the chiral C atom in Radiolabeled Compound (I-1) may be either S or R. Radiolabeled Compound (I-1) has optical isomers based on the chiral C atom, and all optical isomers and mixtures thereof in any ratio are included in Radiolabeled Compound (I-1).

In one embodiment, Compound (I-1) preferably has a configuration represented by the following Formula (I'-1):

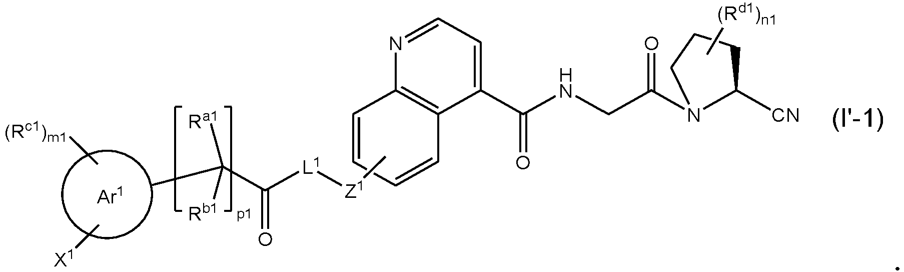

Specific preferred examples of Radiolabeled Compound (I-1) include the following.

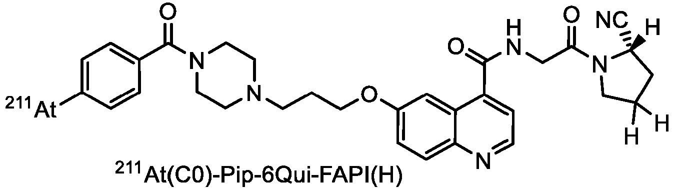

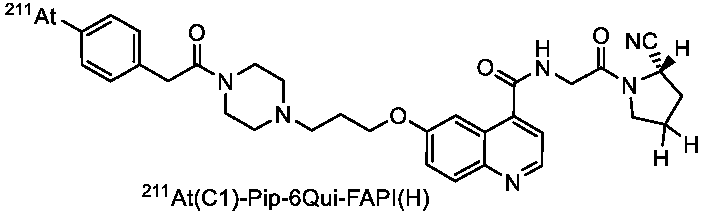

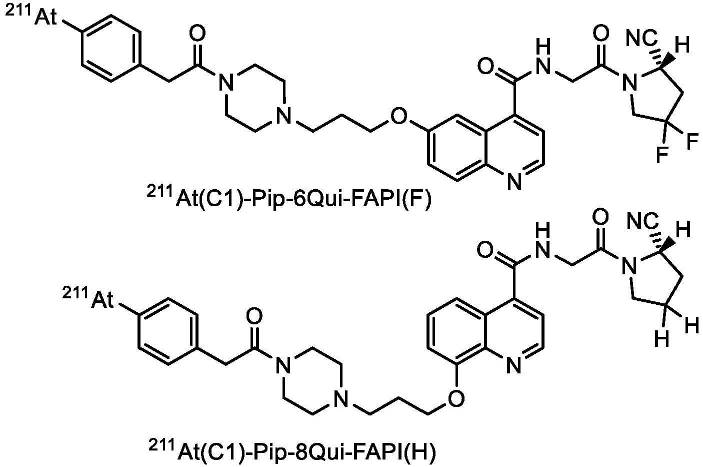

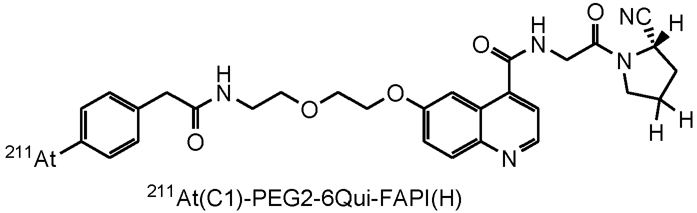

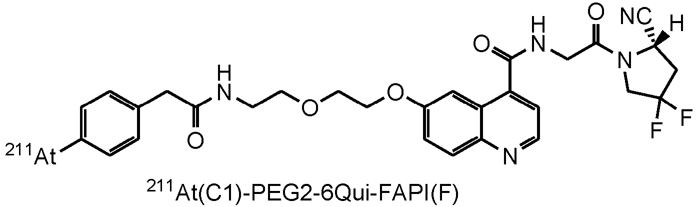

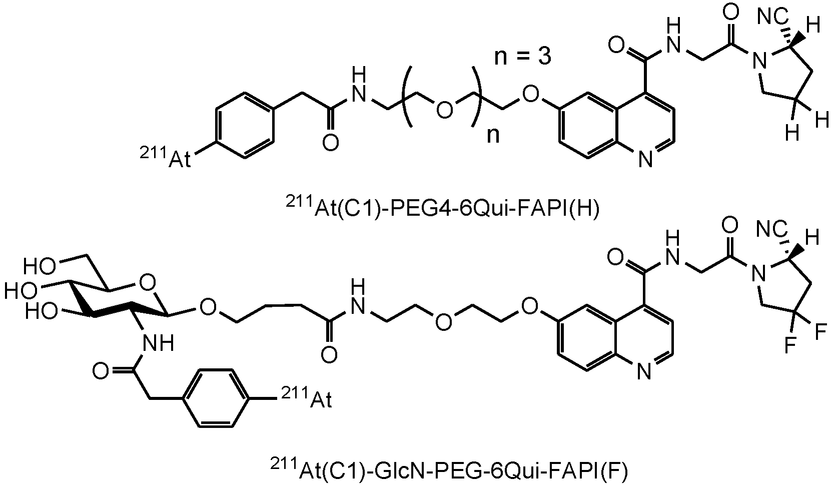

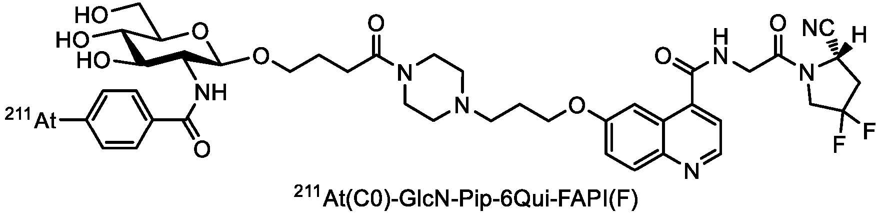

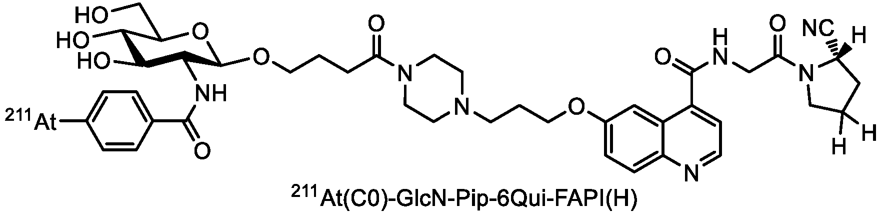

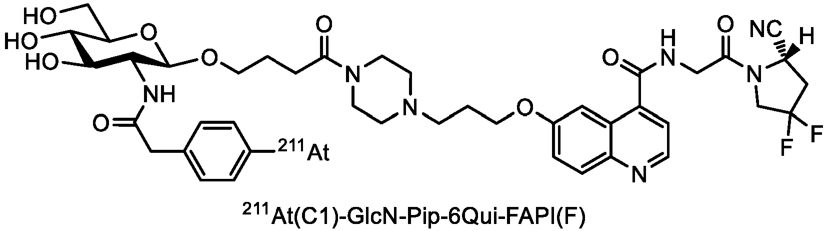

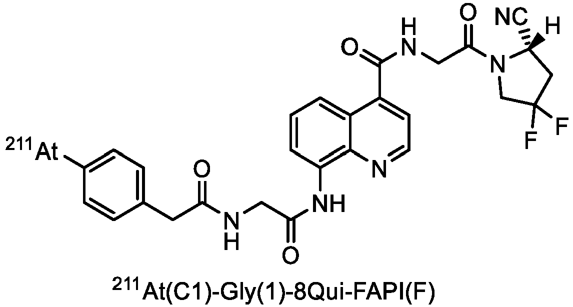

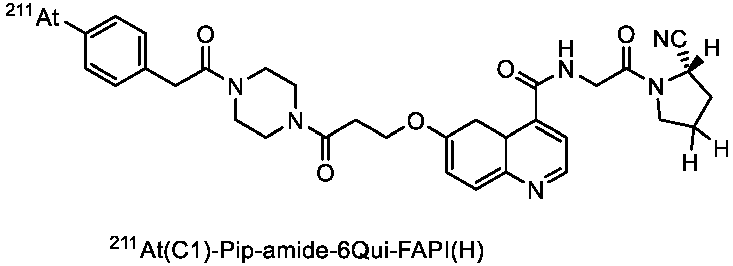

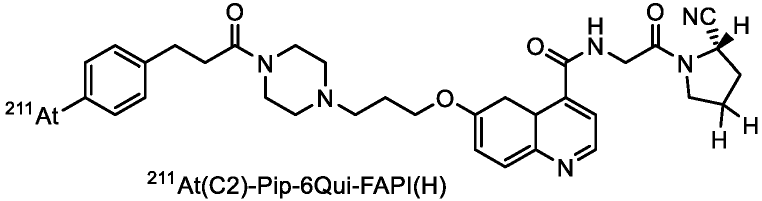

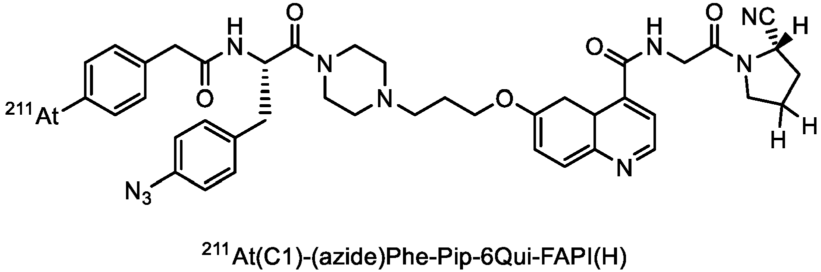

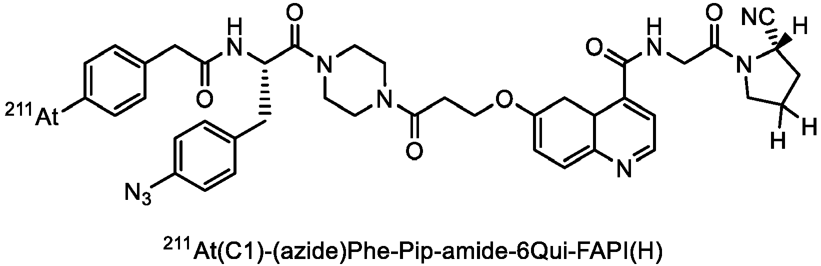

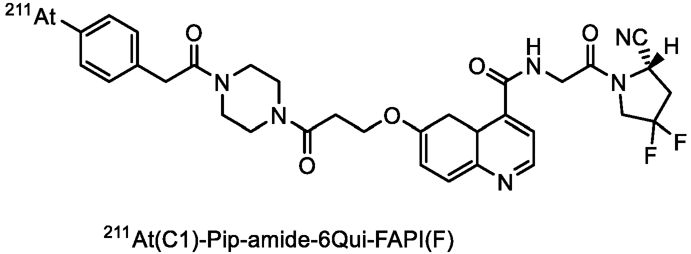

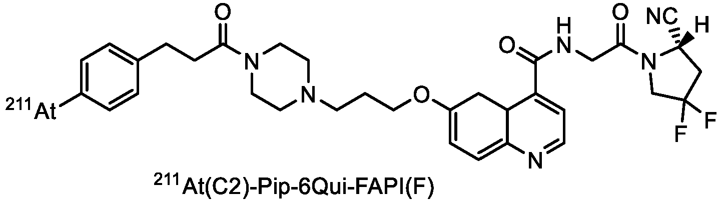

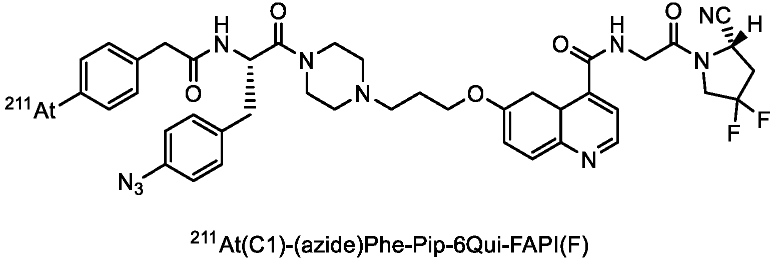

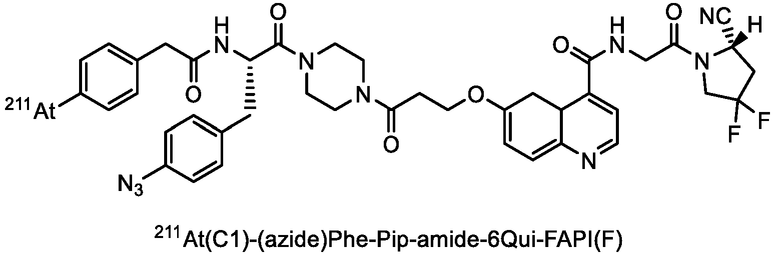

Another embodiment of the conjugate of the present invention includes Radiolabeled Compound (I-2). 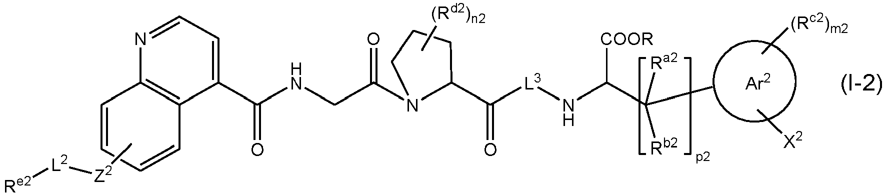 wherein each symbol in the formula is as defined above.

X² is a radionuclide selected from ²¹¹At (α-ray emitting nuclide), ²¹⁰At (α-ray emitting nuclide), ¹³¹I (β-ray emitting nuclide), ¹²⁵I (X-ray emitting nuclide), ¹²⁴I (positron emitting nuclide), ¹²³I (γ-ray emitting nuclide), ⁷⁷Br (auger electron emitting nuclide) and ⁷⁶Br (positron emitting nuclide).

The half-lives of these radionuclides are 7.2 hours for ²¹¹At, 8.3 hours for ²¹⁰At, 8.04 days for 1311, 59.4 days for ¹²⁵I, 4.2 days for ¹²⁴I, 13.2 hours for ¹²³I, 57 hours for ⁷⁷Br, and 16 hours for ⁷⁶Br.

The bonding position of X² on Ar² is not particularly limited. For example, when Ar² is a phenyl group, the 3-position or the 4-position is preferred.

Ar² is a C₆₋₁₄ aryl group.

In one embodiment, Ar² is preferably a phenyl group.

R^{c2} in the number of m2 are each independently a C₁₋₆ alkyl group (e.g., methyl) or a hydroxy group.

m2 is an integer of 0 to 3.

In one embodiment, m2 is preferably 0.

R^{a2} and R^{b2} in the number of p2 are each independently a hydrogen atom or a C₁₋₆ alkyl group.

In one embodiment, R^{a2} and R^{b2} in the number of p2 are preferably each a hydrogen atom.

p2 is an integer of 0 to 3.

In one embodiment, p2 is preferably an integer of 1 to 3, more preferably 1.

R^{d2} in the number of n2 are each independently a halogen atom.

In one embodiment, R^{d2} in the number of n2 are preferably a fluorine atom.

n2 is an integer of 0 to 3.

In one embodiment, n2 is preferably an integer of 0 to 2.

Z² is an oxygen atom, a sulfur atom or NR^{f2} wherein the symbol is as defined above.

L² is
(1) a linker represented by *-L^{c2}-L^{b2}-L^{a2}-**
   wherein each symbol is as defined above, or
(2) a linker represented by *-(NH-A^{a2}-CO)ᵣ₂-**
   wherein each symbol is as defined above.

Preferred combinations of Z² and L² include the following embodiments (A2) and (B2).
(A2) An embodiment in which
   Z² is an oxygen atom or a sulfur atom, and
   L² is a linker represented by *-L^{c2}-L^{b2}-L^{a2}-** wherein each symbol is as defined above.
(B2) An embodiment in which
   Z² is NR^{f2} wherein the symbol is as defined above, and
   L² is a linker represented by *-(NH-A^{a2}-CO)ᵣ₂-** wherein each symbol is as defined above.

In embodiment (A2), preferred embodiments of L² include the following embodiments (A2-1) and (A2-2).

(A2-1) An embodiment in which L² is a linker represented by *-L^{c2}-L^{b2}-L^{a2}-**
   wherein
   * indicates a binding site to CO,
   ** indicates a binding site to Z²,
   L^{a2} is a C₁₋₆ alkylene group,
   L^{b2} is a bond or -CO-, and
   L^{c2} is a divalent cyclic amino group.
(A2-2) An embodiment in which L² is a linker represented by *-L^{c2}-L^{b2}-L^{a2}-**
   wherein
   * indicates a binding site to CO,
   ** indicates a binding site to Z²,
   L^{a2} is -CH₂-(CH₂-O-CH₂)_{q2}-CH₂- wherein the symbol is as defined above,
   L^{b2} is a bond, and
   L^{c2} is NR^{g2} wherein the symbol is as defined above, an oxygen atom or a sulfur atom.

In embodiment (A2-1), L^{a2} is a C₂₋₆ alkylene group.

In one embodiment, L^{a2} is preferably a C₂₋₄ alkylene group.

In embodiment (A2-1), L^{c2} is a divalent cyclic amino group.

In one embodiment, L^{c2} is preferably a divalent 3- to 8-membered (preferably 5- to 8-membered, more preferably 6-membered) cyclic diamino group.

In one embodiment, L^{c2} is more preferably a divalent 3- to 8-membered (preferably 5- to 8-membered, more preferably 6-membered) saturated cyclic diamino group, particularly preferably piperazine-1,4-diyl.

The "divalent cyclic amino group" is bonded to L^{b2} and R^{e2} in Formula (I-2), and the following structure 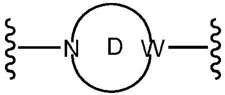 may be either an embodiment in which the nitrogen atom is bonded to L^{b2}, and W is bonded to R^{e2}, or an embodiment in which the nitrogen atom is bonded to R^{e2}, and W is bonded to L^{b2}, preferably an embodiment in which the nitrogen atom is bonded to R^{e2}, and W is bonded to L^{b2}.

In embodiment (A2-1), preferred embodiments include the following embodiments (A2-1-1) to (A2-1-2).

(A2-1-1) An embodiment in which L² is a linker represented by *-L^{c2}-L^{b2}-L^{a2}-**
   wherein
   * indicates a binding site to CO,
   ** indicates a binding site to Z²,
   L^{a2} is a C₁₋₆ alkylene group (preferably a C₂₋₄ alkylene group),
   L^{b2} is a bond, and
   L^{c2} is a divalent cyclic amino group (preferably a divalent 3- to 8-membered (preferably 5- to 8-membered, more preferably 6-membered) cyclic diamino group, more preferably a divalent 3- to 8-membered (preferably 5- to 8-membered, more preferably 6-membered) saturated cyclic diamino group, particularly preferably piperazine-1,4-diyl) .
(A2-1-2) An embodiment in which L² is a linker represented by *-L^{c2}-L^{b2}-L^{a2}-**
   wherein
   * indicates a binding site to CO,
   ** indicates a binding site to Z²,
   L^{a2} is a C₁₋₆ alkylene group (preferably a C₂₋₄ alkylene group),
   L^{b2} is -CO-, and
   L^{c2} is a divalent cyclic amino group (preferably a divalent 3- to 8-membered (preferably 5- to 8-membered, more preferably 6-membered) cyclic diamino group, more preferably a divalent 3- to 8-membered (preferably 5- to 8-membered, more preferably 6-membered) saturated cyclic diamino group, particularly preferably piperazine-1,4-diyl) .

In embodiment (A2-2), L^{a2} is -CH₂-(CH₂-O-CH₂)_{q2}-CH₂- wherein q2 is an integer of 0 to 5.

In one embodiment, q2 is preferably an integer of 1 to 3.

In embodiment (A2-2), L^{c2} is NR^{g2} wherein R^{g2} is a hydrogen atom or a C₁₋₃ alkyl group, an oxygen atom or a sulfur atom.

In one embodiment, R^{g2} is preferably a hydrogen atom.

In one embodiment, L^{c2} is preferably NR^{g2} wherein the symbol is as defined above, more preferably NH.

In embodiment (B2), Z² is NR^{f2} wherein R^{f2} is a hydrogen atom or a C₁₋₃ alkyl group.

In one embodiment, R^{f2} is preferably a hydrogen atom.

In one embodiment, Z² is preferably NH.

In embodiment (B2), L² is *-(NH-A^{a2}-CO)ᵣ₂-** wherein NH-A^{a2}-CO in the number of r2 are each independently an amino acid residue, and r2 is an integer of 1 to 3.

In one embodiment, NH-A^{a2}-CO in the number of r2 are preferably each a glycine residue.

In one embodiment, r2 is preferably 1.

In one embodiment, L² is preferably a glycine residue.

R^{e2} is a C₁₋₆ alkyl-carbonyl group.

In one embodiment, R^{e2} is preferably an acetyl group.

Alternatively, the group R^{e2}-L²-Z²- is a hydrogen atom.

L³ is a linker represented by ***-(NH-A^{b2}-CO)ₛ₂-****
wherein
*** indicates a binding site to CO,
**** indicates a binding site to NH,
NH-A^{b2}-CO in the number of s2 are each independently an amino acid residue, and
s2 is an integer of 0 to 3.

In one embodiment, s2 is preferably 0.

R is a hydrogen atom or a C₁₋₃ alkyl group.

In one embodiment, R is preferably a hydrogen atom.

The configuration of the chiral C atom in Radiolabeled Compound (I-2) may be either S or R. Radiolabeled Compound (I-2) has optical isomers based on the chiral C atom, and all optical isomers and mixtures thereof in any ratio are included in Radiolabeled Compound (I-2).

In one embodiment, Compound (I-2) preferably has a configuration represented by the following Formula (I'-2):

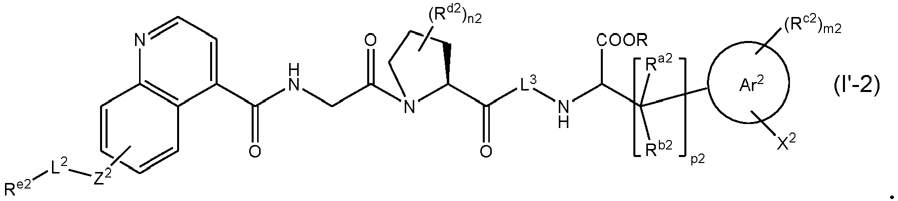

Specific preferred examples of Radiolabeled Compound (I-2) include the following.

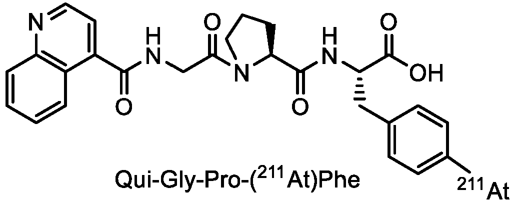

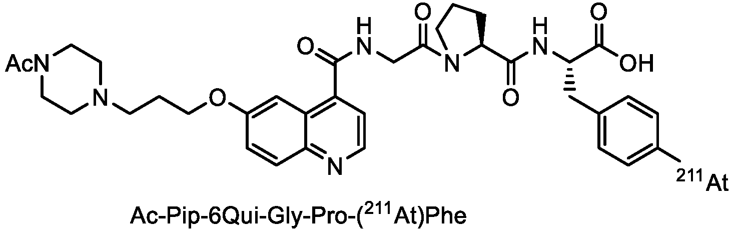

Compound (I-1) or (I-2) may be in the form of a pharmaceutically acceptable salts thereof. As the pharmaceutically acceptable salt, for example, when the compound has an acidic functional group, examples of the salt include inorganic salts such as alkali metal salts (e.g., sodium salt, potassium salt, etc.), alkaline-earth metal salts (e.g., calcium salt, magnesium salt, barium salt, etc.) and ammonium salts, and when the compound has a basic functional group, examples of the salt include salts with inorganic acids such as hydrogen chloride, hydrobromic acid, nitric acid, sulfuric acid and phosphoric acid, and salts with organic acids such as acetic acid, phthalic acid, fumaric acid, oxalic acid, tartaric acid, maleic acid, citric acid, succinic acid, methanesulfonic acid and p-toluenesulfonic acid.

The method for producing Radiolabeled Compound (I-1) or (I-2) of the present invention will be explained below.

In the present specification, when a raw material compound is in the form of a salt, examples of such salt include metal salts (e.g., alkali metal salts such as sodium salt and potassium salt; and alkaline-earth metal salts such as calcium salt, magnesium salt and barium salt), ammonium salts, salts with organic bases (e.g., trimethylamine, triethylamine, pyridine, picoline, 2,6-lutidine), salts with inorganic acids (e.g., hydrochloric acid, hydrobromic acid, nitric acid, sulfuric acid), salts with organic acids (e.g., formic acid, acetic acid, trifluoroacetic acid, phthalic acid, fumaric acid, oxalic acid, tartaric acid, maleic acid, citric acid, succinic acid, malic acid), and the like.

Radiolabeled Compound (I-1) can be produced by the following method comprising Step 1. 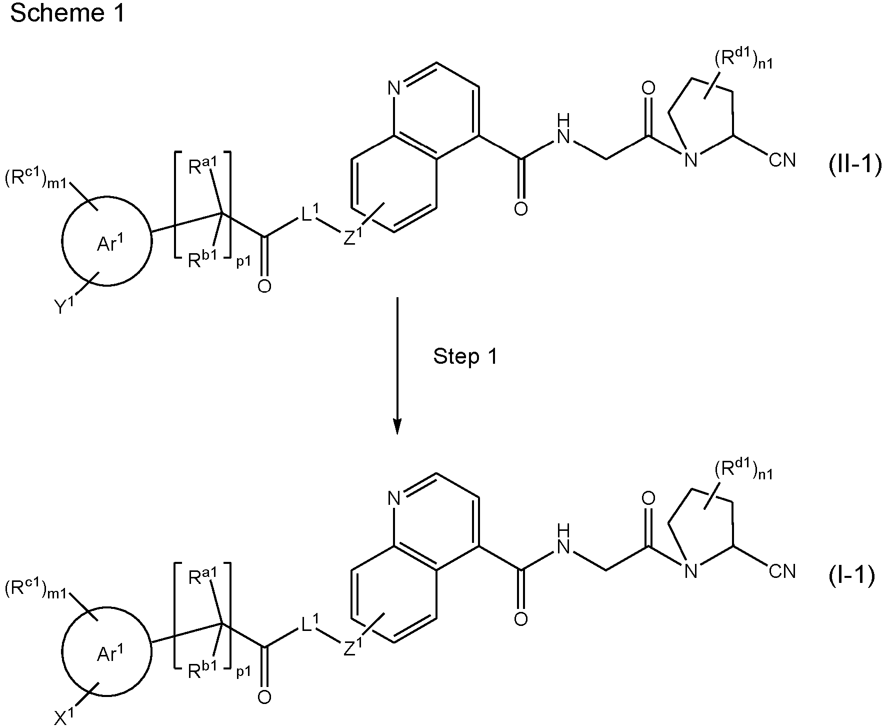 wherein each symbol in the formula is as defined above.

Y¹ is a boryl group (-B(OH)₂) or its ester group.

Y¹ is preferably a boryl group (-B(OH)₂) or a 4,4,5,5-tetramethyl-1,3,2-dioxaboran-2-yl group (a pinacol ester group).

Step 1 is a step of reacting Boronic Acid Compound (II-1) with a radionuclide selected from ²¹¹At, ²¹⁰At, ¹³¹I, ¹²⁵I, ¹²⁴I, ¹²³I, ⁷⁷Br and ⁷⁶Br in the presence of a reagent selected from an alkali metal iodide, an alkali metal bromide, N-bromosuccinimide, N-chlorosuccinimide, N-iodosuccinimide and hydrogen peroxide, in water to obtain Radiolabeled Compound (I-1).

Boronic Acid Compound (II-1) is a novel compound, and specific preferred examples thereof include the following.

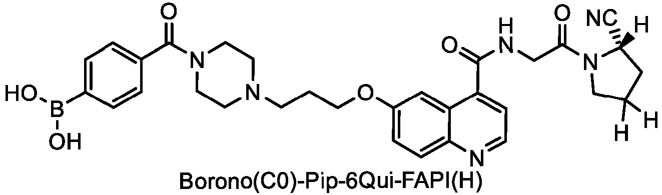

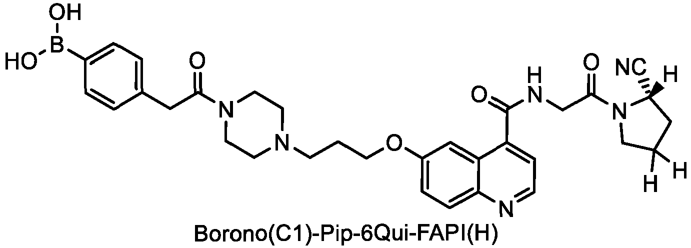

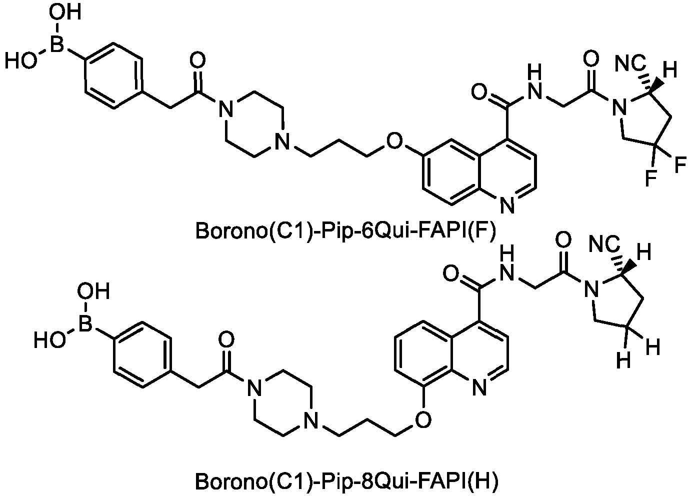

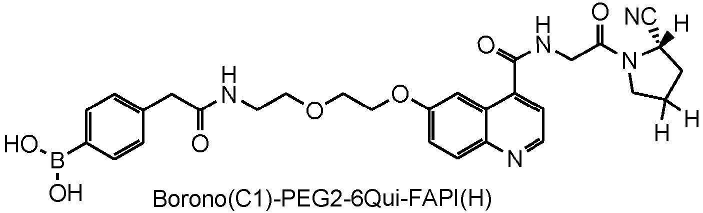

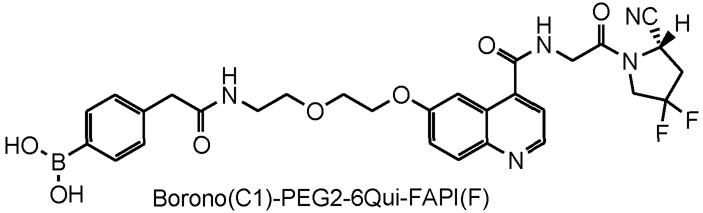

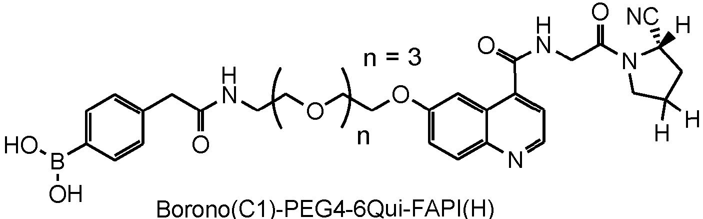

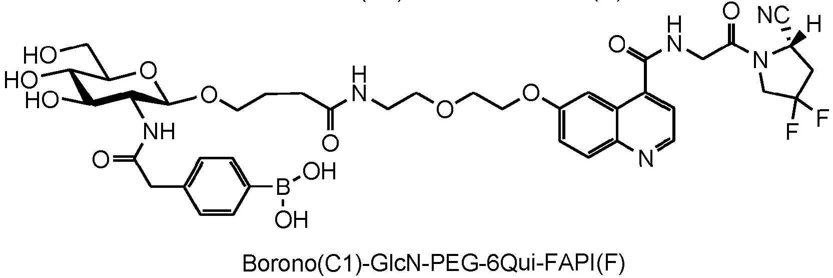

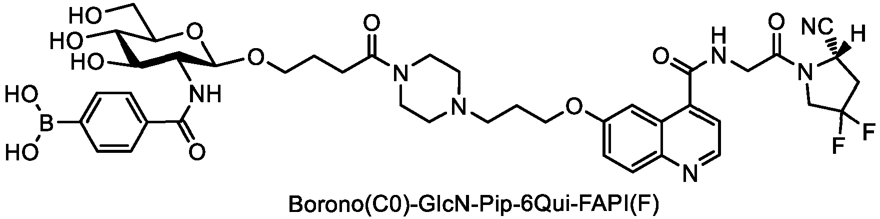

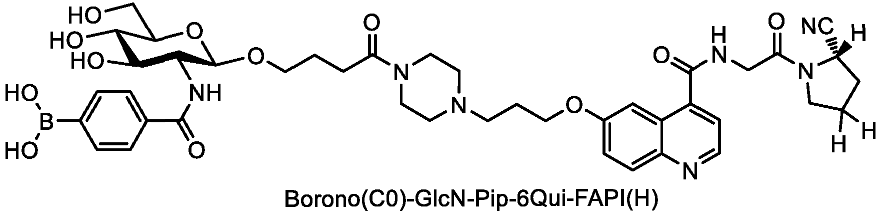

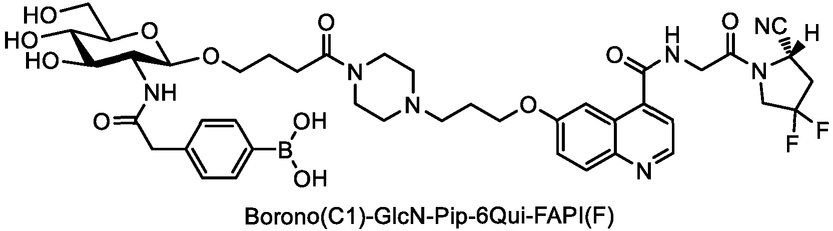

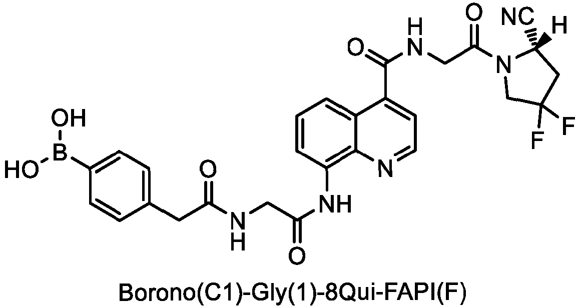

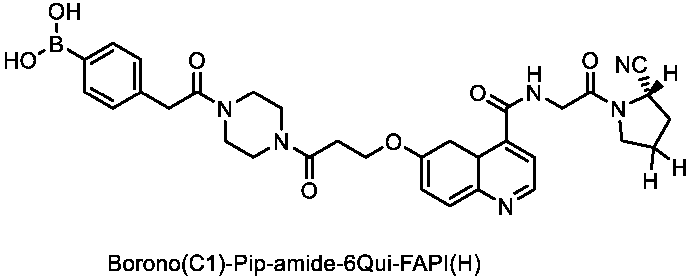

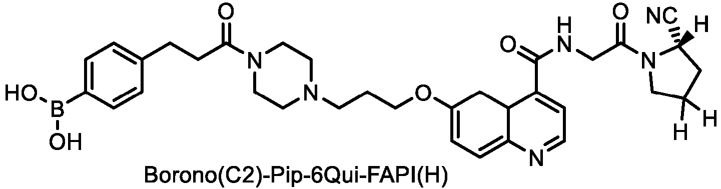

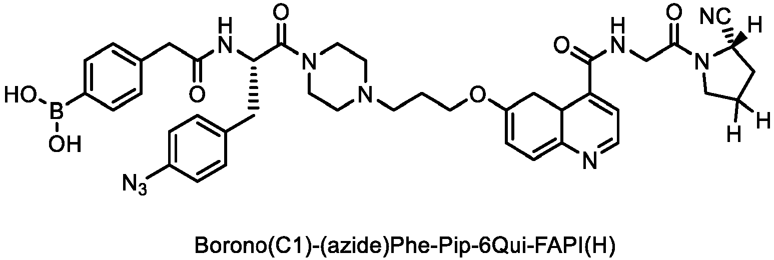

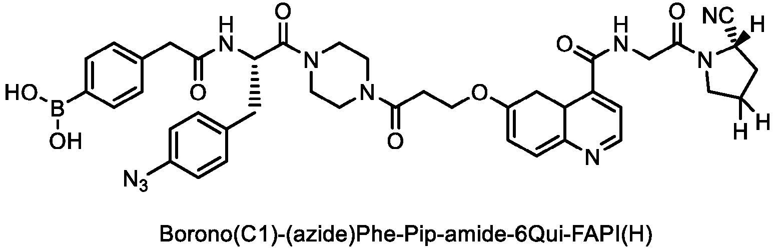

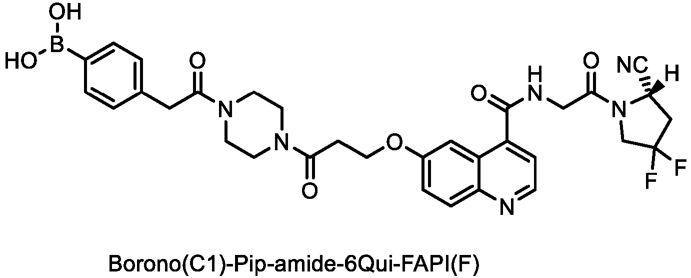

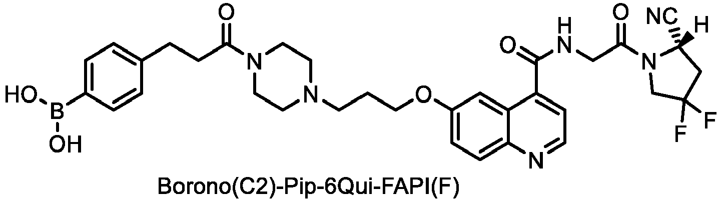

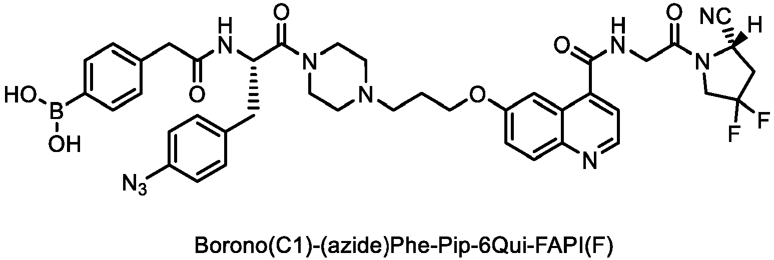

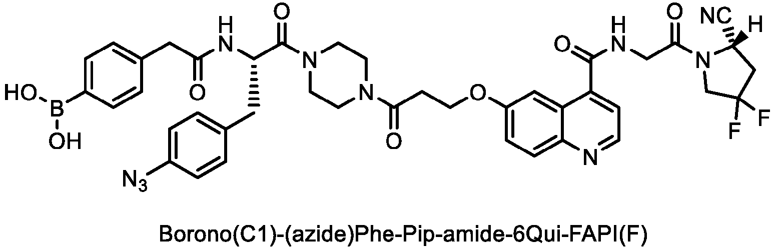

Boronic Acid Compound (II-1) can be produced by the method described below.

Since the reaction in this step is carried out in water, Boronic Acid Compound (II-1) may be in a free form or salt form as long as it can be dissolved in water. Alternatively, it may be used in the form of a solution prepared by dissolving in a weakly basic aqueous solution such as an aqueous sodium hydrogen carbonate solution.

Examples of the alkali metal iodide include potassium iodide, sodium iodide and the like. Among them, potassium iodide is preferably used.

Examples of the alkali metal bromide include sodium bromide, potassium bromide and the like.

Preferred combinations of the radionuclide and the above reagent include
(1) a combination in which the radionuclide is ²¹¹At or ²¹⁰At, and the above reagent is selected from potassium iodide, sodium bromide, N-bromosuccinimide, N-chlorosuccinimide, N-iodosuccinimide and hydrogen peroxide;
(2) a combination in which the radionuclide is ¹²³I, ¹²⁴I, ¹²⁵I or ¹³¹I, and the above reagent is selected from N-bromosuccinimide and N-chlorosuccinimide; and
(3) a combination in which the radionuclide is ⁷⁶Br or ⁷⁷Br, and the above reagent is N-chlorosuccinimide.
The above reagent may be used alone or in combination of two or more. The above reagent is used usually in the form of an aqueous solution.

Preferred embodiments include a combination in which the radionuclide is ²¹¹At or ¹³¹I, and the reagent is selected from potassium iodide and N-bromosuccinimide.

More preferred embodiments include
a combination in which the radionuclide is ²¹¹At, and the reagent is potassium iodide, and
a combination in which the radionuclide is ¹³¹I, and the reagent is N-bromosuccinimide.

The above reagent is used in an amount sufficient to oxidize or reduce the radionuclide, and is used usually in a large excess amount relative to the radionuclide. It is used preferably in a concentration of 0.0001 to 0.2 mol/L, more preferably in a concentration of 0.001 to 0.1 mol/L, in terms of reaction efficiency and economic efficiency.

For the reaction, the radionuclide is used usually in the form of an aqueous solution. If necessary, an alkaline aqueous solution such as sodium hydroxide and buffer solution may be added to the aqueous solution in order to stabilize the radionuclide.

In the case of radionuclide ²¹¹At, first, ²¹¹At is produced by ²⁰⁹Bi(α,2n)²¹¹At nuclear reaction resulting from the irradiation of bismuth with helium particles accelerated to 28 MeV by cyclotron. Next, by heating, the target substance ²⁰⁹Bi is melted and the ²¹¹At is vaporized, and the vaporized ²¹¹At is collected in a cooling trap, and dissolved in water to prepare an ²¹¹At stock solution. If necessary, an alkaline solution such as sodium hydroxide and buffer solution may be added thereto for the purpose of stabilizing ²¹¹At.

In the case of radionuclide ²¹⁰At, first, ²¹⁰At is produced by ²⁰⁹Bi(α,3n)²¹⁰At nuclear reaction resulting from the irradiation of bismuth with helium particles accelerated to 29 MeV or more by cyclotron. Next, by the same procedures as above, an aqueous ²¹⁰At solution is prepared.

In the case of radionuclide ¹²³I, it is available as an aqueous Na¹²³I solution.

In the case of radionuclide ¹²⁴I, first, ¹²⁴I is produced by ¹²⁴Te(p,n)¹²⁴I nuclear reaction resulting from the irradiation of tellurium with proton particles accelerated by cyclotron. Next, the target substance ¹²⁴Te is melted, and the ¹²⁴I is vaporized to prepare an aqueous ¹²⁴I sodium hydroxide solution.

In the case of radionuclide ¹²⁵I, it is available as an aqueous Na¹²⁵I solution.

In the case of radionuclide ¹³¹I, it is available as an aqueous Na¹³¹I solution.

In the case of radionuclide ⁷⁶Br, first, ⁷⁶Br is produced by ⁷⁶Se(p,n)⁷⁶Br nuclear reaction resulting from the irradiation of tellurium with proton particles accelerated by cyclotron. Next, the target substance ⁷⁶Se is melted, and the ⁷⁶Br is vaporized to prepare an aqueous ⁷⁶Br sodium hydroxide solution.

In the case of radionuclide ⁷⁷Br, first, ⁷⁷Br is produced by ⁷⁷Se(p,n)⁷⁷Br nuclear reaction resulting from the irradiation of tellurium with proton particles accelerated by cyclotron. Next, the target substance ⁷⁷Se is melted, and the ⁷⁷Br is vaporized to prepare an aqueous ⁷⁶Br sodium hydroxide solution.

²¹¹At has a half-life of 7.2 hours, ²¹⁰At has a half-life of 8.3 hours, ¹²³I has a half-life of 13.2 hours, and ⁷⁶Br has a half-life of 16 hours. These radionuclides have a short half-life, and therefore, they should be used in the subsequent reaction immediately after the preparation. On the other hand, ¹²⁴I has a half-life of 4.2 days, ¹²⁵I has a half-life of 59.4 days, ¹³¹I has a half-life of 8.04 days, and ⁷⁷Br has a half-life of 57 hours. Although these radionuclides have a relatively long half-life, they are also preferably used in the subsequent reaction immediately after the preparation.

Boronic Acid Compound (II-1) is used usually in a large excess amount relative to the radionuclide, preferably in a concentration of 0.00001 mol/l to 0.5 mol/l, more preferably in a concentration of 0.0001 mol/l to 0.2 mol/l, per 1 Bq to 1,000 GBq of the radionuclide, in terms of reaction efficiency and economic efficiency.

The above reaction is carried out by mixing Boronic Acid Compound (II-1), the above reagent and the radionuclide, and the mixing order is not particularly limited. The reaction is preferably carried out by adding an aqueous solution of the radionuclide, followed by an aqueous solution of the above reagent to an aqueous solution Boronic Acid Compound (II-1), or by adding an aqueous solution of the above reagent, followed by an aqueous solution of the radionuclide to an aqueous solution of Boronic Acid Compound (II-1), more preferably by adding an aqueous solution of the radionuclide, followed by an aqueous solution of the above reagent to an aqueous solution of Boronic Acid Compound (II-1).

The above reaction is carried out in water, i.e., in an organic solvent-free system.

The above reaction is carried out within the range of 0 to 95°C, preferably 10 to 80°C. The reaction time is from 1 minute to 3 hours, preferably from 1 min to 1 hour.

The completion of the reaction is confirmed by the disappearance of the free radionuclide, using thin-layer chromatography (TLC) analysis.

Since the reaction solution after the completion of the reaction contains neither an organic solvent nor a toxic reagent, the reaction solution can be immediately formulated into an injection and the like without isolating Radiolabeled Compound (I-1) .

The reaction of Boronic Acid Compound (II-1) with a radionuclide is an electrophilic substitution reaction and/or nucleophilic substitution reaction. The introduction site of the radionuclide in Boronic Acid Compound (II-1) is the benzene ring, and especially in the case of ²¹¹At or ²¹⁰At, the radionuclide is well introduced into the benzene ring.

Radiolabeled Compound (I-1) may be purified to remove byproducts, if necessary. This purification is preferably carried out by a solid-phase extraction column. As solid-phase extraction columns, those commonly used in the technical field can be used.

Furthermore, after the above purification, ascorbic acid or ascorbate may be added to a final concentration of 0.01% to 10%, preferably 0.1% to 5%. This makes it possible to suppress the decomposition of Radiolabeled Compound (I-1) and retain it for a long period of time.

The production method of the present invention makes it possible to obtain Radiolabeled Compound (I-1) in a high radiochemical yield (RCY) of 60% or more, particularly 80% or more, especially 90% or more.

Furthermore, it is possible to obtain Radiolabeled Compound (I-1) with a high radiochemical purity (RCP) of 80% or more, particularly 90% or more, especially 95% or more.

Radiolabeled Compound (I-2) can be produced by the method shown in the following Scheme 2. 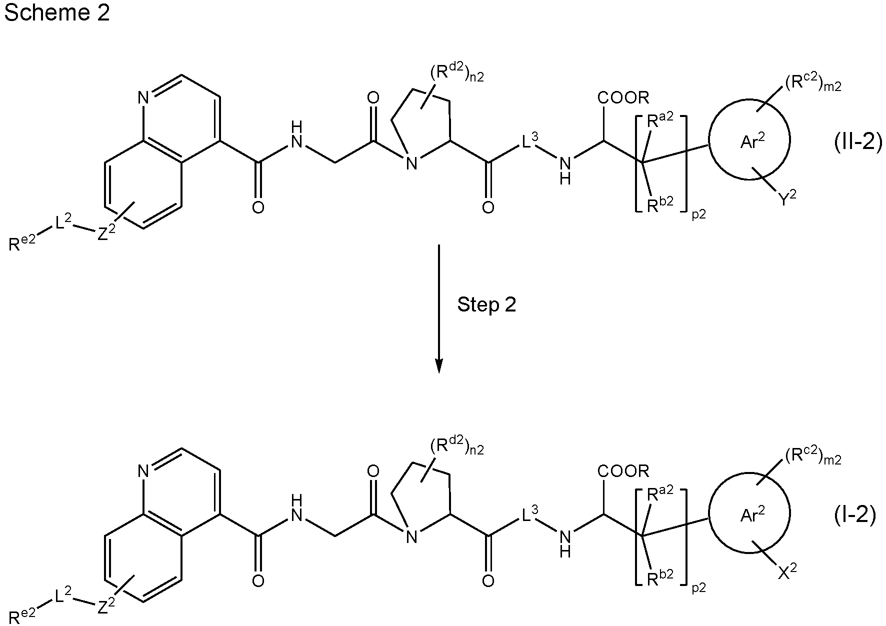 wherein each symbol in the formula is as defined above.

Y² is a boryl group (-B(OH)₂) or its ester group.

Y² is preferably a boryl group (-B(OH)₂) or a 4,4,5,5-tetramethyl-1,3,2-dioxaboran-2-yl group (a pinacol ester group).

Step 2 is a step of reacting Boronic Acid Compound (II-2) with a radionuclide selected from ²¹¹At, ²¹⁰At, ¹³¹I, ¹²⁵I, ¹²⁴I, ¹²³I, ⁷⁷Br and ⁷⁶Br in the presence of a reagent selected from an alkali metal iodide, an alkali metal bromide, N-bromosuccinimide, N-chlorosuccinimide, N-iodosuccinimide and hydrogen peroxide, in water to obtain Radiolabeled Compound (I-2).

Boronic Acid Compound (II-2) is a novel compound, and specific preferred examples thereof include the following.

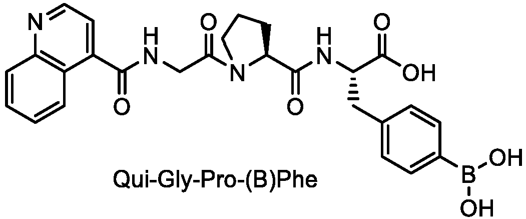

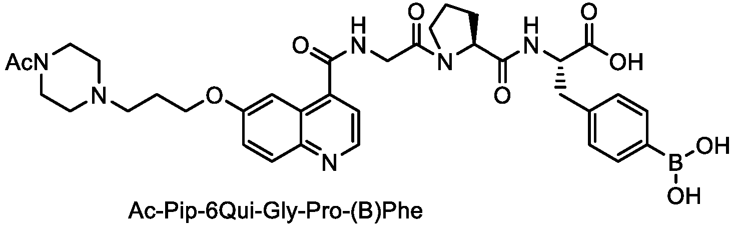

Boronic Acid Compound (II-2) can be produced by the method described below.

Since the reaction in this step is carried out in water, Boronic Acid Compound (II-2) may be in a free form or salt form as long as it can be dissolved in water. Alternatively, it may be used in the form of a solution prepared by dissolving in a weakly basic aqueous solution such as an aqueous sodium hydrogen carbonate solution.

Examples of the alkali metal iodide include potassium iodide, sodium iodide and the like. Among them, potassium iodide is preferably used.

Examples of the alkali metal bromide include sodium bromide, potassium bromide and the like.

Preferred combinations of the radionuclide and the above reagent include
(1) a combination in which the radionuclide is ²¹¹At or ²¹⁰At, and the above reagent is selected from potassium iodide, sodium bromide, N-bromosuccinimide, N-chlorosuccinimide, N-iodosuccinimide and hydrogen peroxide;
(2) a combination in which the radionuclide is ¹²³I, ¹²⁴I, ¹²⁵I or ¹³¹I, and the above reagent is selected from N-bromosuccinimide and N-chlorosuccinimide; and
(3) a combination in which the radionuclide is ⁷⁶Br or ⁷⁷Br, and the above reagent is N-chlorosuccinimide.
The above reagent may be used alone or in combination of two or more. The above reagent is used usually in the form of an aqueous solution.

Preferred embodiments include a combination in which the radionuclide is ²¹¹At or ¹³¹I, and the reagent is selected from potassium iodide and N-bromosuccinimide.

More preferred embodiments include
a combination in which the radionuclide is ²¹¹At, and the reagent is potassium iodide, and
a combination in which the radionuclide is ¹³¹I, and the reagent is N-bromosuccinimide.

The above reagent is used in an amount sufficient to oxidize or reduce the radionuclide, and is used usually in a large excess amount relative to the radionuclide. It is used preferably in a concentration of 0.0001 to 0.2 mol/L, more preferably in a concentration of 0.001 to 0.1 mol/L, in terms of reaction efficiency and economic efficiency.

For the reaction, the radionuclide is used usually in the form of an aqueous solution. If necessary, an alkaline aqueous solution such as sodium hydroxide and buffer solution may be added to the aqueous solution in order to stabilize the radionuclide.

In the case of radionuclide ²¹¹At, first, ²¹¹At is produced by ²⁰⁹Bi(α,2n)²¹¹At nuclear reaction resulting from the irradiation of bismuth with helium particles accelerated to 28 MeV by cyclotron. Next, by heating, the target substance ²⁰⁹Bi is melted and the ²¹¹At is vaporized, and the vaporized ²¹¹At is collected in a cooling trap, and dissolved in water to prepare an ²¹¹At stock solution. If necessary, an alkaline solution such as sodium hydroxide and buffer solution may be added thereto for the purpose of stabilizing ²¹¹At.

In the case of radionuclide ²¹⁰At, first, ²¹⁰At is produced by ²⁰⁹Bi(α,3n)²¹⁰At nuclear reaction resulting from the irradiation of bismuth with helium particles accelerated to 29 MeV or more by cyclotron. Next, by the same procedures as above, an aqueous ²¹⁰At solution is prepared.

In the case of radionuclide ¹²³I, it is available as an aqueous Na¹²³I solution.

In the case of radionuclide ¹²⁴I, first, ¹²⁴I is produced by ¹²⁴Te(p,n)¹²⁴I nuclear reaction resulting from the irradiation of tellurium with proton particles accelerated by cyclotron. Next, the target substance ¹²⁴Te is melted, and the ¹²⁴I is vaporized to prepare an aqueous ¹²⁴I sodium hydroxide solution.

In the case of radionuclide ¹²⁵I, it is available as an aqueous Na¹²⁵I solution.

In the case of radionuclide ¹³¹I, it is available as an aqueous Na¹³¹I solution.

In the case of radionuclide ⁷⁶Br, first, ⁷⁶Br is produced by ⁷⁶Se(p,n)⁷⁶Br nuclear reaction resulting from the irradiation of tellurium with proton particles accelerated by cyclotron. Next, the target substance ⁷⁶Se is melted, and the ⁷⁶Br is vaporized to prepare an aqueous ⁷⁶Br sodium hydroxide solution.

In the case of radionuclide ⁷⁷Br, first, ⁷⁷Br is produced by ⁷⁷Se(p,n)⁷⁷Br nuclear reaction resulting from the irradiation of tellurium with proton particles accelerated by cyclotron. Next, the target substance ⁷⁷Se is melted, and the ⁷⁷Br is vaporized to prepare an aqueous ⁷⁶Br sodium hydroxide solution.

²¹¹At has a half-life of 7.2 hours, ²¹⁰At has a half-life of 8.3 hours, ¹²³I has a half-life of 13.2 hours, and ⁷⁶Br has a half-life of 16 hours. These radionuclides have a short half-life, and therefore, they should be used in the subsequent reaction immediately after the preparation. On the other hand, ¹²⁴I has a half-life of 4.2 days, ¹²⁵I has a half-life of 59.4 days, ¹³¹I has a half-life of 8.04 days, and ⁷⁷Br has a half-life of 57 hours. Although these radionuclides have a relatively long half-life, they are also preferably used in the subsequent reaction immediately after the preparation.

Boronic Acid Compound (II-2) is used usually in a large excess amount relative to the radionuclide, preferably in a concentration of 0.00001 mol/l to 0.5 mol/l, more preferably in a concentration of 0.0001 mol/l to 0.2 mol/l, per 1 Bq to 1,000 GBq of the radionuclide, in terms of reaction efficiency and economic efficiency.

The above reaction is carried out by mixing Boronic Acid Compound (II-2), the above reagent and the radionuclide, and the mixing order is not particularly limited. The reaction is preferably carried out by adding an aqueous solution of the radionuclide, followed by an aqueous solution of the above reagent to an aqueous solution Boronic Acid Compound (II-2), or by adding an aqueous solution of the above reagent, followed by an aqueous solution of the radionuclide to an aqueous solution of Boronic Acid Compound (II-2), more preferably by adding an aqueous solution of the radionuclide, followed by an aqueous solution of the above reagent to an aqueous solution of Boronic Acid Compound (II-2).

The above reaction is carried out in water, i.e., in an organic solvent-free system.

The above reaction is carried out within the range of 0 to 95°C, preferably 10 to 80°C. The reaction time is from 1 minute to 3 hours, preferably from 1 min to 1 hour.

The completion of the reaction is confirmed by the disappearance of the free radionuclide, using thin-layer chromatography (TLC) analysis.

Since the reaction solution after the completion of the reaction contains neither an organic solvent nor a toxic reagent, the reaction solution can be immediately formulated into an injection and the like without isolating Radiolabeled Compound (I-2) .

The reaction of Boronic Acid Compound (II-2) with a radionuclide is an electrophilic substitution reaction and/or nucleophilic substitution reaction. The introduction site of the radionuclide in Boronic Acid Compound (II-2) is the benzene ring, and especially in the case of ²¹¹At or ²¹⁰At, the radionuclide is well introduced into the benzene ring.

Radiolabeled Compound (I-2) may be purified to remove byproducts, if necessary. This purification is preferably carried out by a solid-phase extraction column. As solid-phase extraction columns, those commonly used in the technical field can be used.

Furthermore, after the above purification, ascorbic acid or ascorbate may be added to a final concentration of 0.01% to 10%, preferably 0.1% to 5%. This makes it possible to suppress the decomposition of Radiolabeled Compound (I-2) and retain it for a long period of time.

The production method of the present invention makes it possible to obtain Radiolabeled Compound (I-2) in a high radiochemical yield (RCY) of 60% or more, particularly 80% or more, especially 90% or more.

Furthermore, it is possible to obtain Radiolabeled Compound (I-2) with a high radiochemical purity (RCP) of 80% or more, particularly 90% or more, especially 95% or more.

Boronic Acid Compounds (II-1) and (II-2) can be produced by the methods shown in the following Schemes 3 to 9.

Boronic Acid Compound (IIa-1), which is Boronic Acid Compound (II-1) wherein L¹ is *-L^{c1}-L^{b1}-L^{a1}-** wherein each symbol is as defined above, i.e., L¹ is *-L^{d1}-L^{c1}-L^{b1}-L^{a1}-** and L^{d1} is a bond, can be produced according to the method shown in the following Scheme 3. 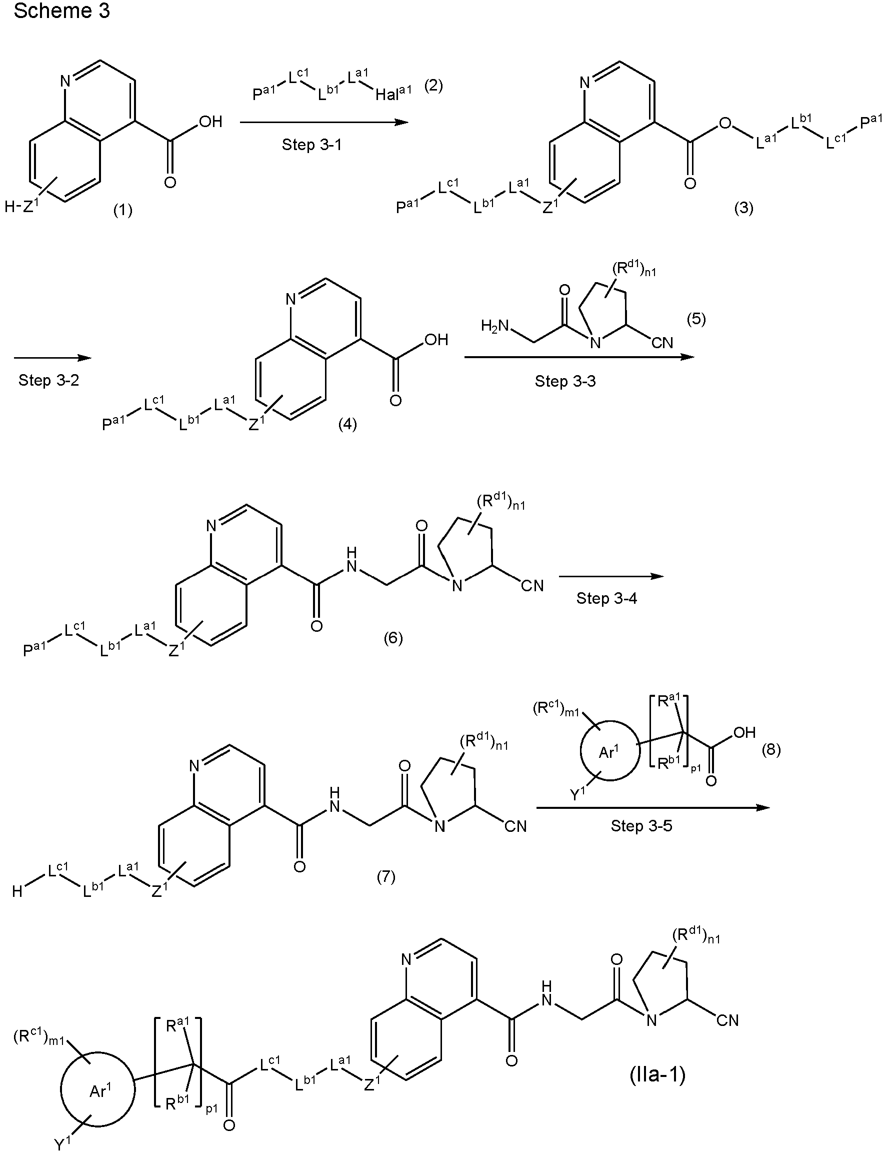 wherein P^{a1} is a protecting group (an amino-protecting group, a hydroxy-protecting group, a mercapto-protecting group), Hal^{a1} is a bromine atom or a chlorine atom, and the other symbols are as defined above.

When L^{c1} is NR^{g1} wherein the symbol is as defined above, or a divalent cyclic amino group, then P^{a1} is an amino-protecting group, preferably a tert-butoxycarbonyl group (Boc group).

When L^{c1} is an oxygen atom, then P^{a1} is a hydroxy-protecting group.

When L^{c1} is a sulfur atom, then P^{a1} is a mercapto-protecting group.

Hal^{a1} is preferably a bromine atom.

Step 3-1 is a step of reacting Compound (1) with Compound (2) to obtain Compound (3).

The reaction is carried out in the presence of a base, in a solvent.

Compound (1) and Compound (2) may be commercially available products or can be produced according to a synthesis method known per se.

The amount of Compound (2) to be used is generally 2 to 10 mol, preferably 2 to 5 mol, per 1 mol of Compound (1).

Examples of the base include inorganic bases such as potassium carbonate, cesium carbonate and the like.

The amount of the base to be used is generally 2 to 20 mol, preferably 4 to 15 mol, per 1 mol of Compound (1).

Examples of the solvent include N,N-dimethylformamide (DMF), tetrahydrofuran (THF), dioxane, acetonitrile and the like.

The reaction is carried out generally at temperature within the range of 25 to 100°C, preferably at temperature within the range of 50 to 80°C, generally for 6 to 24 hr, preferably for 10 to 24 hr.

After the completion of the reaction, the reaction mixture is subjected to a conventional work-up, and then, if necessary, purified by column chromatography or the like to obtain Compound (3) .

Step 3-2 is a step of converting Compound (3) to Compound (4) .

The reaction is carried out by treating with a base in a solvent.

For example, the reaction is carried out in the presence of an inorganic base such as lithium hydroxide, sodium hydroxide, potassium hydroxide or the like, in a mixed solvent of tetrahydrofuran/1,4-dioxane/water.

Alternatively, the reaction is carried out the presence of sodium methoxide, in methanol. In this case, the corresponding methyl ester is obtained, and then this compound is converted to Compound (4) by subjecting hydrolysis with lithium hydroxide, sodium hydroxide, potassium hydroxide or the like, in a mixed solvent of tetrahydrofuran/1,4-dioxane/water.

The reaction is carried out generally at temperature within the range of -20 to 40°C, preferably at temperature within the range of 0 to 30°C, generally for 10 min to 24 hr, preferably for 0.5 to 2 hr.

After the completion of the reaction, the reaction mixture is subjected to a conventional work-up, and then, if necessary, purified by column chromatography or the like to obtain Compound (4) .

Step 3-3 is a step of reacting Compound (4) with Compound (5) to obtain Compound (6).

For example, when L^{c1} is NR^{g1} wherein the symbol is as defined above, or a divalent cyclic amino group, the reaction may be carried out in the presence of a condensing agent, in a solvent, or by converting Compound (4) to the corresponding reactive derivative (e.g., an acid chloride) in a solvent, and then reacting the resulting compound in the presence of a base.

Compound (5) may be a commercially available product or can be produced according to a synthesis method known per se.

The amount of Compound (5) to be used is generally 1 to 10 mol, preferably 2 to 5 mol, per 1 mol of Compound (4).

Examples of the condensing agent include 2-(7-aza-1H-benzotriazol-1-yl)-1,1,3,3-tetramethyluronium hexafluorophosphate (HATU), (benzotriazol-1-yloxy)tripyrrolidinophosphonium hexafluorophosphate (PyBOP), (7-azabenzotriazol-1-yloxy)tripyrrolidinophosphonium hexafluorophosphate (PyAOP), (benzotriazol-1-yloxy)tris(dimethylamino)phosphonium hexafluorophosphate (BOP), 1-ethyl-3-(3-dimethylaminopropyl)carbodiimide (EDC), diisopropylcarbodiimide (DIC) and the like. Among them, HATU, EDC and PyBOP are preferably used.

The amount of the condensing agent to be used is generally 1 to 10 mol, preferably 1 to 3 mol, per 1 mol of Compound (4).

When the reaction is carried out in the presence of a condensing agent, the reaction may be carried out in the presence of a base. Examples of the base include organic bases such as N,N-diisopropylethylamine (DIEA), triethylamine (TFA) and the like.

The amount of the base to be used is generally 1 to 10 mol, preferably 2 to 5 mol, per 1 mol of Compound (4).

When EDC is used as a condensing agent, the reaction is preferably carried out in the presence of an additive such as 1-hydroxybenzotriazole (HOBt), 1-hydroxy-7-azabenzotriazole (HOAt) and the like.

The amount of the additive to be used is generally 1 to 10 mol, preferably 1 to 3 mol, per 1 mol of Compound (4).

Examples of the solvent include N,N-dimethylformamide (DMF), N-methyl-2-pyrrolidone (NMP), tetrahydrofuran (THF), acetonitrile and the like.

The reaction is carried out generally at temperature within the range of 0 to 60°C, preferably at temperature within the range of 0 to 30°C, generally for 6 to 72 hr, preferably for 6 to 48 hr.

After the completion of the reaction, the reaction mixture is subjected to a conventional work-up, and then, if necessary, purified by column chromatography or the like to obtain Compound (6) .

Step 3-4 is a step of subjecting Compound (6) to deprotection to obtain Compound (7).

The deprotection method is appropriately selected depending on the type of the protecting group. For example, when P^{a1} is a tert-butoxycarbonyl group (Boc group), Compound (7) can be obtained by treating Compound (6) under an acidic condition.

The treatment under an acidic condition includes an acid-treatment with trifluoroacetic acid (TFA) or the like. The acid-treatment may be carried out in a solvent such as dichloromethane, dichloroethane or the like.

After the completion of the reaction, the reaction mixture is subjected to a conventional work-up, and then, if necessary, purified by column chromatography or the like to obtain Compound (7) .

Step 3-5 is a step of reacting Compound (7) with Compound (8) to obtain Compound (IIa-1).

For example, when L^{c1} is NR^{g1} wherein the symbol is as defined above, or a divalent cyclic amino group, the step is carried out in the same manner as Step 3-3.

Compound (8) may be a commercially available product or can be produced according to a synthesis method known per se.

After the completion of the reaction, the reaction mixture is subjected to a conventional work-up, and then, if necessary, purified by column chromatography or the like to obtain Compound (IIa-1).

Boronic Acid Compound (IIb-1), which is Boronic Acid Compound (II-1) wherein L¹ is *-L^{d1}-L^{c1}-L^{b1}-L^{a1}-** wherein each symbol is as defined above, and L^{d1} is *-(NH-A^{a1}-CO)ᵣ₁-*** wherein each symbol is as defined above, can be produced according to the method shown in the following Scheme 4. 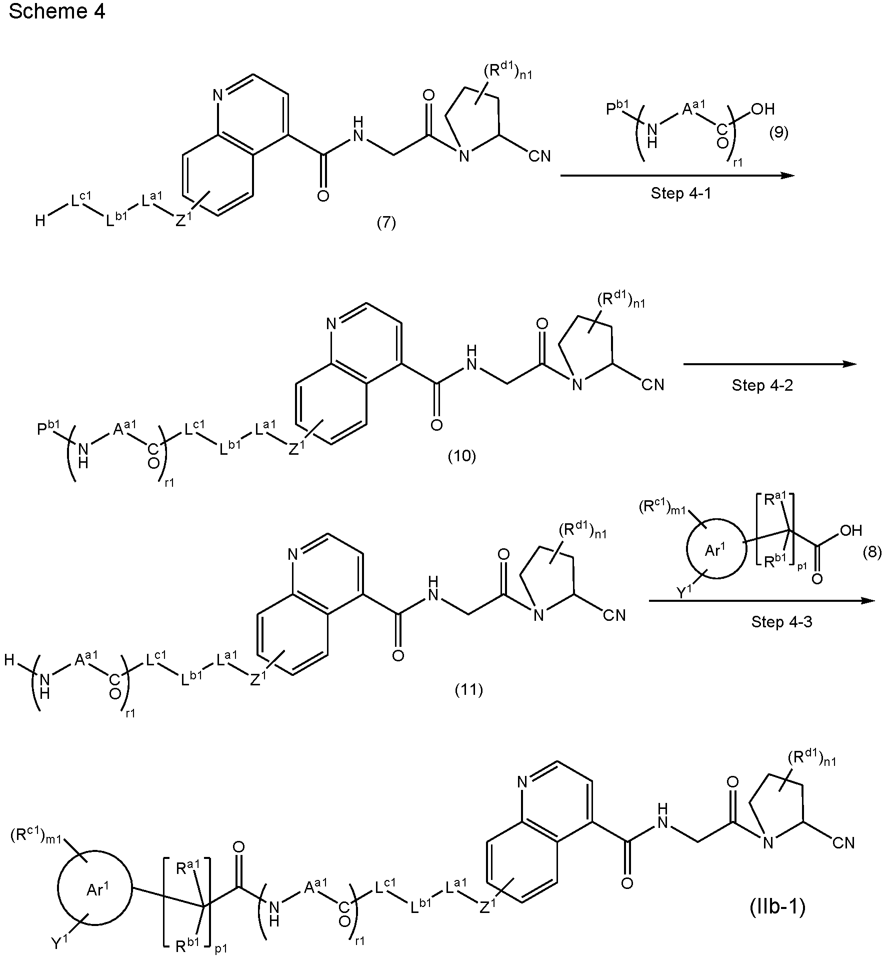 wherein P^{b1} is an amino-protecting group, and the other symbols are as defined above.

P^{b1} is preferably a tert-butoxycarbonyl group (Boc group).

Step 4-1 is a step of reacting Compound (7) with Compound (9) to obtain Compound (10).

Compound (9) may be a commercially available product or can be produced according to a synthesis method known per se.

For example, when L^{c1} is NR^{g1} wherein the symbol is as defined above, or a divalent cyclic amino group, the step is carried out in the same manner as Step 3-3.

After the completion of the reaction, the reaction mixture is subjected to a conventional work-up, and then, if necessary, purified by column chromatography or the like to obtain Compound (10) .

Step 4-2 is a step of subjecting Compound (10) to deprotection to obtain Compound (11).

The step is carried out in the same manner as Step 3-4.

After the completion of the reaction, the reaction mixture is subjected to a conventional work-up, and then, if necessary, purified by column chromatography or the like to obtain Compound (11) .

Step 4-3 is a step of reacting Compound (11) with Compound (8) to obtain Compound (IIb-1).

The step is carried out in the same manner as Step 3-3.

After the completion of the reaction, the reaction mixture is subjected to a conventional work-up, and then, if necessary, purified by column chromatography or the like to obtain Compound (IIb-1).

Boronic Acid Compound (IIc-1), which is Boronic Acid Compound (II-1) wherein L¹ is *-L^{d1}-L^{c1}-L^{b1}-L^{a1}-** wherein each symbol is as defined above, and L^{d1} is *-NH-B^{a1}-O-B^{b1}-CO-*** wherein each symbol is as defined above, can be produced according to the method shown in the following Scheme 5. 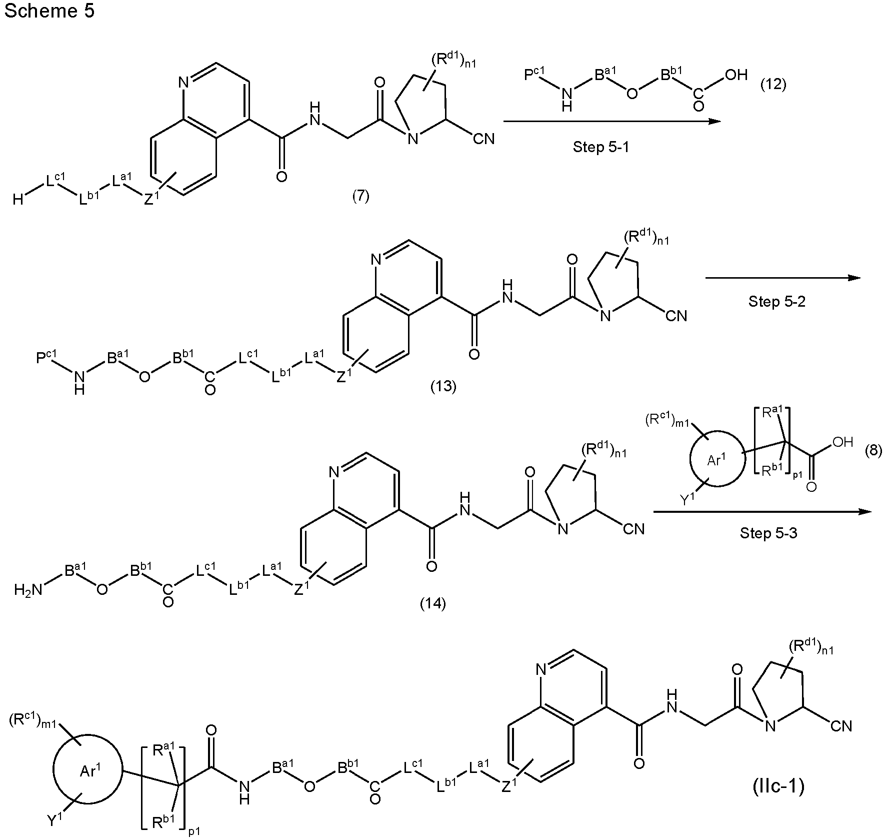 wherein P^{c1} is an amino-protecting group, and the other symbols are as defined above.

P^{c1} is preferably a tert-butoxycarbonyl group (Boc group).

Step 5-1 is a step of reacting Compound (7) with Compound (12) to obtain Compound (13).

The step is carried out by converting Compound (12) to the corresponding activated ester such as a pentafluorophenyl ester or the like, and then reacting the resulting compound with Compound (7) .

Compound (12) can be produced according to a synthesis method known per se, or the method described below. In Compound (12), the hydroxy group present in the divalent residue derived from an aminosaccharide or a derivative thereof is preferably protected. Examples of the protecting group include a tert-butyldimethylsilyl group (TBS group), a benzylidene group (formation of benzylideneacetal) and the like.

The conversion to the corresponding pentafluorophenyl ester is carried out by reacting Compound (12) with pentafluorophenyl trifluoroacetate in the presence of a base, in a solvent.

The amount of the pentafluorophenyl trifluoroacetate to be used is generally 1 to 10 mol, preferably 2 to 8 mol, per 1 mol of Compound (12).

Examples of the base include organic bases such as pyridine, triethylamine, N,N-diisopropylethylamine (DIEA) and the like.

The amount of the base to be used is generally 1 to 10 mol, preferably 2 to 8 mol, per 1 mol of Compound (12).

Examples of the solvent include N,N-dimethylformamide (DMF), dichloromethane, tetrahydrofuran (THF) and the like.

The reaction is carried out generally at temperature within the range of -20 to 60°C, preferably at temperature within the range of 0 to 30°C, generally for 0.5 to 24 hr, preferably for 0.5 to 6 hr.

After the completion of the reaction, the reaction mixture is subjected to an operation such as concentration or the like, and the obtained pentafluorophenyl ester is reacted with Compound (7) .

The reaction is carried out in the presence of a base, in a solvent.

The amount of the pentafluorophenyl ester to be used is generally 1 to 10 mol, preferably 1 to 3 mol, per 1 mol of Compound (7).

Examples of the base include organic bases such as N,N-diisopropylethylamine (DIEA), triethylamine (TEA), pyridine and the like.

The amount of the base to be used is generally 1 to 30 mol, preferably 2 to 25 mol, per 1 mol of Compound (7).

Examples of the solvent include N,N-dimethylformamide (DMF), tetrahydrofuran (THF), dichloromethane and the like.

The reaction is carried out generally at temperature within the range of -20 to 60°C, preferably at temperature within the range of 0 to 30°C, generally for 1 to 48 hr, preferably for 1 to 24 hr.

After the completion of the reaction, the reaction mixture is subjected to a conventional work-up, and then, if necessary, purified by column chromatography or the like to obtain Compound (13) .

Step 5-2 is a step of subjecting Compound (13) to deprotection to obtain Compound (14).

The step is carried out in the same manner as Step 3-4.

After the completion of the reaction, the reaction mixture is subjected to a conventional work-up, and then, if necessary, purified by column chromatography or the like to obtain Compound (14) .

Step 5-3 is a step of reacting Compound (14) with Compound (8) to obtain Compound (IIc-1).

The step is carried out in the same manner as Step 3-3.

After the completion of the reaction, the reaction mixture is subjected to a conventional work-up, and then, if necessary, purified by column chromatography or the like to obtain Compound (IIc-1).

Compound (12), which is used as a raw material in Scheme 5, can be produced according to the following method. 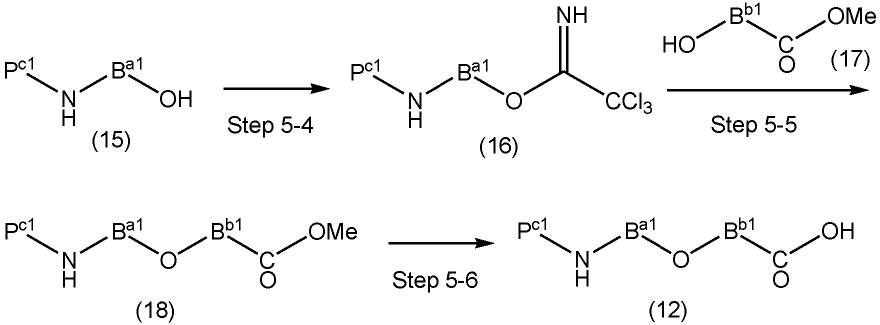 wherein each symbol is as defined above.

Step 5-4 is a step of reacting Compound (15) with trichloroacetonitrile to obtain Compound (16).

The reaction is carried out in the presence of a base, in a solvent.

Compound (15) may be a commercially available product or can be produced according to a synthesis method known per se. In Compound (15), the hydroxy group present in the divalent residue derived from an aminosaccharide or a derivative thereof is preferably protected. Examples of the protecting group include a tert-butyldimethylsilyl group (TBS group), a benzylidene group (formation of benzylideneacetal) and the like. Furthermore, in Compound (15), P^{c1} is preferably a trichloroethoxycarbonyl group (Troc group).

The amount of the trichloroacetonitrile to be used is generally 1 to 20 mol, preferably 5 to 15 mol, per 1 mol of Compound (15).

Examples of the base include inorganic or organic bases such as cesium carbonate, potassium carbonate, diazabicycloundecene and the like.

The amount of the base to be used is generally 1 to 10 mol, preferably 1 to 5 mol, per 1 mol of Compound (15).

Examples of the solvent include dichloromethane, dichloroethane, toluene, acetonitrile and the like.

The reaction is carried out generally at temperature within the range of -20 to 50°C, preferably at temperature within the range of 0 to 30°C, generally for 10 min to 12 hr, preferably for 0.5 to 4 hr.

After the completion of the reaction, the reaction mixture is subjected to a conventional work-up, and then, if necessary, purified by column chromatography or the like to obtain Compound (16) .

Step 5-5 is a step of reacting Compound (16) with Compound (17) to obtain Compound (18).

The reaction is carried out in the presence of a Lewis acid or a protic acid, in a solvent.

Compound (17) may be a commercially available product or can be produced according to a synthesis method known per se.

The amount of the Compound (17) to be used is generally 0.1 to 10 mol, preferably 0.5 to 5 mol, per 1 mol of Compound (16).

Examples of the Lewis acid or protic acid include trimethylsilyl trifluoromethanesulfonate, triethylsilyl trifluoromethanesulfonate, tert-butyldimethylsilyl trifluoromethanesulfonate, anhydrous trifluoromethanesulfonic acid, boron trifluoride diethyl ether complex, trifluoromethanesulfonic acid and the like.

The amount of the Lewis acid or protic acid to be used is generally 0.001 to 10 mol, preferably 0.001 to 1 mol, per 1 mol of Compound (16).

Examples of the solvent include dichloromethane, dichloroethane, tetrahydrofuran (THF), diethyl ether, cyclopentyl methyl ether (CPME), acetonitrile, toluene and the like.

The reaction may be carried out under argon atmosphere.

The reaction may be carried out in the presence of molecular sieves.

The reaction is carried out generally at temperature within the range of -78 to 50°C, preferably at temperature within the range of -20 to 30°C, generally for 10 min to 24 hr, preferably for 0.5 to 4 hr.

After the completion of the reaction, the reaction mixture is subjected to a conventional work-up, and then, if necessary, purified by column chromatography or the like to obtain Compound (18) .

Compound (18) may be subjected to conversion of the protecting group (P^{c1}). For example, the trichloroethoxycarbonyl group (Troc group) may be converted to a tert-butoxycarbonyl group (Boc group) in order to use Compound (12) obtained in the next step as a raw material in Scheme 5.

Step 5-6 is a step of subjecting Compound (18) to hydrolysis to obtain Compound (12).

The hydrolysis carried out according to a conventional method, for example, with lithium hydroxide in a mixed solvent of tetrahydrofuran/1,4-dioxane/water.

After the completion of the reaction, the reaction mixture is subjected to a conventional work-up, and then, if necessary, purified by column chromatography or the like to obtain Compound (12) .

Boronic Acid Compound (IId-1), which is Boronic Acid Compound (II-1) wherein L¹ is *-(NH-A^{b1}-CO)ₛ₁-** wherein each symbol is as defined above, can be produced according to the method shown in the following Scheme 6. 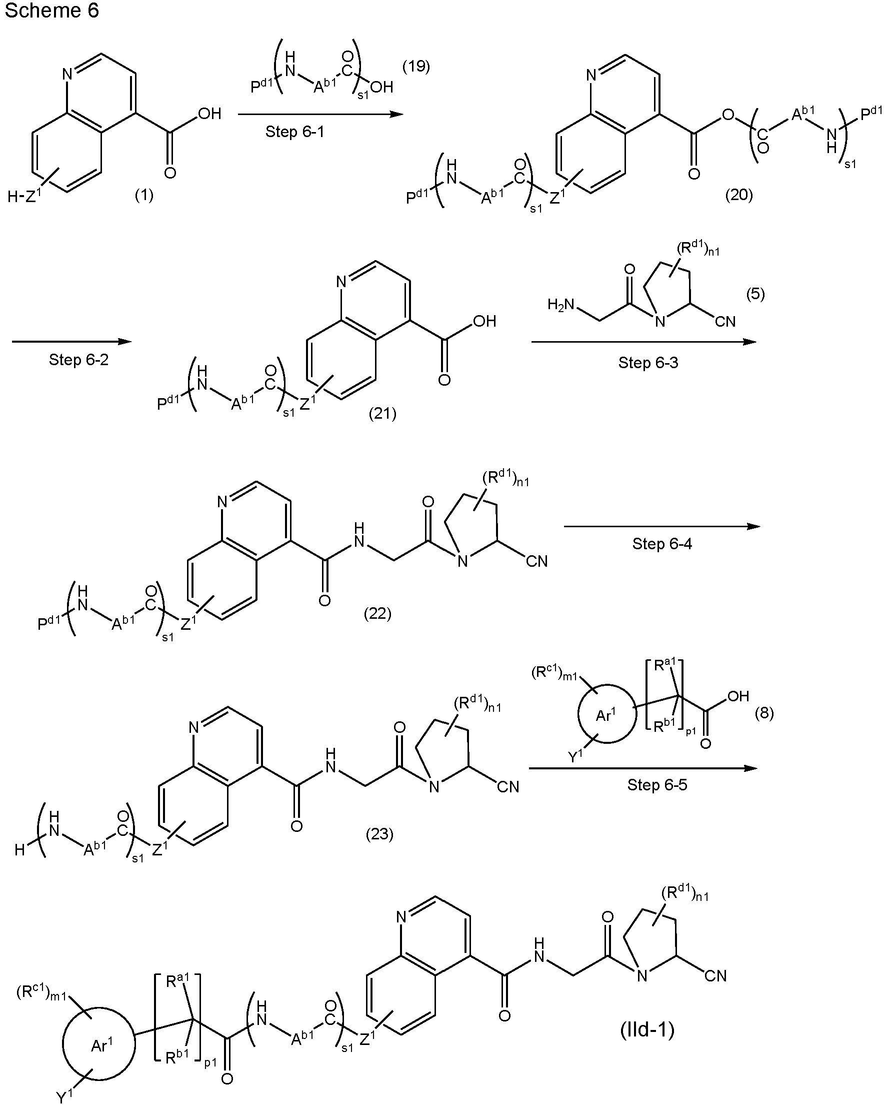 wherein P^{d1} is an amino-protecting group, and the other symbols are as defined above.

P^{d1} is preferably a tert-butoxycarbonyl group (Boc group).

Step 6-1 is a step of reacting Compound (1) with Compound (19) to obtain Compound (20).

Compound (19) may be a commercially available product or can be produced according to a synthesis method known per se.

The step is carried out in the same manner as Step 3-3.

After the completion of the reaction, the reaction mixture is subjected to a conventional work-up, and then, if necessary, purified by column chromatography or the like to obtain Compound (20) .

Step 6-2 is a step of converting Compound (20) to Compound (21) .

The step is carried out in the same manner as Step 3-2.

After the completion of the reaction, the reaction mixture is subjected to a conventional work-up, and then, if necessary, purified by column chromatography or the like to obtain Compound (21) .

Step 6-3 is a step of reacting Compound (21) with Compound (5) to obtain Compound (22).

The step is carried out in the same manner as Step 3-3.

After the completion of the reaction, the reaction mixture is subjected to a conventional work-up, and then, if necessary, purified by column chromatography or the like to obtain Compound (22) .

Step 6-4 is a step of subjecting Compound (22) to deprotection to obtain Compound (23).

The step is carried out in the same manner as Step 3-4.

After the completion of the reaction, the reaction mixture is subjected to a conventional work-up, and then, if necessary, purified by column chromatography or the like to obtain Compound (23) .

Step 6-5 is a step of reacting Compound (23) with Compound (8) to obtain Compound (IId-1).

The step is carried out in the same manner as Step 3-3.

After the completion of the reaction, the reaction mixture is subjected to a conventional work-up, and then, if necessary, purified by column chromatography or the like to obtain Compound (IId-1).

Boronic Acid Compound (IIa-2), which is Boronic Acid Compound (11-2) wherein L² is *-L^{c2}-L^{b2}-L^{a2}-** wherein each symbol is as defined above, can be produced according to the method shown in the following Scheme 7. 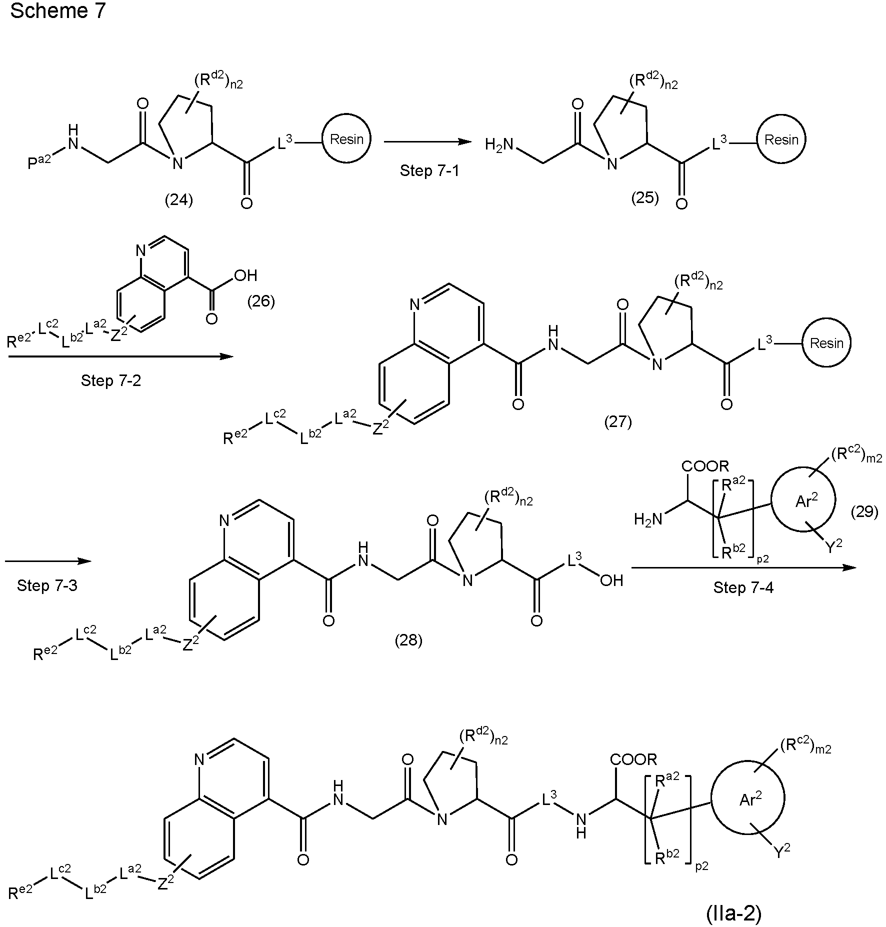 wherein P^{a2} is an amino-protecting group, Resin is a resin used in peptide synthesis, and the other symbols are as defined above.

P^{a2} is preferably a 9-fluorenylmethyloxycarbonyl group (Fmoc group).

Examples of the resin used in peptide synthesis for Resin include resins used in solid-phase synthesis, for example, Cl-Trt(2-Cl)-Resin and the like.

Step 7-1 is a step of subjecting Compound (24) to deprotection to obtain Compound (25).

The reaction is carried out according to a conventional method. For example, when P^{a2} is a 9-fluorenylmethyloxycarbonyl group (Fmoc group), the reaction is carried out using a secondary amine such as piperidine, pyrrolidine, morpholine or the like, in a solvent such as N,N-dimethylformamide (DMF), N-methyl-2-pyrrolidone (NMP) or the like.

Compound (24) can be produced according to a synthesis method known per se.

After the completion of the reaction, the reaction mixture is subjected to a conventional work-up, and then, if necessary, purified by column chromatography or the like to obtain Compound (25) .

Step 7-2 is a step of reacting Compound (25) with Compound (26) to obtain Compound (27).

Compound (26) can be synthesized by a method similar to that of Scheme 3. Alternatively, it can also be produced by the method described below.

The step is carried out in the same manner as Step 3-3.

After the completion of the reaction, the reaction mixture is subjected to a conventional work-up, and then, if necessary, purified by column chromatography or the like to obtain Compound (27) .

Step 7-3 is a step of subjecting Compound (27) to deresination to obtain Compound (28).

The deresination method is appropriately selected depending on the type of the resin. For example, when Resin is Cl-Trt(2-Cl)-Resin, the reaction is carried out by treating with hexafluoro-2-propanol (HFIP)/chloroform (TCM) solution.

After the completion of the reaction, the separated resin is removed, and the reaction mixture is subjected to a conventional work-up, and then, if necessary, purified by column chromatography or the like to obtain Compound (28).

Step 7-4 is a step of reacting Compound (28) with Compound (29) to obtain Compound (IIa-2).

The reaction is carried out by converting Compound (28) to the corresponding activated ester such as pentafluorophenyl ester or the like, and then reacting the resulting compound with Compound (29).

The conversion to the corresponding pentafluorophenyl ester is carried out by reacting Compound (28) with pentafluorophenyl trifluoroacetate in the presence of a condensing agent, in a solvent.

The amount of the pentafluorophenyl trifluoroacetate to be used is generally 1 to 10 mol, preferably 1 to 5 mol, per 1 mol of Compound (28).

Examples of the condensing agent include dicyclohexylcarbodiimide (DCC), diisopropylcarbodiimide (DIC) and the like.

The amount of the condensing agent to be used is generally 1 to 10 mol, preferably 1 to 5 mol, per 1 mol of Compound (28).

Examples of the solvent include tetrahydrofuran (THF), N,N-dimethylformamide (DMF), chloroform, acetonitrile, dichloromethane and the like.

The reaction is carried out generally at temperature within the range of -20 to 50°C, preferably at temperature within the range of 0 to 30°C, generally for 1 to 48 hr, preferably for 1 to 24 hr.

After the completion of the reaction, the reaction mixture is subjected to an operation such as concentration or the like, and the obtained pentafluorophenyl ester is reacted with Compound (29) .

Compound (29) may be a commercially available product or can be produced according to a synthesis method known per se.

The reaction is carried out in the same manner as the reaction of pentafluorophenyl ester with Compound (7) described in Step 5-1, but the base, solvent and the like are limited to those used in Step 5-1.

After the completion of the reaction, the reaction mixture is subjected to a conventional work-up, and then, if necessary, purified by column chromatography or the like to obtain Compound (IIa-2).

Compound (26'), which is Compound (26) wherein L^{c2} is a divalent cyclic diamino group (piperazine-1,4-diyl, etc.) and is used as a raw material in Scheme 7, can also be produced according to the following method. 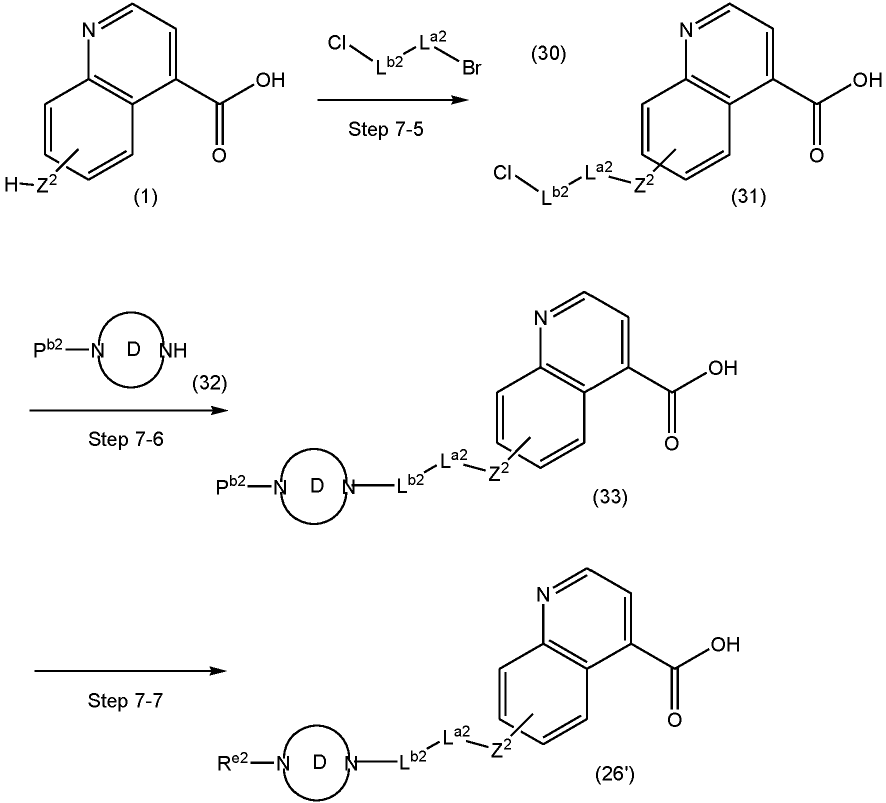 wherein P^{b2} is an amino-protecting group, and the other symbols are as defined above.

P^{b2} is preferably a tert-butoxycarbonyl group (Boc group).

Step 7-5 is a step of reacting Compound (1) with Compound (30) to obtain Compound (31).

Compound (30) may be a commercially available product or can be produced according to a synthesis method known per se.

The step is carried out in the same manner as Step 3-1.

After the completion of the reaction, the reaction mixture is subjected to a conventional work-up, and then, if necessary, purified by column chromatography or the like to obtain Compound (31) .

Step 7-6 is a step of reacting Compound (31) with Compound (32) to obtain Compound (33).

The reaction is carried out in the presence of sodium iodide, in a solvent.

Compound (32) may be a commercially available product or can be produced according to a synthesis method known per se.

The amount of the Compound (32) to be used is generally 1 to 10 mol, preferably 1 to 5 mol, per 1 mol of Compound (31).

The amount of the sodium iodide to be used is generally 1 to 10 mol, preferably 1 to 5 mol, per 1 mol of Compound (31).

Examples of the solvent include N,N-dimethylformamide (DMF), tetrahydrofuran (THF), acetonitrile and the like.

The reaction is carried out generally at temperature within the range of 0 to 80°C, preferably at temperature within the range of 20 to 60°C, generally for 1 to 48 hr, preferably for 4 to 30 hr.

After the completion of the reaction, the reaction mixture is subjected to a conventional work-up, and then, if necessary, purified by column chromatography or the like to obtain Compound (33) .

Step 7-7 is a step of subjecting Compound (33) to deprotection and acylation (introduction of R^{e2}) to obtain Compound (26').

The deprotection is carried out in the same manner as in Step 3-4.

The acylation is carried out by reacting the deprotected compound with an acylating agent corresponding to R^{e2} in the presence of a base, in a solvent.

Examples of the acylating agent include acetic anhydride, acetyl chloride and the like.

The amount of the acylating agent to be used is generally 1 to 10 mol, preferably 1 to 5 mol, per 1 mol of Compound (33).

Examples of the base include pyridine, triethylamine (TEA), N,N-diisopropylethylamine (DIEA) and the like.

The amount of the base to be used is generally 1 to 20 mol, preferably 1 to 10 mol, per 1 mol of Compound (33). Alternatively, the base is added as a solvent.

Examples of the solvent include N,N-dimethylformamide (DMF), acetonitrile, tetrahydrofuran (THF), pyridine, triethylamine and the like.

The reaction is carried out generally at temperature within the range of -20 to 60°C, preferably at temperature within the range of 0 to 30°C, generally for 0.5 to 24 hr, preferably for 0.5 to 12 hr.

After the completion of the reaction, the reaction mixture is subjected to a conventional work-up, and then, if necessary, purified by column chromatography or the like to obtain Compound (26').

Boronic Acid Compound (IIa-2), which is Boronic Acid Compound (11-2) wherein L² is *-L^{c2}-L^{b2}-L^{a2}-** wherein each symbol is as defined above, can also be produced according to the method shown in the following Scheme 8. 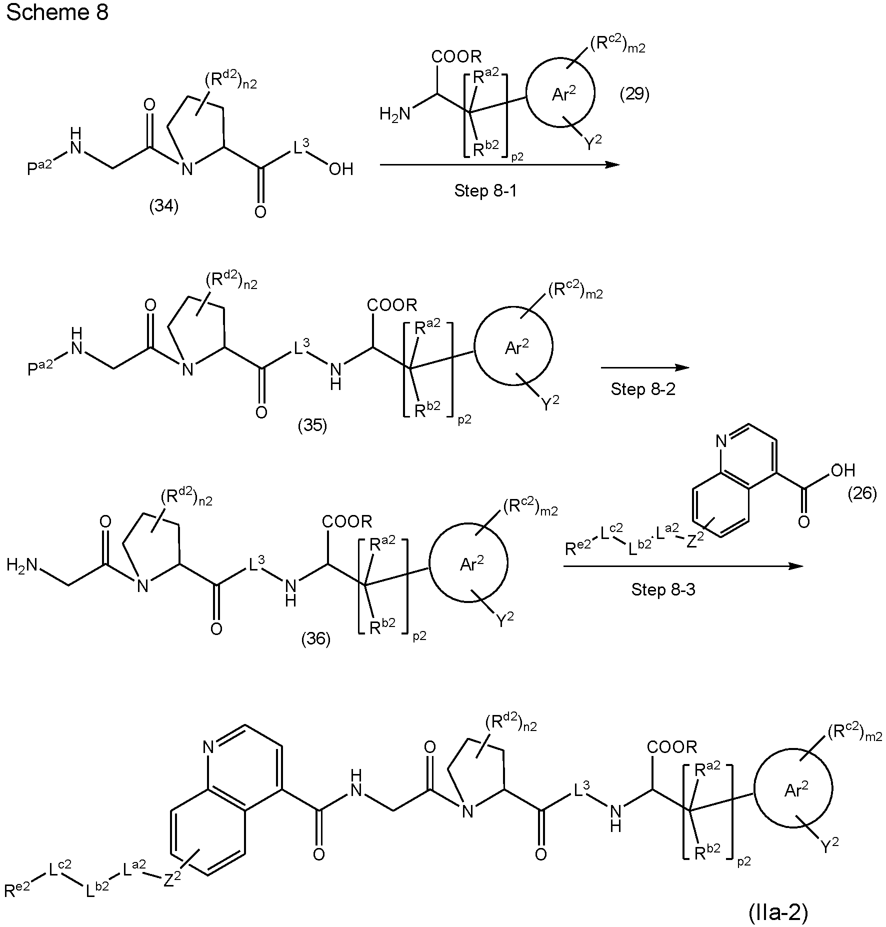 wherein each symbol is as defined above.

Step 8-1 is a step of reacting Compound (34) with Compound (29) to obtain Compound (35).

Compound (34) may be a commercially available product or can be produced according to a synthesis method known per se.

The step is carried out in the same manner as Step 7-4.

After the completion of the reaction, the reaction mixture is subjected to a conventional work-up, and then, if necessary, purified by column chromatography or the like to obtain Compound (35) .

Step 8-2 is a step of subjecting Compound (35) to deprotection to obtain Compound (36).

The step is carried out in the same manner as Step 3-3.

After the completion of the reaction, the reaction mixture is subjected to a conventional work-up, and then, if necessary, purified by column chromatography or the like to obtain Compound (36) .

Step 8-3 is a step of reacting Compound (36) with Compound (26) to obtain Compound (IIa-2).

The step is carried out in the same manner as Step 3-3.

After the completion of the reaction, the reaction mixture is subjected to a conventional work-up, and then, if necessary, purified by column chromatography or the like to obtain Compound (IIa-2).

Boronic Acid Compound (IIb-2), which is Boronic Acid Compound (II-2) wherein L² is *-(NH-A^{a2}-CO)ᵣ₂-** wherein each symbol is as defined above, can be produced according to the method shown in the following Scheme 9. 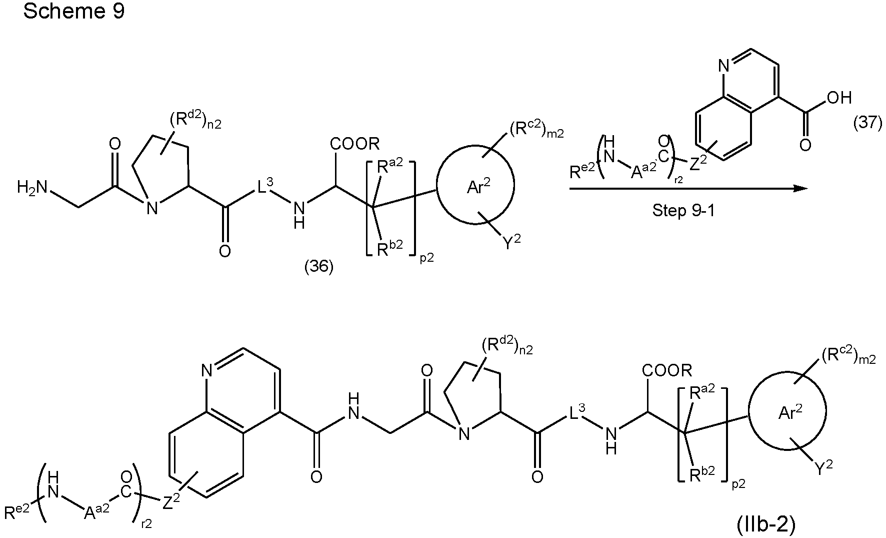 wherein each symbol is as defined above.

Step 9-1 is a step of reacting Compound (36) with Compound (37) to obtain Compound (IIa-2).

Compound (37) can be synthesized by a method similar to that of Scheme 6. Alternatively, it can also be produced by the method described below.

The step is carried out in the same manner as Step 3-3.

After the completion of the reaction, the reaction mixture is subjected to a conventional work-up, and then, if necessary, purified by column chromatography or the like to obtain Compound (IIb-2).

Compound (37), which is used as a raw material in Scheme 9, can be produced according to the following method. 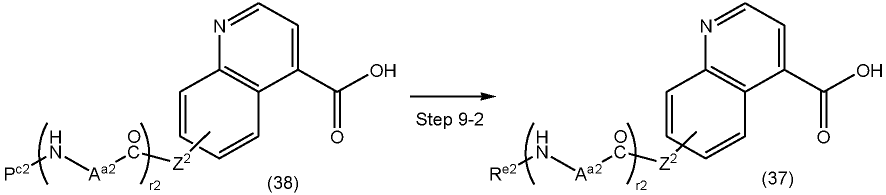 wherein P^{c2} is an amino-protecting group, and the other symbols are as defined above.

P^{c2} is preferably a tert-butoxycarbonyl group (Boc group).

Step 9-2 is a step of subjecting Compound (38) to deprotection and acylation (introduction of R^{e2}) to obtain Compound (37) .

Compound (38) can be produced by a method similar to that of Scheme 6.

The step is carried out in the same manner as Step 7-7.

After the completion of the reaction, the reaction mixture is subjected to a conventional work-up, and then, if necessary, purified by column chromatography or the like to obtain Compound (37) .

The reaction conditions such as solvent and reaction temperature in each step in the production method of the present invention described above are described in detail as representative examples in Synthesis Examples and Examples below, but are not necessarily limited thereto, and those skilled in the art can make appropriate selections based on their general knowledge in organic synthesis.

Thus-produced Radiolabeled Compounds (I-1) and (I-2) (hereinafter collectively referred to as Compound (I)) bind specifically to fibroblast activation protein α (FAPα), and accumulate in cancer-associated fibroblasts (CAFs). They destroy the stroma by emitting α-ray, thereby damaging the cells. In addition, since cancer cells themselves may express FAPα, they can exert a more potent therapeutic effect by simultaneously damaging both the stroma and cancer cells.

Since Radiolabeled Compound (I) targets cells expressing FAPα, Radiolabeled Compound (I) comprising a therapeutically effective radionuclide can be useful in the treatment of tumors or cancers containing cancerous stroma expressing FAPα (also referred to herein as "tumors or cancers expressing FAPα"). Examples of the therapeutically effective radionuclide include ²¹¹At, ²¹⁰At, ¹³¹I , ¹²⁵I and ⁷⁷Br.

Furthermore, since Radiolabeled Compound (I) targets cells expressing FAPα, Radiolabeled Compound (I) comprising an imaging-effective radionuclide can image tumors or cancers expressing FAPα, and thus can be useful in the diagnosis of the tumors or cancers. Examples of the imaging-effective radionuclide include ²¹¹At, ¹³¹I, ¹²⁴I, ¹²³I, ⁷⁷Br and ⁷⁶Br. Radiolabeled Compound (I) with a radionuclide selected from ²¹¹At, ¹³¹I, ¹²⁴I, ¹²³I, ⁷⁷Br and ⁷⁶Br is used for imaging in positron emission tomography (PET) or single photon emission computed tomography (SPECT).

Examples of the tumors or cancers expressing FAPα include solid cancers such as pancreatic cancer, sarcoma, esophageal cancer, lung cancer, breast cancer, prostate cancer, head and neck cancer, ovarian cancer, colon cancer, neuroendocrine tumor, thyroid cancer, uterine cancer, liver cancer and the like. Therefore, Radiolabeled Compound (I) is effective in the treatment and imaging of these solid cancers, particularly, pancreatic cancer.

The dose of Radiolabeled Compound (I) used for therapeutic or diagnostic purposes is generally determined by the radionuclide used, the patient's weight, age and sex, therapeutic/diagnostic site, and the like. For example, for human subjects, the estimated effective dosage of Radiolabeled Compound (I) with ²¹¹At per dose is approximately 100 MBq to 900 MBq.

Radiolabeled Compound (I) are usually mixed with a pharmaceutically acceptable carrier and used as a pharmaceutical composition. A pharmaceutically acceptable carrier refers to a biocompatible solution with due consideration for sterility, pH, isotonicity, stability, etc., and may include any and all solvents, diluents (including sterile saline, sodium chloride injection, Ringer's solution, dextrose injection, dextrose and sodium chloride injection, lactated Ringer's solution, and other aqueous buffers), dispersants, coatings, antibacterial and antifungal agents, isotonic agents, and the like. Pharmacologically acceptable carriers can also contain stabilizers, preservatives, antioxidants, or other additives known to those skilled in the art.

The dosage form of the pharmaceutical composition is not particularly limited, and it can be prepared as a pharmaceutical composition for oral administration in the form of granules, fine granules, powders, hard capsules, soft capsules, syrups, emulsions, suspensions, liquids and the like; or for parenteral administration such as intravenous administration, intramuscular administration and subcutaneous administration, in the form of injections, drip infusions, transdermal absorptions, transmucosal absorptions, nasal drops, inhalations, suppositories, and the like. These formulations can be prepared according to conventional methods. Preferred are liquid formulations for oral administration or for injection.

Such liquid formulations are prepared by dissolving Radiolabeled Compound (I) in water, or may also be prepared by dissolving Radiolabeled Compound (I) in saline or glucose solution. Buffers or preservatives may be added as necessary. As described above, a reducing agent such as ascorbic acid can also be included. In particular, for the production of injections, the active ingredient is dissolved in distilled water for injection, together with a pH adjuster such as hydrochloric acid, sodium hydroxide, lactose, sodium lactate, sodium monohydrogen phosphate and sodium dihydrogen phosphate, and an isotonic agent such as sodium chloride and glucose, as needed, and then the mixture is sterilely filtered and filled into an ampule to prepare an injection. Mannitol, dextrin, cyclodextrin, gelatin and the like may also be further added, and the mixture is vacuum lyophilized to prepare an injection that is dissolved immediately before use. Furthermore, lecithin, polysorbate 80, polyoxyethylene hydrogenated castor oil and the like may be added to the active ingredient, and the mixture is emulsified in water to prepare an emulsion for injection.

The half-life of the radionuclide contained in Radiolabeled Compound (I) is short; it is 7.2 hours for ²¹¹At, 8.3 hours for ²¹⁰At, 8.04 days for ¹³¹I, 59.4 days for ¹²⁵I, 4.2 days for ¹²⁴I, 13.2 hours for ¹²³I, 57 hours for ⁷⁷Br, and 16 hours for ⁷⁶Br. Therefore, it is desirable to prepare the pharmaceutical composition immediately before administration to the subject so that it contains the amount of Radiolabeled Compound (I) required for administration.

### [Example]

The present invention will be explained in detail by the following Synthesis Examples, Examples and Experimental Examples, which are merely examples and are not intended to limit the present invention and can be modified without departing from the scope of the present invention.

### Synthesis Example 1 Synthesis of Compound 7 (Borono(C1)-Pip-6Qui-FAPI(F))

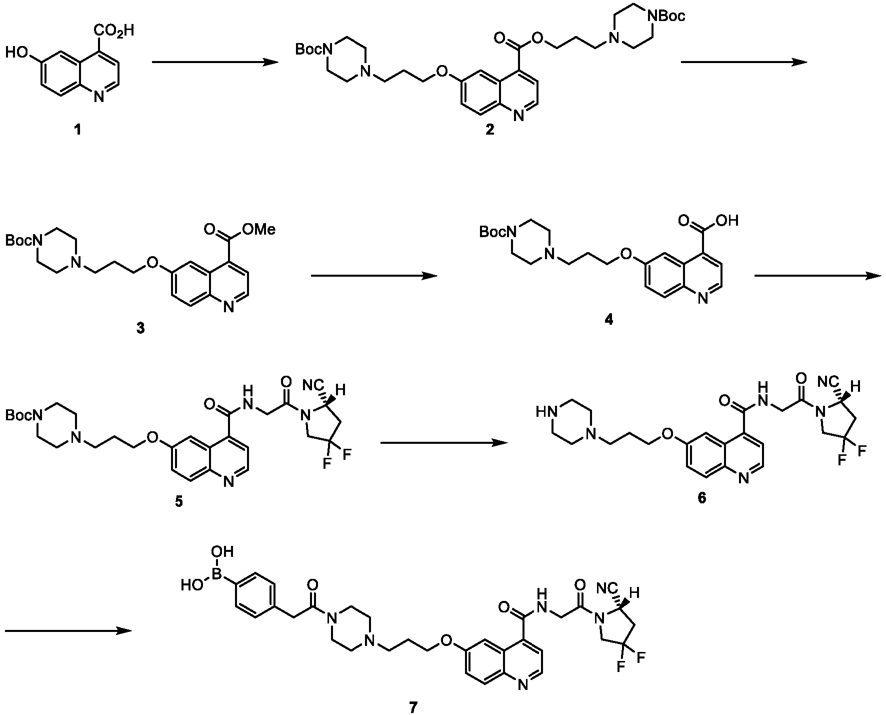

### (1) Synthesis of Compound 4

Under argon atmosphere, to commercially available Compound 1 (14.9 mg, 55.2 µmol) were added N,N-dimethylformamide (1.1 mL), potassium carbonate (76.3 mg, 552 µmol) and 4-(3-bromopropyl)-1-piperazinecarboxylic acid 1,1-dimethylethyl ester (51.8 mg, 168.6 µmol), and the mixture was stirred at 60°C for 12 hr. After cooling to room temperature, the mixture was subjected to extraction with ethyl acetate, and the organic layer was washed with saturated brine. The obtained organic layer was dried over sodium sulfate, and the sodium sulfate was removed by filtration. The solvent was evaporated under reduced pressure, and the residue was roughly purified by silica gel column chromatography to give Compound 2 as a yellow solid.

Under argon atmosphere, to the obtained Compound 2 were added methanol (1.0 mL), and sodium methoxide (8.4 µL, 5.7 M, 47.7 µmol) dissolved in methanol, and the mixture was stirred at room temperature for 40 min. To the reaction solution was added saturated aqueous ammonium chloride solution, and the mixture was subjected to extraction with ethyl acetate, and the organic layer was washed with water and saturated brine. The obtained organic layer was dried over sodium sulfate, and the sodium sulfate was removed by filtration. The solvent was evaporated under reduced pressure, and the residue was roughly purified by silica gel column chromatography to give Compound 3 as a yellow solid.

Under argon atmosphere, to the obtained Compound 3 were added tetrahydrofuran/1,4-dioxane/water (4/2/1) mixed solvent (1.4 mL), and lithium hydroxide monohydrate (17.8 mg, 424 µmol), and the mixture was stirred at room temperature for 40 min. To the reaction solution was added saturated aqueous ammonium chloride solution, and the mixture was subjected to extraction with chloroform, and the organic layer was washed with water and saturated brine. The obtained organic layer was dried over sodium sulfate, and the sodium sulfate was removed by filtration. The solvent was evaporated under reduced pressure, and the residue was purified by silica gel column chromatography to give Compound 4 as a yellow solid (15.7 mg, yield 68% in 3 steps).
¹H-NMR (DMSO-D₆) δ: 8.88 (d, J = 4.3 Hz, 1H), 8.19 (d, J = 2.4 Hz, 1H), 8.06 (d, J = 9.2 Hz, 1H), 7.94 (d, J = 4.3 Hz, 1H), 7.50 (dd, J = 9.2, 2.5 Hz, 1H), 4.22 (t, J = 5.7 Hz, 2H), 4.03 (s, 2H), 3.52 (s, 2H), 3.15-3.04 (br m, 6H), 2.25-2.20 (m, 2H), 1.43 (s, 9H).

### (2) Synthesis of Compound 7

Under argon atmosphere, to Compound 4 (31.0 mg, 74.6 µmol) were added N,N-dimethylformamide (7.5 mL), HATU (34.0 mg, 90.0 µmol) and N,N-diisopropylethylamine (54.6 µL, 313 µmol), and the mixture was stirred at room temperature for 5 min. Then, (S)-1-(2-aminoacetyl)-4,4-difluoropyrrolidine-2-carbonitrile (42.3 mg, 224 µmol) was added thereto, and the mixture was stirred overnight at room temperature. The reaction solution was concentrated under reduced pressure, and dried in vacuum to give Compound 5 as a yellow solid.

To the obtained Compound 5 was added dichloromethane (1.9 mL), and the mixture was cooled to 0°C. After cooling, trifluoroacetic acid (475 µL) was added thereto, and the mixture was stirred at room temperature for 2 hr. The reaction solution was concentrated under reduced pressure, and dried in vacuum to give Compound 6 as a white solid.

Under argon atmosphere, to the obtained Compound 6 were added N,N-dimethylformamide (1.0 mL), HATU (14.0 mg, 36.8 umol) and N,N-diisopropylethylamine (20.0 µL, 115 µmol), and the mixture was stirred at room temperature for 5 min. Then, 4-(carboxymethyl)phenylboronic acid (8.0 mg, 44.5 µmol) was added thereto, and the mixture was stirred overnight at room temperature. The reaction solution was concentrated under reduced pressure, and the residue was purified by high-performance liquid chromatography to give Compound 7 as a white solid (0.6 mg, yield 1% in 4 steps).

### Synthesis Example 2 Synthesis of Compound 13 (Borono(Cl)-PEG2-6Qui-FAPI(F))

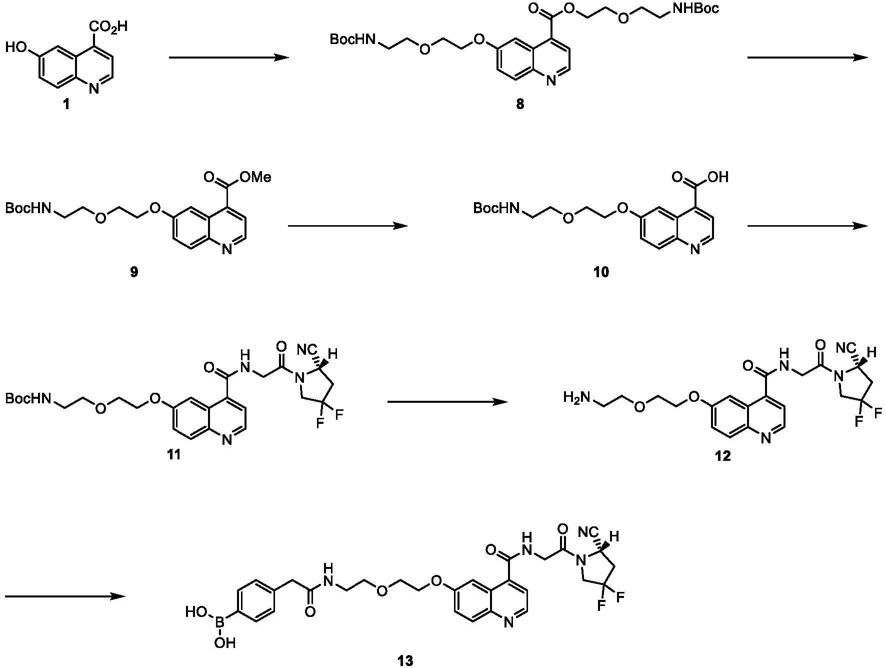

### (1) Synthesis of Compound 8

Under argon atmosphere, to commercially available Compound 1 (247 mg, 915 µmol) were added N,N-dimethylformamide (18.3 mL), potassium carbonate (1.26 g, 9.15 mmol) and N-[2-(2-bromoethoxy)ethyl]carbamic acid tert-butyl (574 µL, 2.74 mmol), and the mixture was stirred at 60°C for 21 hr. After cooling to room temperature, the mixture was subjected to extraction with ethyl acetate, and the organic layer was washed with saturated brine. The obtained organic layer was dried over sodium sulfate, and the sodium sulfate was removed by filtration. The solvent was evaporated under reduced pressure, and the residue was purified by silica gel column chromatography to give Compound 8 as a yellow oil (416.6 mg, yield 90%).
¹H-NMR (CDCl₃) δ: 8.76 (d, J = 4.0 Hz, 1H), 8.11 (s, 1H), 7.98 (d, J = 9.2 Hz, 1H), 7.81 (d, J = 3.7 Hz, 1H), 7.35 (d, J = 9.0 Hz, 1H), 5.47 (br d, J = 10.0 Hz, 2H), 4.48 (s, 2H), 4.18 (s, 2H), 3.81 (s, 2H), 3.77 (s, 2H), 3.57-3.56 (m, 4H), 3.31 (s, 4H), 1.38 (s, 9H), 1.35 (s, 9H).

### (2) Synthesis of Compound 9

To Compound 8 (301 mg, 534 µmol) were added methanol (10.7 mL) and sodium methoxide (19.7 mg, 534 µmol), and the mixture was stirred at room temperature for 1 hr. To the reaction solution was added saturated aqueous ammonium chloride solution, and the mixture was subjected to extraction with ethyl acetate, and the organic layer was washed with water and saturated brine. The obtained organic layer was dried over sodium sulfate, and the sodium sulfate was removed by filtration. The solvent was evaporated under reduced pressure, and the residue was purified by silica gel column chromatography to give Compound 9 as a yellow oil (187.3 mg, yield 93%).
¹H-NMR (CDCl₃) δ: 8.75 (t, J = 2.1 Hz, 1H), 8.16 (t, J = 2.5 Hz, 1H), 7.97 (dt, J = 9.2, 2.1 Hz, 1H), 7.81-7.79 (m, 1H), 7.36 (dt, J = 9.2, 2.4 Hz, 1H), 5.18 (br s, 1H), 4.20 (t, J = 4.4 Hz, 2H), 3.92 (s, 3H), 3.81 (dd, J = 5.2, 3.7 Hz, 2H), 3.56 (t, J = 4.4 Hz, 2H), 3.29 (d, J = 4.3 Hz, 2H), 1.35 (s, 9H).

### (3) Synthesis of Compound 10

To Compound 9 (32.5 mg, 83.2 umol) were added tetrahydrofuran/1,4-dioxane/water (4/2/1) mixed solvent (2.8 mL), and lithium hydroxide monohydrate (34.9 mg, 832 µmol), and the mixture was stirred at room temperature for 40 min. To the reaction solution was added 0.5 N hydrogen chloride aqueous solution, and the mixture was subjected to extraction with dichloromethane, and the organic layer was washed with water and saturated brine. The obtained organic layer was dried over sodium sulfate, and the sodium sulfate was removed by filtration. The solvent was evaporated under reduced pressure, and the residue was purified by silica gel column chromatography to give Compound 10 quantitatively.
¹H-NMR (DMSO-D₆) δ: 8.86 (d, J = 4.4 Hz, 1H), 8.18 (d, J = 2.7 Hz, 1H), 8.02 (d, J = 9.2 Hz, 1H), 7.92 (d, J = 4.4 Hz, 1H), 7.52 (dd, J = 9.2, 2.9 Hz, 1H), 6.77 (br s, 1H), 4.23 (t, J = 4.4 Hz, 2H), 3.81 (t, J = 4.4 Hz, 2H), 3.49 (t, J = 6.0 Hz, 2H), 3.11 (q, J = 5.8 Hz, 2H), 1.36 (s, 9H).

### (4) Synthesis of Compound 12

Under argon atmosphere, to Compound 10 (31.3 mg, 83.2 umol) were added N,N-dimethylformamide (8.3 mL), HATU (47.5 mg, 125 µmol) and N,N-diisopropylethylamine (29.0 µL, 116 µmol), and the mixture was stirred at room temperature for 5 min. Then, (S)-1-(2-aminoacetyl)-4,4-difluoropyrrolidine-2-carbonitrile (47.3 mg, 250 µmol) was added thereto, and the mixture was stirred overnight at room temperature. The reaction solution was evaporated under reduced pressure, and dried in vacuum to give Compound 11 as a yellow solid.

To the obtained Compound 11 was added dichloromethane (2.1 mL), and the mixture was cooled to 0°C. After cooling, trifluoroacetic acid (525 µL) was added thereto, and the mixture was stirred at room temperature for 2 hr. The reaction solution was evaporated under reduced pressure, and the residue was purified by high-performance liquid chromatography to give Compound 12 as a white solid (32.4 mg, yield 87% in 2 steps). ¹H-NMR (DMSO-D₆) δ: 8.83 (d, J = 4.4 Hz, 1H), 8.01 (d, J = 9.3 Hz, 1H), 7.88 (d, J = 2.7 Hz, 1H), 7.84 (br s, 2H), 7.53 (d, J = 4.4 Hz, 1H), 7.49 (dd, J = 9.2, 2.8 Hz, 1H), 5.14 (dd, J = 9.3, 2.8 Hz, 1H), 4.32 (t, J = 4.4 Hz, 3H), 4.24 (d, J = 6.0 Hz, 2H), 4.20-4.10 (br m, 1H), 3.89 (t, J = 4.2 Hz, 2H), 3.69 (dd, J = 9.8, 4.4 Hz, 2H), 3.04-2.99 (m, 2H), 2.97-2.83 (m, 2H).

### (5) Synthesis of Compound 13

Under argon atmosphere, to Compound 12 (2.8 mg, 6.3 µmol) were added N,N-dimethylformamide (1.0 mL), HATU (14.0 mg, 36.8 umol) and N,N-diisopropylethylamine (20.0 µL, 115 µmol), and the mixture was stirred at room temperature for 5 min. Then, 4-(carboxymethyl)phenylboronic acid (8.0 mg, 44.5 µmol) was added thereto, and the mixture was stirred overnight at room temperature. The reaction solution was evaporated under reduced pressure, and the residue was purified by high-performance liquid chromatography to give Compound 13 as a white solid quantitatively.
¹H-NMR (DMSO-D₆) δ: 8.81 (d, J = 4.5 Hz, 1H), 7.99 (d, J = 9.1 Hz, 1H), 7.92 (s, 2H), 7.89 (d, J = 2.8 Hz, 1H), 7.69 (d, J = 7.8 Hz, 2H), 7.51 (d, J = 4.2 Hz, 1H), 7.47 (dd, J = 9.3, 2.8 Hz, 1H), 7.20 (d, J = 7.6 Hz, 2H), 5.14 (d, J = 6.8 Hz, 1H), 4.33-4.31 (m, 1H), 4.28 (d, J = 3.8 Hz, 2H), 4.24 (t, J = 6.5 Hz, 2H), 4.17-4.11 (m, 1H), 3.82 (t, J = 4.3 Hz, 2H), 3.50 (t, J = 5.8 Hz, 2H), 3.40 (s, 2H), 3.24 (t, J = 5.6 Hz, 2H), 2.91-2.88 (m, 1H), 2.82-2.80 (br m, 1H).

### Synthesis Example 3 Synthesis of Compound 19 (Borono(C1)-Gly(1)-8Qui-FAPI(F))

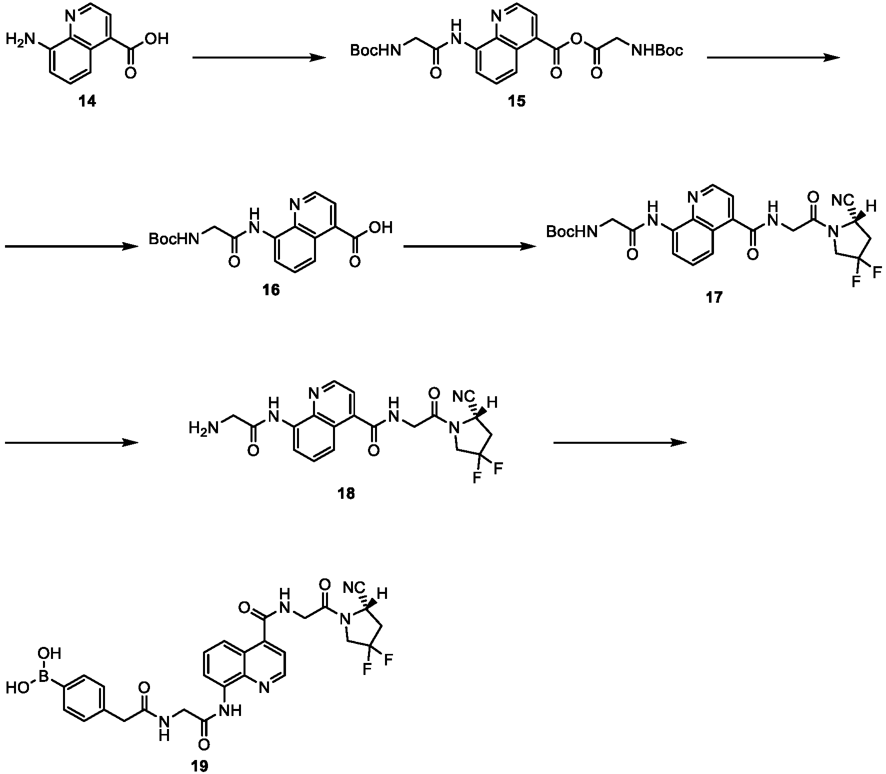

### (1) Synthesis of Compound 16

Under argon atmosphere, to N-(tert-butoxycarbonyl)glycine (55.9 mg, 319 µmol) were added N,N-dimethylformamide (5.3 mL), HATU (121 mg, 319 µmol) and N,N-diisopropylethylamine (66.6 µL, 382 µmol), and the mixture was stirred at room temperature for 5 min. Then, commercially available Compound 14 (10.0 mg, 53.1 µmol) was added thereto, and the mixture was stirred at room temperature for 23 hr. The reaction solution was concentrated under reduced pressure, and dried in vacuum to give Compound 15 as a yellow solid.

To the obtained Compound 15 were added tetrahydrofuran/1,4-dioxane/water (4/2/1) mixed solvent (5.3 mL), and potassium carbonate (73.4 mg, 531 µmol), and the mixture was stirred overnight at room temperature. To the reaction solution was added saturated aqueous ammonium chloride solution, and the mixture was subjected to extraction with dichloromethane, and the organic layer was washed with water and saturated brine. The obtained organic layer was dried over sodium sulfate, and the sodium sulfate was removed by filtration. The solvent was evaporated under reduced pressure, and the residue was purified by high-performance liquid chromatography to give Compound 16 as a white solid (11.8 mg, yield 64% in 2 steps).
¹H-NMR (CD₃OD) δ: 8.96 (d, J = 4.3 Hz, 1H), 8.73 (d, J = 7.7 Hz, 1H), 8.48 (d, J = 8.6 Hz, 1H), 8.04 (d, J = 4.3 Hz, 1H), 7.65 (t, J = 8.3 Hz, 1H), 3.97 (s, 2H), 1.53 (s, 9H).

### (2) Synthesis of Compound 18

Under argon atmosphere, to Compound 16 (4.0 mg, 9.6 µmol) were added N,N-dimethylformamide (1.0 mL), HATU (11.0 mg, 28.8 µmol) and N,N-diisopropylethylamine (21.4 µL, 123 µmol), and the mixture was stirred at room temperature for 5 min. Then, (S)-1-(2-aminoacetyl)-4,4-difluoropyrrolidine-2-carbonitrile (19.5 mg, 103 µmol) was added thereto, and the mixture was stirred overnight at room temperature. The reaction solution was concentrated under reduced pressure, and dried in vacuum to give Compound 17 as a yellow solid.

To the obtained Compound 17 was added dichloromethane (3.4 mL), and the mixture was cooled to 0°C. After cooling, trifluoroacetic acid (855 µL) was added thereto, and the mixture was stirred at room temperature for 2 hr. The reaction solution was concentrated under reduced pressure, and the residue was purified by high-performance liquid chromatography to give Compound 18 as a yellow solid (7.5 mg, yield 53% in 2 steps). ¹H-NMR (CD₃OD) δ: 8.97 (d, J = 4.3 Hz, 1H), 8.70 (d, J = 7.6 Hz, 1H), 8.09 (d, J = 8.3 Hz, 1H), 7.70 (d, J = 4.3 Hz, 1H), 7.64 (t, J = 8.1 Hz, 1H), 5.15 (dd, J = 9.4, 2.8 Hz, 1H), 4.31 (dd, J = 39.5, 16.9 Hz, 2H), 4.28-4.21 (m, 1H), 4.19-4.10 (m, 1H), 4.13 (s, 2H), 3.07-3.02 (br m, 1H), 2.93-2.91 (br m, 1H), 2.81-2.79 (br m, 1H) .

### (3) Synthesis of Compound 19

Under argon atmosphere, to 4-(carboxymethyl)phenylboronic acid (5.2 mg, 28.8 µmol) were added N,N-dimethylformamide (1.0 mL), HATU (11.0 mg, 28.8 µmol) and N,N-diisopropylethylamine (6.0 µL, 34.6 µmol), and the mixture was stirred at room temperature for 5 min. Then, Compound 18 (2.8 mg, 6.3 µmol) was added thereto, and the mixture was stirred overnight at room temperature. The reaction solution was concentrated under reduced pressure, and the residue was purified by high-performance liquid chromatography to give Compound 19 as a white solid (3.9 mg, yield 70%) .
¹H-NMR (DMSO-D₆) δ: 10.39 (s, 1H), 9.14 (t, J = 5.9 Hz, 1H), 8.98 (d, J = 4.2 Hz, 1H), 8.72 (t, J = 5.8 Hz, 1H), 8.67 (d, J = 7.4 Hz, 1H), 8.01 (d, J = 8.5 Hz, 1H), 7.96 (s, 2H), 7.72 (d, J = 7.8 Hz, 2H), 7.66 (dd, J = 11.4, 6.1 Hz, 2H), 7.33 (d, J = 7.8 Hz, 2H), 5.17 (dd, J = 9.4, 2.2 Hz, 1H), 4.34-4.30 (m, 1H), 4.24 (ddd, J = 31.7, 17.0, 5.8 Hz, 2H), 4.18-4.12 (m, 1H), 4.06 (d, J = 5.9 Hz, 2H), 3.63 (s, 2H), 2.94-2.89 (br m, 1H), 2.82 (t, J = 13.9 Hz, 1H) . LRMS (ESI-Q-TOF) calcd for C₂₇H₂₅BF₂N₆O₆,

### Synthesis Example 4 Synthesis of Compound 29 (Borono(C0)-GlcN-Pip-6Qui-FAPI(F))

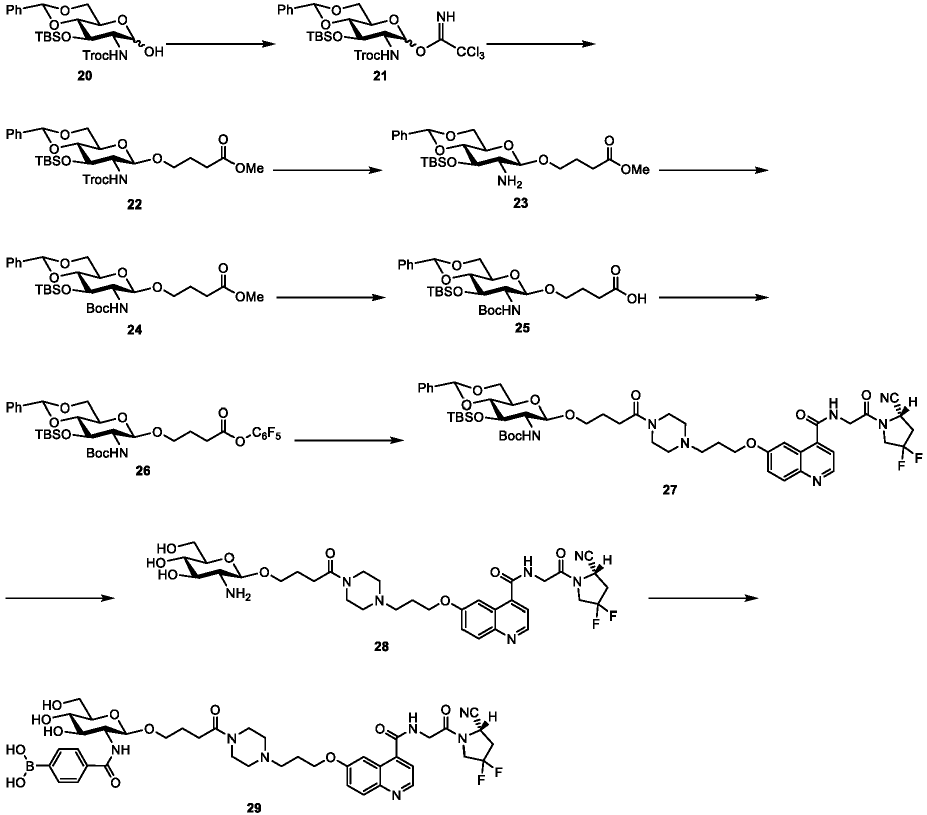

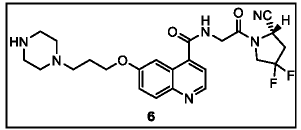

### (1) Synthesis of Compound 22

Under argon atmosphere, to the known Compound 20 (4.6 g, 8.26 mmol) were added dichloromethane (160 mL), trichloroacetonitrile (6.6 mL, 82.6 mmol) and cesium carbonate (5.38 g, 16.5 mmol), and the mixture was stirred at room temperature for 40 min. Then, the mixture was filtered through Celite with dichloromethane. The solvent was evaporated under reduced pressure, and the residue was roughly purified by silica gel column chromatography to give Compound 21 as a white amorphous solid (4.6 g).

Under argon atmosphere, to the obtained Compound 21 were added dichloromethane (138 mL), molecular sieve 4A and 4-hydroxybutyric acid methyl ester (2.3 mL, 20.7 mmol), and the mixture was cooled to 0°C. After cooling, trimethylsilyl trifluoromethanesulfonate (125 µL, 804 µmol) was added thereto, and the mixture was stirred at 0°C for 20 min. After raising the temperature to room temperature, to the reaction solution was added triethylamine, and the mixture was subjected to extraction with ethyl acetate, and the organic layer was washed with water and saturated brine. The obtained organic layer was dried over sodium sulfate, and the sodium sulfate was removed by filtration. The solvent was evaporated under reduced pressure, and the residue was purified by silica gel column chromatography to give Compound 22 as a white amorphous solid (3.2 g, yield 59% in 2 steps). ¹H-NMR (CDCl₃) δ: 7.44 (d, J = 7.0 Hz, 2H), 7.31 (d, J = 7.2 Hz, 3H), 5.75 (d, J = 8.3 Hz, 1H), 5.37 (s, 1H), 4.70 (dd, J = 19.3, 11.9 Hz, 2H), 4.52 (d, J = 8.2 Hz, 1H), 4.26 (dd, J = 10.0, 4.2 Hz, 1H), 3.90 (t, J = 8.9 Hz, 1H), 3.87-3.82 (m, 1H), 3.70 (t, J = 10.1 Hz, 1H), 3.63 (s, 3H), 3.49 (dt, J = 11.8, 4.9 Hz, 1H), 3.43 (t, J = 9.0 Hz, 2H), 3.38-3.33 (m, 1H), 2.39-2.36 (m, 2H), 1.88-1.82 (m, 2H), 0.81 (s, 9H), 0.03 (s, 3H), -0.03 (s, 3H).

### (2) Synthesis of Compound 23

To zinc (1.5 g) was added water, and the mixture was stirred under ultrasonic irradiation for 30 min. 2% Aqueous copper sulfate solution was added thereto, and the mixture was washed with water. The zinc-copper couple was collected by filtration. Under argon atmosphere, to Compound 22 (500 mg, 761 µmol) were added tetrahydrofuran (38 mL), acetic acid (AcOH, 38 mL), cesium carbonate (5.38 g, 16.5 mmol) and zinc-copper couple, and the mixture was stirred at room temperature for 2 hr. Then, the mixture was filtered through Celite with ethyl acetate. The filtrate was azeotroped with toluene, to residue was added saturated aqueous sodium hydrogencarbonate solution, and the mixture was subjected to extraction with ethyl acetate, and the organic layer was washed with water and saturated brine. The solvent was evaporated under reduced pressure, and the residue was purified by silica gel column chromatography to give Compound 23 as a colorless amorphous solid quantitatively.
¹H-NMR (CDCl₃) δ: 7.48-7.46 (m, 2H), 7.37-7.35 (m, 3H), 5.49 (s, 1H), 4.34 (dd, J = 7.9, 1.4 Hz, 1H), 4.29 (dd, J = 10.5, 4.7 Hz, 1H), 3.94 (dt, J = 11.3, 4.9 Hz, 1H), 3.77 (t, J = 10.1 Hz, 1H), 3.70-3.68 (m, 1H), 3.68 (s, 3H), 3.58 (dt, J = 11.7, 4.8 Hz, 1H), 3.47 (q, J = 7.8 Hz, 1H), 3.44-3.40 (m, 1H), 2.84 (t, J = 8.6 Hz, 1H), 2.44 (td, J = 7.4, 2.1 Hz, 2H), 1.99-1.93 (m, 2H), 0.85 (s, 9H), 0.09 (s, 3H), -0.02 (s, 3H).

### (3) Synthesis of Compound 25

To Compound 23 (328.5 mg, 682 mmol) were added dichloromethane (68.2 mL), 2,6-lutidine (219.7 µL, 2.05 mmol) and di-tert-butyl dicarbonate (2.0 mL, 6.82 mmol), and the mixture was stirred at room temperature for two nights. To the reaction solution was added saturated aqueous ammonium chloride solution, and the mixture was subjected to extraction with dichloromethane, and the organic layer was washed with water and saturated brine. The obtained organic layer was dried over sodium sulfate, and the sodium sulfate was removed by filtration. The solvent was evaporated under reduced pressure, and the residue was roughly purified by silica gel column chromatography to give Compound 24 as a white amorphous solid.

To the obtained Compound 24 were added tetrahydrofuran/methanol/water (3/2/2) mixed solvent (70 mL), and lithium hydroxide monohydrate (171.6 mg, 4.10 mmol), and the mixture was stirred overnight at room temperature. To the reaction solution was added 0.5 N hydrogen chloride aqueous solution until the pH reached approximately 3, and the mixture was subjected to extraction with dichloromethane, and the organic layer was washed with water and saturated brine. The obtained organic layer was dried over sodium sulfate, and the sodium sulfate was removed by filtration. The solvent was evaporated under reduced pressure, and the residue was purified by silica gel column chromatography to give Compound 25 as a white amorphous solid (303.6 mg, yield 78% in 2 steps).
¹H-NMR (CD₃OD) δ: 7.46 (dd, J = 6.6, 2.9 Hz, 2H), 7.33 (dd, J = 6.4, 2.6 Hz, 3H), 6.73 (d, J = 9.7 Hz, 1H), 5.51 (s, 1H), 4.43 (d, J = 8.4 Hz, 1H), 4.24 (dd, J = 10.3, 4.9 Hz, 1H), 3.87 (dt, J = 11.0, 5.0 Hz, 1H), 3.78 (t, J = 9.2 Hz, 1H), 3.76 (t, J = 10.1 Hz, 1H), 3.53 (dt, J = 11.6, 4.9 Hz, 1H), 3.45 (q, J = 9.7 Hz, 2H), 3.40-3.39 (br m, 1H), 2.46-2.35 (m, 2H), 1.86-1.81 (m, 2H), 1.45 (s, 9H), 0.84 (s, 9H), 0.06 (s, 3H), -0.03 (s, 3H).

### (4) Synthesis of Compound 29

To Compound 25 (5.8 mg, 10.3 µmol) were added N,N-dimethylformamide (1.0 mL), pyridine (4.2 µL, 51.5 µmol) and pentafluorophenyl trifluoroacetate (8.8 µL, 51.5 µmol), and the mixture was stirred at room temperature for 1.5 hr. The reaction solution was concentrated under reduced pressure, and dried in vacuum to give Compound 26 as a white solid.

To the obtained Compound 26 were added N,N-dimethylformamide (1.0 mL), Compound 6 (10.3 µmol) and N,N-diisopropylethylamine (34.0 µL, 146 µmol), and the mixture was stirred overnight at room temperature. The reaction solution was concentrated under reduced pressure, and the residue was roughly purified by preparative thin-layer chromatography to give Compound 27 as a white solid.

To the obtained Compound 27 were added dichloromethane (1.0 mL) and water (100 µL), and the mixture was cooled to 0°C. After cooling, trifluoroacetic acid (100 µL) was added thereto, and the mixture was stirred at room temperature for 2.5 hr. The reaction solution was concentrated under reduced pressure, and the residue was roughly purified by high-performance liquid chromatography to give Compound 28 as a white solid.

Under argon atmosphere, to 4-carboxyphenylboronic acid (16.3 mg, 98.1 µmol) were added N,N-dimethylformamide (1.0 mL), HATU (37.3 mg, 98.1 µmol) and N,N-diisopropylethylamine (51.2 µL, 294 µmol), and the mixture was stirred at room temperature for 5 min. Then, the obtained Compound 28 was added thereto, and the mixture was stirred at room temperature for 3 hr. The reaction solution was concentrated under reduced pressure, and the residue was purified by high-performance liquid chromatography to give Compound 29 as a white solid (3.1 mg, yield 35% in 4 steps). HRMS (ESI-LTQ-orbitrap) calcd for C₄₁H₅₁BF₂N₇O₁₂H (M + H)⁺ 882.3651, found: 882.3657

### Synthesis Example 5 Synthesis of Compound 32 (Borono(C0)-GlcN-Pip-6Qui-FAPI(H))

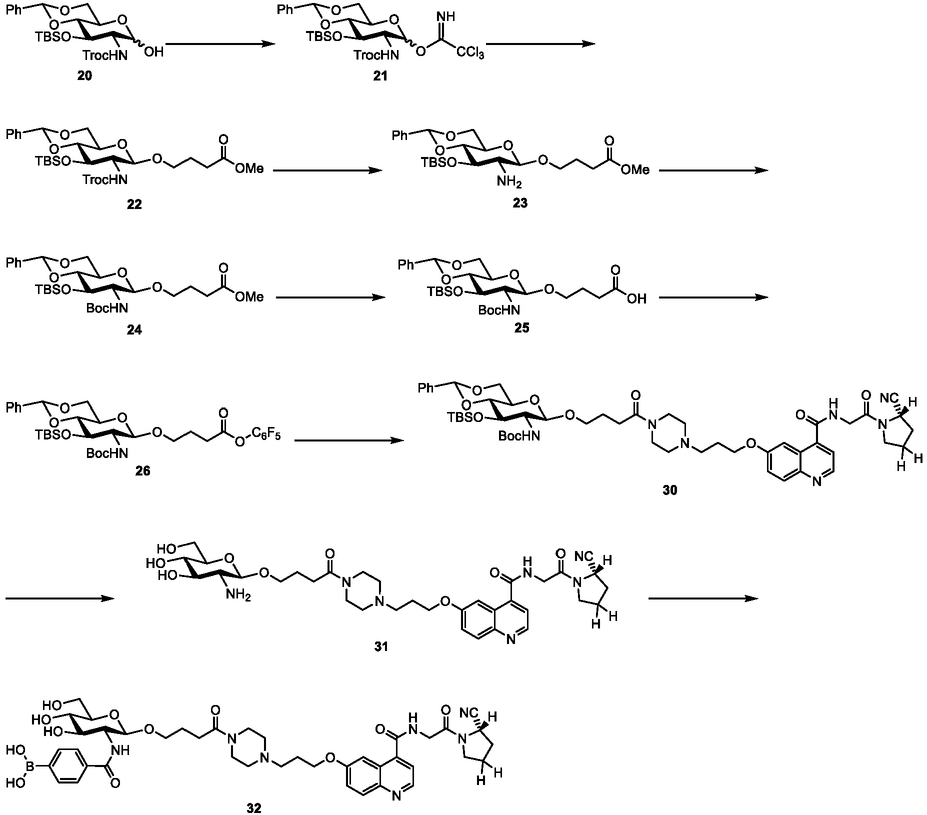

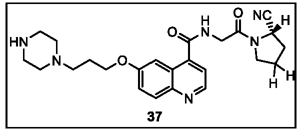

To Compound 25 (2.9 mg, 5.09 µmol) synthesized in the same manner as in Synthesis Example 4 were added N,N-dimethylformamide (510 µL), pyridine (2.1 µL, 25.5 µmol) and pentafluorophenyl trifluoroacetate (4.3 µL, 25.5 µmol), and the mixture was stirred at room temperature for 1.5 hr. The reaction solution was concentrated under reduced pressure, and dried in vacuum to give Compound 26 as a white solid.

To the obtained Compound 26 were added N,N-dimethylformamide (1.0 mL), Compound 37 (5.09 µmol, synthesized in Reference Example 1 described below) and N,N-diisopropylethylamine (4.3 µL, 24.4 µmol), and the mixture was stirred overnight at room temperature. The reaction solution was concentrated under reduced pressure, and the residue was roughly purified by preparative thin-layer chromatography to give Compound 30 as a white solid.

To the obtained Compound 30 were added dichloromethane (500 µL) and water (50 µL), and the mixture was cooled to 0°C. After cooling, trifluoroacetic acid (50 µL) was added thereto, and the mixture was stirred at room temperature for 2.5 hr. The reaction solution was concentrated under reduced pressure, and the residue was roughly purified by high-performance liquid chromatography to give Compound 31 as a white solid.

Under argon atmosphere, to 4-carboxyphenylboronic acid (10.1 mg, 61.1 µmol) were added N,N-dimethylformamide (1.0 mL), HATU (23.2 mg, 61.6 µmol) and N,N-diisopropylethylamine (31.9 µL, 183 µmol), and the mixture was stirred at room temperature for 5 min. Then, the obtained Compound 31 was added thereto, and the mixture was stirred overnight at room temperature. The reaction solution was concentrated under reduced pressure, and the residue was purified by high-performance liquid chromatography to give Compound 32 as a white solid 2.9 mg (yield 67% in 4 steps).
HRMS (ESI-orbitrap) calcd for C₄₁H₅₃BN₇O₁₂ (M + H)⁺ 846.3840, found: 846.3844

### Synthesis Example 6 Synthesis of Compound 35 (Borono(C1)-GlcN-PEG-6Qui-FAPI(F))

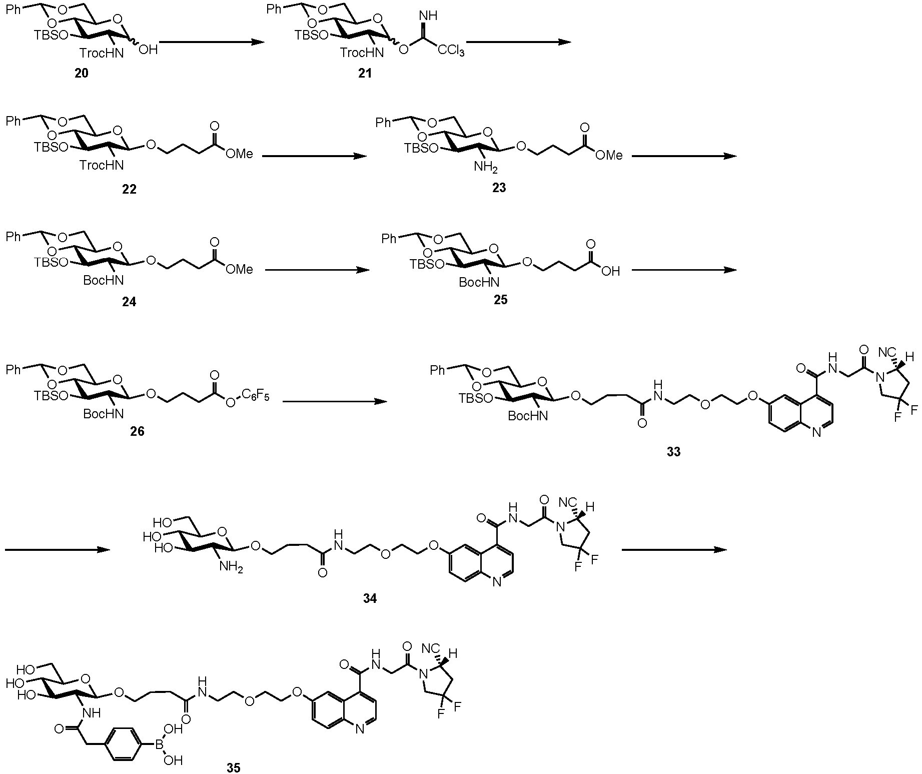

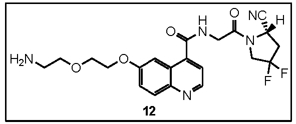

To Compound 25 (10.0 mg, 17.8 µmol) synthesized in the same manner as in Synthesis Example 4 were added N,N-dimethylformamide (1.8 mL), pyridine (7.1 µL, 88.1 µmol) and pentafluorophenyl trifluoroacetate (15.0 µL, 88.1 µmol), and the mixture was stirred at room temperature for 1.5 hr. The reaction solution was concentrated under reduced pressure, and dried in vacuum to give Compound 26 as a white solid.

To the obtained Compound 26 were added N,N-dimethylformamide (800 µL), Compound 12 (7.9 mg, 17.6 µmol) and N,N-diisopropylethylamine (84.5 µL, 14.7 µmol), and the mixture was stirred overnight at room temperature. The reaction solution was concentrated under reduced pressure, and dried in vacuum to give Compound 33 as a white solid.

To the obtained Compound 33 were added dichloromethane (1.6 mL) and water (160 µL), and the mixture was cooled to 0°C. After cooling, trifluoroacetic acid (160 µL) was added thereto, and the mixture was stirred at room temperature for 2.5 hr. The reaction solution was concentrated under reduced pressure, and the residue was purified by high-performance liquid chromatography to give Compound 34 as a white solid.

Under argon atmosphere, to 4-(carboxymethyl)phenylboronic acid (2.0 mg, 11.2 µmol) were added N,N-dimethylformamide (500 µL), HATU (4.3 mg, 11.2 µmol) and N,N-diisopropylethylamine (2.4 µL, 13.5 µmol), and the mixture was stirred at room temperature for 5 min. Then, the obtained Compound 34 was added thereto, and the mixture was stirred at room temperature for 3 hr. The reaction solution was concentrated under reduced pressure, and the residue was purified by high-performance liquid chromatography to give Compound 35 as a white solid 3.6 mg (yield 24% in 4 steps). LRMS (ESI-Q-TOF) calcd for C₃₉H₄₈BN₆O₁₃ (M + H)⁺ 857.33, found: 857.33

### Reference Example 1 Synthesis of Compound 37

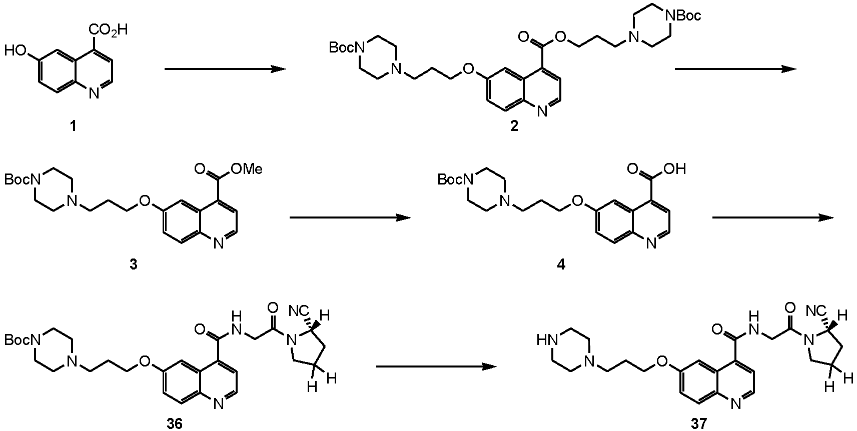

### (1) Synthesis of Compound 36

Under argon atmosphere, to Compound 4 (3.8 mg, 9.23 µmol) synthesized in the same manner as in Synthesis Example 1 were added N,N-dimethylformamide (1.0 mL), HATU (4.2 mg, 11.1 µmol) and N,N-diisopropylethylamine (1.9 µL, 11.1 µmol), and the mixture was stirred at room temperature for 5 min. Then, (S)-1-(2-aminoacetyl)-pyrrolidine-2-carbonitrile (10.2 mg, 27.8 µmol) was added thereto, and the mixture was stirred at room temperature for two nights. The reaction solution was concentrated under reduced pressure, and the residue was purified by silica gel column chromatography to give Compound 36 as a white solid 2.8 mg (yield 55%) .

### (2) Synthesis of Compound 37

To Compound 36 was added dichloromethane (130 µL), and the mixture was cooled to 0°C. After cooling, trifluoroacetic acid (33 µL) was added thereto, and the mixture was stirred at room temperature for 1 hr. The reaction solution was concentrated under reduced pressure, and dried in vacuum to give Compound 37 as a white solid (TFA salt). The entire amount of the obtained Compound 37 was used for condensation with Compound 26 in Synthesis Example 5.

### Synthesis Example 7 Synthesis of Compound 39 (Borono(C1)-PEG2-6Qui-FAPI(H))

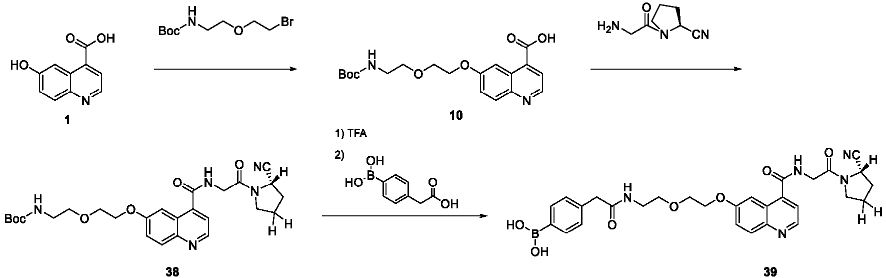

### (1) Synthesis of Compound 10

Compound 1 (1.1 g, 4.1 mmol) was dissolved in DMF (20 mL), potassium carbonate (5.7 g, 41 mmol) was added thereto, and then 1,1-dimethylethyl N-[2-(2-bromoethoxy)ethyl]carbamate (3.9 g, 15 mmol) was added dropwise thereto. The reaction solution was stirred at 60°C for 2 hr, the insoluble substance was removed by filtration, and the solvent was evaporated under reduced pressure. The obtained residue was dissolved in 60% acetonitrile-containing water (20 mL), and 5 M aqueous sodium hydroxide solution (4.1 mL, 21 mmol) was added thereto. The mixture was stirred for 1.5 hr, and neutralized to pH 4 with AcOH under ice-cooling, and the solvent was evaporated under reduced pressure. The precipitate was suspended in DMF (40 mL), water was added thereto, and the precipitate was collected by filtration, washed with water and diethyl ether, and dried to give Compound 10 (1.17 g, 75%).

### (2) Synthesis of Compound 38

(S)-1-(2-(tert-Butoxycarbonylamino)acetyl)-pyrrolidine-2-carbonitrile (440 mg, 1.7 mmol) was dissolved in cool TFA, the solution was stirred for 1 hr, and the solvent was evaporated under reduced pressure. The obtained (S)-1-(2-aminoacetyl)-pyrrolidine-2-carbonitrile TFA salt (1.7 mmol) was dissolved in NMP (10 mL), and the solution was neutralized with TEA. Compound 10 (0.62 g, 1.7 mmol), HOAt (0.24 g, 1.7 mmol) and water (1 mL) were added thereto, and then EDC (0.6 mL, 3.3 mmol) was added dropwise thereto under ice-cooling. The mixture was stirred at room temperature for 3.5 hr, and the reaction solution was diluted with ethyl acetate, and the organic layer was washed with brine, and the solvent of the organic layer was evaporated under reduced pressure. The obtained residue was dissolved in 30% AcOH-containing water, and purified by reverse-phase HPLC to give Compound 38 (0.43 g, 51%).

### (3) Synthesis of Compound 39

Compound 38 (0.23 g, 0.45 mmol) was dissolved in cool TFA, the solution was stirred for 30 min, and the solvent was evaporated under reduced pressure. The obtained residue was dissolved in DMF (2 mL), and the solution was neutralized with TEA. 4-(Carboxymethyl)phenylboronic acid pinacol ester (89 mg, 0.50 mmol), HOAt (67 mg, 0.50 mmol) and water (0.2 mL) were added thereto, and then EDC (0.11 mL, 0.63 mmol) was added dropwise thereto under ice-cooling, and the mixture was stirred at room temperature for 1 hr. The reaction solution was diluted with 0.1% TFA-containing water, and purified by reverse-phase HPLC. The fraction containing the target product was lyophilized to give Compound 39 as a lyophilized powder (0.24 g, 93%). HPLC purity: 99.8%, ESI-MS MH+: 574.3 (theoretical value: 574.2)

### Synthesis Example 8 Synthesis of Compound 42 (Borono(C1)-PEG4-6Qui-FAPI(H))

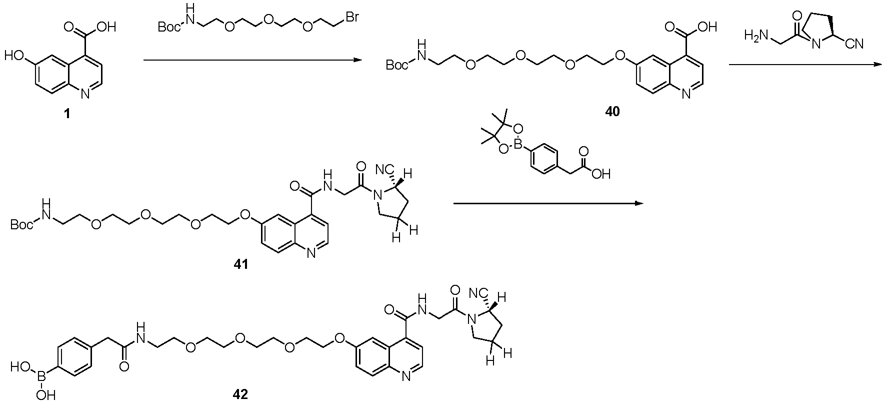

### (1) Synthesis of Compound 40

Compound 1 (0.32 g, 1.2 mmol) was dissolved in DMF (5 mL), potassium carbonate (1.6 g, 12 mmol) was added thereto, and 1-(tert-butoxycarbonylamino)-3,6,9-trioxaundecanyl-11-bromide (1.3 g, 3.6 mmol) was added dropwise thereto. The reaction solution was stirred at 65°C for 4 hr, the insoluble substance was removed by filtration, and the solvent was evaporated under reduced pressure. The obtained residue was dissolved in 60% acetonitrile-containing water (10 mL), 5 M aqueous sodium hydroxide solution (1.2 mL, 6.0 mmol) was added thereto, and the mixture was stirred at room temperature for 30 min. The mixture was neutralized with AcOH under ice-cooling, and the solvent was evaporated under reduced pressure. The residue was dissolved in ethyl acetate, the solution was washed with saturated brine, and dried over anhydrous sodium sulfate, and the solvent was evaporated under reduced pressure. The obtained residue was dissolved in small amount of chloroform, hexane was added thereto for solidification, and the solid was collected by filtration, and dried to give Compound 40 (0.55 g, 99%).

### (2) Synthesis of Compound 41

(S)-1-(2-(tert-Butoxycarbonylamino)acetyl)-pyrrolidine-2-carbonitrile (301 mg, 1.2 mmol) was dissolved in cool TFA, the solution was stirred for 40 min, and the solvent was evaporated under reduced pressure. The obtained (S)-1-(2-aminoacetyl)-pyrrolidine-2-carbonitrile TFA salt was dissolved in DMF (9 mL), and the solution was neutralized with TEA. Compound 40 (0.55 g, 1.2 mmol), HOAt (0.18 g, 1.3 mmol) and water (1 mL) were added thereto, and the EDC (0.24 mL, 1.3 mmol) was added dropwise thereto under ice-cooling. The mixture was stirred at room temperature for 1.5 hr, and the solvent was evaporated under reduced pressure. The residue was dissolved in 10% AcOH-containing water, and purified by reverse-phase HPLC. The fraction containing the target product was lyophilized to give Compound 41 as a lyophilized powder (0.56 g, 78%).

### (3) Synthesis of Compound 42

Compound 41 (0.21 g, 0.35 mmol) was dissolved in TFA (5 mL), the solution was stirred for 1 hr, and the solvent was evaporated under reduced pressure. The obtained residue was dissolved in DMF (2 mL), and the solution was neutralized with TEA. 4-(Carboxymethyl)phenylboronic acid pinacol ester (0.10 g, 0.39 mmol), HOAt (52 mg, 0.39 mmol) and water (0.2 mL) were added thereto, and then EDC (69 µL, 0.39 mmol) was added dropwise thereto under cooling at -78°C, and the mixture was stirred at room temperature for 1.5 hr. The solvent was evaporated under reduced pressure, the residue was dissolved in 20% AcOH-containing water (2 mL), and the solution was stirred at room temperature for 17 hr. The reaction solution was diluted with water, and purified by reverse-phase HPLC. The fraction containing the target product was lyophilized to give Compound 42 as a lyophilized powder (0.14 g, 61%). HPLC purity: 99.7%, ESI-MS MH+: 662.3 (theoretical value: 662.3)

### Synthesis Example 9 Synthesis of Compound 44 (Borono(C1)-Pip-6Qui-FAPI(H))

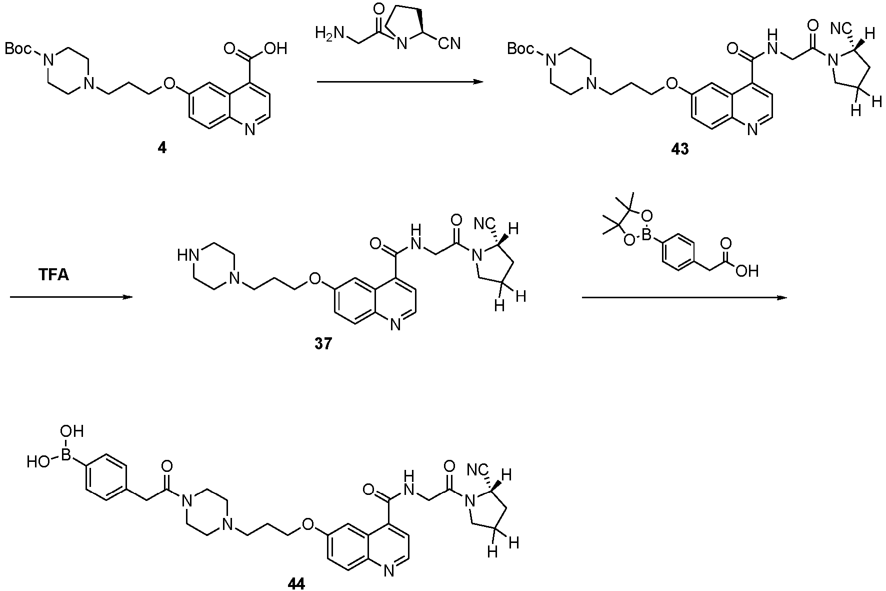

### (1) Synthesis of Compound 37

(S)-1-(2-(tert-Butoxycarbonylamino)acetyl)-pyrrolidine-2-carbonitrile (0.16 g, 0.66 mmol) was dissolved in 95% TFA/acetonitrile (8.4 mL), and the solution was stirred for 40 min. The solvent was evaporated under reduced pressure, and the residue was dissolved in water, and the solution was lyophilized. The obtained (S)-1-(2-aminoacetyl)-pyrrolidine-2-carbonitrile TFA salt was dissolved in 90% DMF-containing water (6 mL), and the solution was neutralized with TEA. Compound 4 (0.35 g, 0.66 mmol) and HOAt (99 mg, 0.73 mmol) were added thereto, and then EDC (0.13 mL, 0.73 mmol) was added dropwise thereto under ice-cooling. The mixture was stirred at room temperature for 1.5 hr, and the solvent was evaporated under reduced pressure to give Compound 43. This was dissolved in 98% TFA/acetonitrile (20 mL), the solution was stirred for 30 min, and the solvent was evaporated under reduced pressure. Diisopropyl ether was added thereto for solidification, and the solid was collected by filtration. The filtered solid was dissolved in water, and purified by reverse-phase HPLC. The fraction containing the target product was lyophilized to give Compound 37 (TFA salt, 0.21 g, 48%).

### (2) Synthesis of Compound 44

Compound 37 (0.20 g, 0.30 mmol) was dissolved in 90% DMF-containing water (6 mL), and the solution was neutralized with TEA. 4-(Carboxymethyl)phenylboronic acid pinacol ester (75 mg, 0.29 mmol) and HOAt (39 mg, 0.29 mmol) were added thereto, and then EDC (58 µL, 0.32 mmol) was added dropwise thereto under ice-cooling, and the mixture was stirred at room temperature for 1 hr. The reaction solution was concentrated, the residue was diluted with 0.1% TFA/30% AcOH-containing water (7 mL), and the solution was stirred at 40°C for 1.5 hr. The reaction solution was purified by reverse-phase HPLC, and the fraction containing the target product was lyophilized to give Compound 44 as a lyophilized powder (0.15 g, 69%). HPLC purity: 99.3%, ESI-MS MH+: 613.3 (theoretical value: 613.3)

### Synthesis Example 10 Synthesis of Compound 49 (Borono(C1)-Pip-8Qui-FAPI(H))

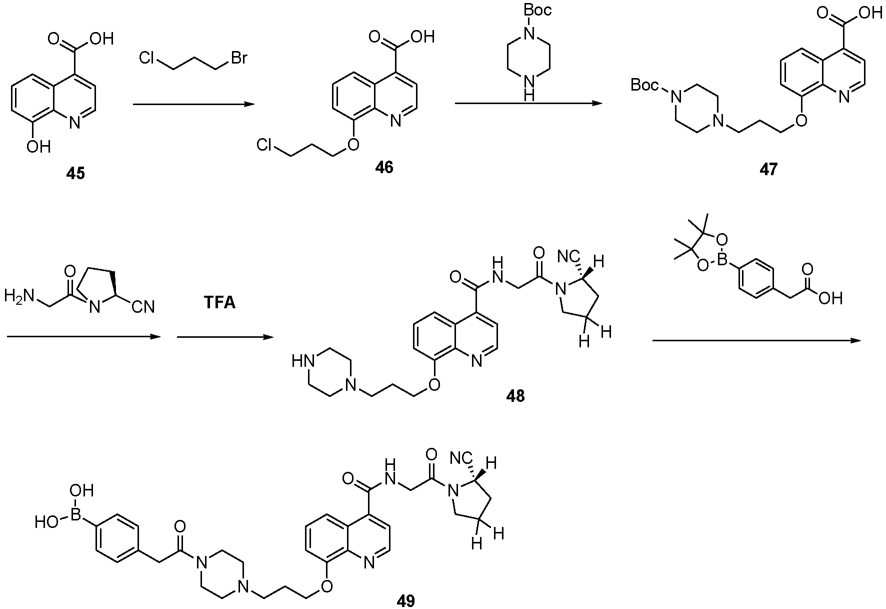

### (1) Synthesis of Compound 46

Compound 45 (0.50 g, 2.6 mmol) was dissolved in DMF (20 mL), potassium carbonate (4.4 g, 32 mmol) was added thereto, and then 1-bromo-3-chloropropane (0.91 mL, 9.3 mmol) was added dropwise thereto. The reaction solution was stirred at 65°C for 1.5 hr, the insoluble substance was removed by filtration, and the solvent of the filtrate was evaporated under reduced pressure. The obtained residue was dissolved in 50% MeCN-containing water (50 mL), 5 M aqueous sodium hydroxide solution (2.6 mL, 13 mmol) was added thereto under ice-cooling, and the mixture was stirred for 2 hr. The mixture was neutralized with AcOH, and the solvent was evaporated under reduced pressure. The obtained precipitate was collected by filtration, washed with water, and dried to give Compound 46 (0.49 g, 70%).

### (2) Synthesis of Compound 47

Compound 46 (0.49 g, 1.8 mmol), 1-(tert-butoxycarbonyl)piperazine (1.8 g, 9.8 mmol) and sodium iodide (1.5 g, 10 mmol) were dissolved in DMF (25 mL), the solution was stirred at 55°C for 20 hr, and the solvent was evaporated under reduced pressure. The obtained residue was diluted with 50% AcOH-containing water, and purified by reverse-phase HPLC, and the fraction containing the target product was lyophilized to give Compound 47 (0.79 g, 81%).

### (3) Synthesis of Compound 48

(S)-1-(2-(tert-Butoxycarbonylamino)acetyl)-pyrrolidine-2-carbonitrile (0.17 g, 0.66 mmol) was dissolved in 95% TFA/acetonitrile (8 mL), the solution was stirred for 40 min, and the solvent was evaporated under reduced pressure. The residue was dissolved in water, and the solution was lyophilized. The obtained (S)-1-(2-aminoacetyl)-pyrrolidine-2-carbonitrile TFA salt was dissolved in 90% DMF-containing water (10 mL), and the solution was neutralized with DIEA. Compound 47 (0.35 g, 0.66 mmol) and HOAt (99 mg, 0.73 mmol) were added thereto, and then EDC (0.13 mL, 0.73 mmol) was added dropwise thereto under ice-cooling. The mixture was stirred at room temperature for 1.5 hr, and the solvent was evaporated under reduced pressure. The obtained residue was dissolved in 97% TFA/acetonitrile (15 mL), the solution was stirred for 1 hr, and the solvent was evaporated under reduced pressure. To the residue was added diisopropyl ether, and the precipitate was collected by filtration. The filtered precipitate was dissolved in water, and purified by reverse-phase HPLC, and the fraction containing the target product was lyophilized to give Compound 48 (0.26 g, 59%).

### (4) Synthesis of Compound 49

Compound 48 (0.26 g, 0.39 mmol) was dissolved in 90% DMF-containing water (10 mL), and the solution was neutralized with TEA. 4-(Carboxymethyl)phenylboronic acid pinacol ester (92 mg, 0.35 mmol) and HOAt (53 mg, 0.39 mmol) were added thereto, and the EDC (79 µL, 0.43 mmol) was added dropwise thereto under ice-cooling, and the mixture was stirred at room temperature for 3 hr. The solvent was evaporated under reduced pressure, the residue was diluted with 2.5% TFA/15% AcOH-containing water (60 mL), and the mixture was stirred at 40°C for 1.5 hr. The reaction solution was purified by reverse-phase HPLC, and the fraction containing the target product was lyophilized to give Compound 49 as a lyophilized powder (0.14 g, 50%). HPLC purity: 99.9%, ESI-MS MH+: 613.3 (theoretical value: 613.3)

### Synthesis Example 11 Synthesis of Compound 51 (Borono(C0)-Pip-6Qui-FAPI(H))

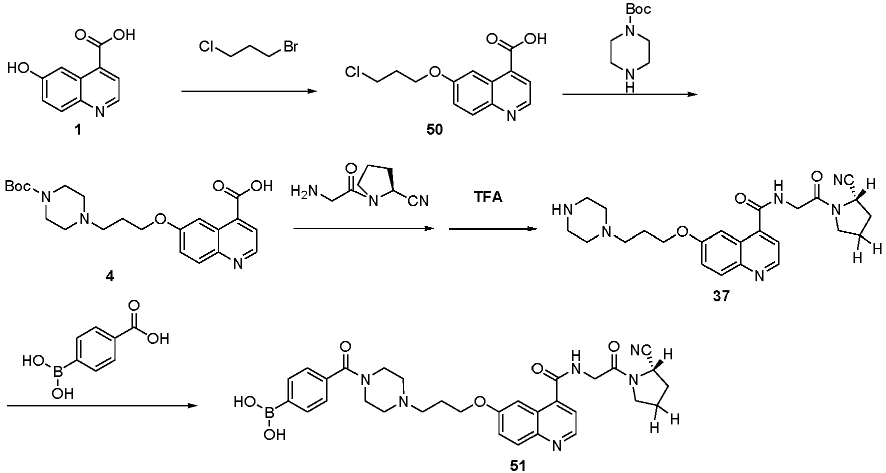

### (1) Synthesis of Compound 50

Compound 1 (4.3 g, 16 mmol) was dissolved in DMF (70 mL), potassium carbonate (27 g, 0.19 mol) was added thereto, and then 1-bromo-3-chloropropane (5.5 mL, 56 mmol) was added dropwise thereto. The reaction solution was stirred at 60°C for 2 hr, the insoluble substance was removed by filtration, and the solvent was evaporated under reduced pressure. The obtained residue was dissolved in 60% acetonitrile-containing water 100 mL, and 5 M aqueous sodium hydroxide solution (16 mL, 80 mmol) was added thereto. The mixture was stirred for 30 min, and neutralized with AcOH under ice-cooling, and the solvent was evaporated under reduced pressure. The precipitate was collected by filtration, washed with water, and dried to give Compound 50 (3.1 g, 72%).

### (2) Synthesis of Compound 4

Compound 50 (1.4 g, 5.1 mmol), 1-(tert-butoxycarbonyl)piperazine (5.0 g, 27 mmol) and sodium iodide (4.1 g, 27 mmol) were dissolved in DMF (50 mL), the solution was stirred at 55°C for 23 hr, and the solvent was evaporated under reduced pressure. The obtained residue was diluted with 30% AcOH-containing water, and purified by reverse-phase HPLC. The fraction containing the target product was lyophilized to give Compound 4 (2.1 g, 77%).

### (3) Synthesis of Compound 37

Compound 4 (0.47 g, 0.89 mmol), HOAt (0.12 g, 0.89 mmol) and DIEA (0.31 mL, 1.8 mmol) were dissolved in DMF (7 mL), and then HATU (0.32 g, 0.84 mmol) was added thereto, and the mixture was stirred for 10 min. To the reaction solution was added (S)-1-(2-aminoacetyl)-pyrrolidine-2-carbonitrile TFA salt, which was obtained by dissolving (S)-1-(2-(tert-butoxycarbonylamino)acetyl)-pyrrolidine-2-carbonitrile (0.24 g, 0.95 mmol) in TFA, stirring the solution for 30 min, and evaporating the solvent under reduced pressure. The mixture was stirred at room temperature for 1 hr, the solvent was evaporated under reduced pressure, and the obtained crude product was dissolved in TFA (30 mL). After stirring for 30 min, the solvent was evaporated under reduced pressure, diisopropyl ether was added thereto for solidification, and the solid was collected by filtration. The filtered solid was dissolved in 0.1% TFA/1% acetonitrile-containing water, and purified by reverse-phase HPLC. The fraction containing the target product was lyophilized to give Compound 37 (TFA salt, 0.36 g, 60%).

### (4) Synthesis of Compound 51

Compound 37 (0.31 g, 0.46 mmol), 4-carboxyphenylboronic acid (72 mg, 0.43 mmol) and PyAOP (0.24 g, 0.46 mmol) were dissolved in DMF (3 mL), and then DIEA (0.28 mL, 1.6 mmol) was added thereto. The mixture was stirred at room temperature for 2 hr, and the solvent was evaporated under reduced pressure. The obtained residue was diluted with 20% AcOH-containing water, and purified by reverse-phase HPLC. The fraction containing the target product was lyophilized to give Compound 51 (0.25 g, 76 %). HPLC purity: 99.9%, ESI-MS MH+: 598.4 (theoretical value: 598.5)

### Synthesis Example 12 Synthesis of Compound 53 (Qui-Gly-Pro-(B) Phe)

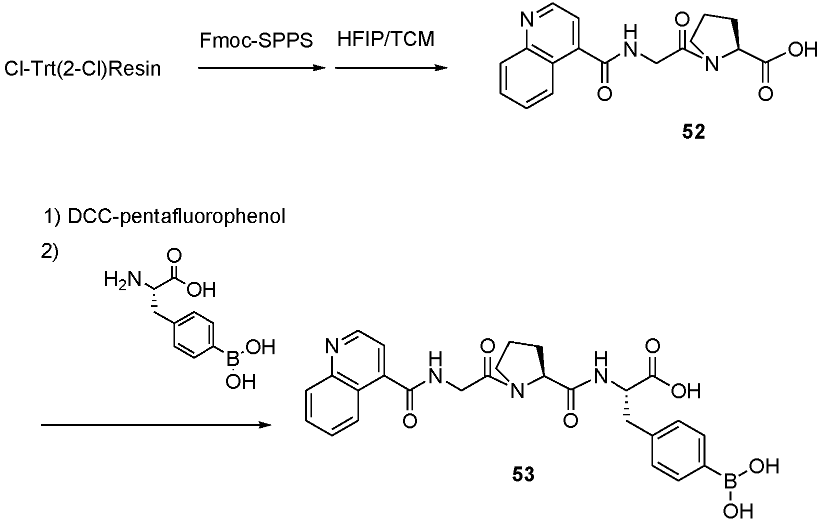

### (1) Synthesis of Compound 52

The following solid-phase peptide synthesis (SPPS) was conducted. To Cl-Trt(2-Cl)-Resin (1.2 g, 2.1 mmol) was added a chloroform solution (15 mL) of Fmoc-Pro·H₂O (0.53 g, 1.5 mmol) and DIEA (0.71 mL, 4.2 mmol), and the mixture was stirred at room temperature for 1.5 hr. The resin was washed with TCM/MeOH/DIEA (17/2/1) solution (20 mL), and TCM. The peptide chain was elongated sequentially with Fmoc-Gly and 4-quinolinecarboxylic acid by deprotection with 20% piperidine/NMP and condensation with PyAOP-DIEA. To the obtained protected peptide resin was added 20% HFIP/TCM solution, and the mixture was stirred at room temperature for 1 hr, and the resin was removed by filtration. The solvent was evaporated under reduced pressure, diethyl ether was added thereto for solidification, and the precipitate was collected by filtration, and dried to give Compound 52 (0.33 g, 47%).

### (2) Synthesis of Compound 53

Compound 52 (0.16 g, 0.51 mmol) and pentafluorophenol (0.11 g, 0.61 mmol) were dissolved in THF, and then DCC (87 µL, 0.53 mmol) was added dropwise thereto under ice-cooling, and the mixture was stirred at room temperature for 3 hr. AcOH was added thereto, the reaction solution was filtered, and the solvent was evaporated under reduced pressure. The obtained residue was washed with hexane to give the corresponding activated ester (0.27 g). The activated ester (0.24 g) was dissolved in DMF (4 mL), and a mixed suspension of 4-borono-L-phenylalanine (0.10 g, 0.48 mmol) and 40% tetrabutylammonium hydroxide aqueous solution (0.14 µL, 0.42 mmol) was added dropwise thereto under ice-cooling. The mixture was stirred at room temperature for 18 hr, and to the reaction solution were added water and TFA, and the mixture was purified by reverse-phase HPLC. The fraction containing the target product was lyophilized to give Compound 53 as a lyophilized powder (80 mg, 30%). HPLC purity: 99.5%, ESI-MS MH+: 519.2 (theoretical value: 519.2)

### Synthesis Example 13 Synthesis of Compound 57 (Ac-Pip-6Qui-Gly-Pro- (B) Phe)

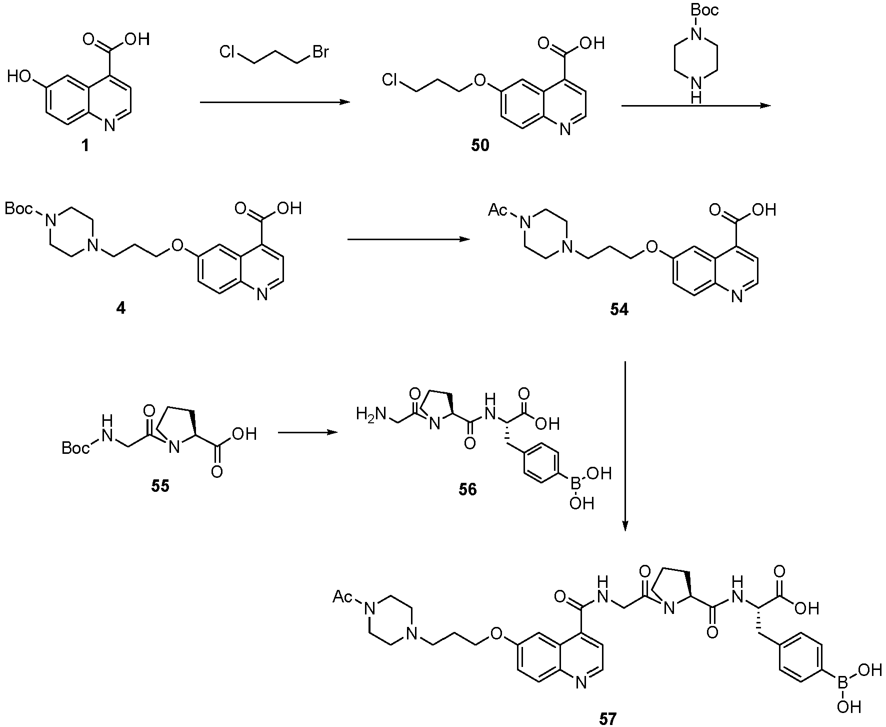

### (1) Synthesis of Compound 54

Compound 4 (0.5 g, 0.78 mmol) synthesized in the same manner as in Synthesis Example 11 was dissolved in TFA (30 mL), the solution was stirred for 1 hr, and the solvent was evaporated under reduced pressure. To the residue was added 4.5 N hydrochloric acid/dioxane (0.55 mL, 2.5 mmol), and then diethyl ether was added thereto for solidification, and the solid was collected by filtration, and dried. The obtained powder was dissolved in a mixed solvent of DMF (15 mL), pyridine (15 mL) and water (3 mL), and then acetic anhydride (0.18 mL, 1.9 mmol) was added thereto, and the mixture was stirred for 2 hr. The solvent was evaporated under reduced pressure, to the residue was added diethyl ether for solidification, and the solid was collected by filtration. The obtained powder was dissolved in 0.5% TFA/25% acetonitrile-containing water, and the solution was lyophilized. The lyophilized product was dissolved in 0.1% TFA-containing water, and purified by reverse-phase HPLC. The fraction containing the target product was lyophilized to give Compound 54 (0.45 g, 97%).

### (2) Synthesis of Compound 56

Compound 55 (0.14 g, 0.5 mmol) and pentafluorophenol (97 mg, 0.53 mmol) were dissolved in THF (10 mL), and then DIC (82 µL, 0.53 mmol) was added thereto, and the mixture was stirred for 2 hr. The reaction solution was neutralized with AcOH, and the solvent was evaporated under reduced pressure. The residue was dissolved in DMF (10 mL), 4-borono-L-phenylalanine (0.10 g, 0.5 mmol) and 40% tetrabutylammonium hydroxide aqueous solution (0.29 mL, 0.45 mmol) were added thereto, and the mixture was stirred for 1.5 hr. The solvent was evaporated under reduced pressure, and the residue was dissolved in TFA, and the solution was stirred for 1 hr. The reaction solution was concentrated, diethyl ether was added thereto for solidification, and the solid was collected by filtration. The obtained powder was dissolved in 0.1% TFA-containing water, and purified by reverse-phase HPLC. The fraction containing the target product was lyophilized to give Compound 56 (0.11 g, 46%).

### (3) Synthesis of Compound 57

Compound 54 (72 mg, 0.12 mmol) was dissolved in DMF (3 mL), and then PyAOP (76 mg, 0.15mmol) and DIEA (78 µL, 0.46 mmol) were added thereto, and the mixture was stirred for 10 min. To the solution was added Compound 56 (0.11 g, 0.23 mmol), which was dissolved in 2% hydrous DMF (3 mL), and the mixture was stirred for 1 hr. The solvent was evaporated under reduced pressure, to the residue was added diethyl ether for solidification, and the solid was collected by filtration. The obtained powder was dissolved in 1% TFA-containing water, and purified by reverse-phase HPLC. The fraction containing the target product was lyophilized to give Compound 57 (55 mg, 49%). HPLC purity: 99.3%, ESI-MS MH+: 703.3 (theoretical value: 703.3)

### Synthesis Example 14 Synthesis of Compound 61 (Borono(C1)-Pip-amido-6Qui-FAPI(H))

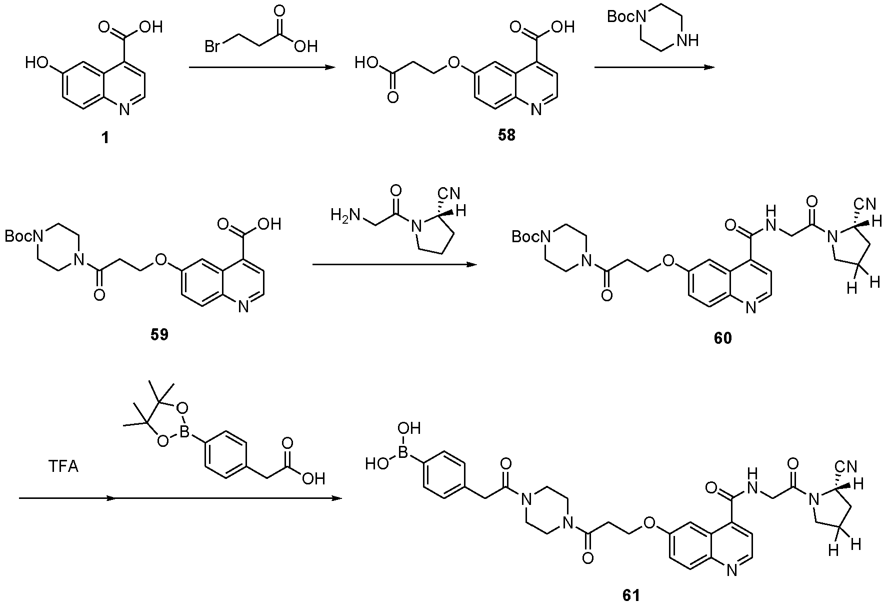

### (1) Synthesis of Compound 58

Commercially available Compound 1 (1.0 g, 5.3 mmol) and 3-bromopropionic acid (2.8 g, 19 mmol) were dissolved in DMF (10 mL), and then sodium hydride (60%, dispersed in liquid paraffin, 1.9 g, 48 mmol) was added thereto under ice-cooling. The mixture was stirred for 2 hr, and 3-bromopropionic acid (2.8 g, 19 mmol) and sodium hydride (60%, dispersed in liquid paraffin, 1.9 g, 48 mmol) were further added thereto. The mixture was stirred for 2 hr, and the reaction solution was neutralized AcOH, diluted with 50% AcOH-containing water (100 mL), and purified by reverse-phase HPLC. The fraction containing the target product was lyophilized to give Compound 58 (0.37 g, 26%).

### (2) Synthesis of Compound 59

1-(tert-Butoxycarbonyl)piperazine (0.43 g, 2.3 mmol), Compound 58 (0.60 g, 2.3 mmol) and HOBt (0.37 g, 2.8 mmol) were dissolved in DMF (5 mL), and then DIC (0.43 mL, 0.28 mmol) was added dropwise thereto under ice-cooling. The mixture was stirred at room temperature for 2 hr, AcOH was added thereto, and the solvent was evaporated under reduced pressure. The residue was dissolved in 50% AcOH-containing water, and purified by reverse-phase HPLC. The fraction containing the target product was neutralized with saturated aqueous sodium bicarbonate solution, and the acetonitrile was concentrated. To the obtained aqueous solution was added ethyl acetate, and then 0.1N hydrochloric acid was added thereto, and the mixture was subjected to extraction. The organic layer was washed with saturated brine, and dried over anhydrous sodium sulfate. The solvent was evaporated under reduced pressure to give Compound 59 (0.68 g, 69%).

### (3) Synthesis of Compound 60

(S)-1-(2-Aminoacetyl)-pyrrolidine-2-carbonitrile TFA salt (0.79 mmol), which was obtained by the same method as in the synthesis of Compound 38 in Synthesis Example 7, was dissolved in 90% DMF-containing water (8 mL), and the solution was neutralized with DIEA. Compound 59 (0.34 g, 0.79 mmol) and HOAt (0.12 g, 0.87 mmol) were added thereto, and then EDC (0.16 mL, 0.87 mmol) was added dropwise thereto under ice-cooling. The mixture was stirred at room temperature for 1.5 hr, and the solvent was evaporated under reduced pressure. The residue was dissolved in 50% AcOH-containing water (25 mL), and purified by reverse-phase HPLC. The fraction containing the target product was lyophilized to give Compound 60 (0.31 g, 70%).

### (4) Synthesis of Compound 61

Compound 60 (0.15 g, 0.27 mmol) was dissolved in 95% TFA/acetonitrile (4.5 mL), and the mixture was stirred for 60 min. The solvent was evaporated under reduced pressure, the obtained residue was dissolved in 90% DMF-containing water (2.7 mL), and the solution was neutralized with DIEA. 4-(Carboxymethyl)phenylboronic acid pinacol ester (70 mg, 0.27 mmol) and HOAt (40 mg, 0.29 mmol) were added thereto, and then EDC (54 µL, 0.29 mmol) was added dropwise thereto under ice-cooling, and the mixture was stirred at room temperature for 2 hr. The solvent of the reaction solution was evaporated under reduced pressure, and the residue was dissolved in 30% AcOH-containing water (5 mL), and the solution was stirred overnight. The reaction solution was purified by reverse-phase HPLC, and the fraction containing the target product was lyophilized. The obtained powder was dissolved in 10% acetonitrile-containing water, and purified again by reverse-phase HPLC, and the fraction containing the target product was lyophilized to give Compound 61 (79 mg, 48%). HPLC purity: 99.4%, ESI-MS [M+H]⁺: 627.3 (theoretical value: 627.3)

### Synthesis Example 15 Synthesis of Compound 62 (Borono(C2)-Pip-6Qui-FAPI(H))

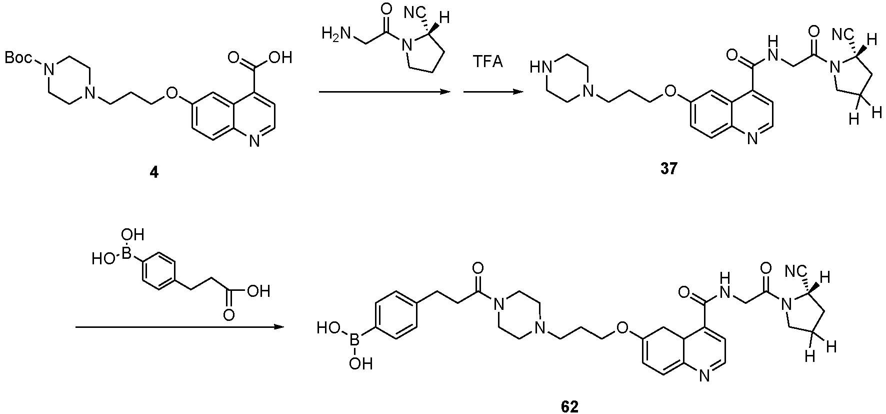

Compound 37 (TFA salt, 0.30 g, 0.45 mmol) synthesized from Compound 4 by the same method as in Reference Example 1 was dissolved in 90% DMF-containing water (10 mL), and the solution was neutralized with TEA. 4-(2-Carboxyethyl)phenylboronic acid (87 mg, 0.45 mmol) and HOAt (61 mg, 0.45 mmol) were added thereto, and then EDC (90 µL, 0.49 mmol) was added dropwise thereto under ice-cooling, and the mixture was stirred at room temperature for 3.5 hr. The solvent of the reaction solution was evaporated under reduced pressure, and the residue was dissolved in 30% AcOH-containing water (20 mL), and purified by reverse-phase HPLC. The fraction containing the target product was lyophilized. The obtained powder was dissolved in 0.1% TFA-containing water, and purified again by reverse-phase HPLC, and the fraction containing the target product was lyophilized to give the titled product ((81 mg, 29%)). HPLC purity: 99.4%, ESI-MS [M+H]⁺: 627.3 (theoretical value: 627.3)

The Borono-FAPI derivatives synthesized in Synthesis Examples was ²¹¹At-labeled according to the basic procedures shown below. The outline is shown in Fig. 1.

0.1 or 1% Borono-FAPI derivative (1 pg, 10 pg or 100 pg), 7% Meylon solution (10 µL), water (90 µL), ²¹¹At aqueous solution (0.5-10 MBq, 1-10 µL) and 0.1M potassium iodide (KI) solution (30 µL) were placed in a 2 mL volume of polyprene (PP) tube, and the mixture was heated at 50°C or 80°C for 45 min. This reaction solution was passed through a solid-phase extraction cartridge (Oasis HLB, Waters) and retained therein. This HLB cartridge was washed with water (1.0 mL) (Fraction E1). Then, 40% ethanol solution (0.5 mL) and 100% ethanol (0.5 mL) were passed sequentially through this HLB cartridge to elute the target product (fractions E2 and E3). 1-2 µL of the reaction solution and Fraction E2 (or E3) were sampled, and the radiochemical yield (RCY, %) and radiochemical purity (RCP, %) were determined by thin-layer chromatograph method (TLC). The radiochemical yield was calculated according to the following formula. Radiochemical yield (%) = (radioactivity of the target compound on thin-layer plate/total radioactivity on thin-layer plate) × 100

The specific ²¹¹At-labeling method, chemical structure of the product, and quality analysis results (TLC) for each Borono-FAPI derivative are shown below.

### Example 1 Synthesis of ²¹¹At(C1)-Pip-6Qui-FAPI(F)/PIP(1,F)

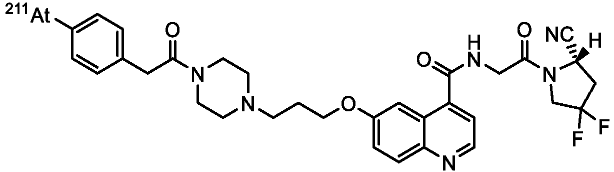

A 0.1% aqueous solution (10 µL, mass 10 pg) of Compound 7 (Borono(C1)-Pip-6Qui-FAPI(F)) synthesized in Synthesis Example 1, 7% Meylon (10 µL), water (90 µL), ²¹¹At aqueous solution (2 µL) and 0.1M potassium iodide solution (30 µL) were placed in a polypropylene (PP) tube. This solution was heated at 80°C for 45 min, passed through Oasis HLB and retained therein. Water (1 mL) (Fraction E1) was passed through the HLB cartridge for washing, followed by 40% ethanol (0.5 mL) (Fraction E2) and 100% ethanol (0.5 mL) (Fraction E3) for elution. The target compound was eluted in Fraction E3. The RCY was 91.0%, and the RCP was 99.9%. The TLC result of the reaction solution is shown in Fig. 2.

### Example 2 Synthesis of ²¹¹At(C1)-Pip-6Qui-FAPI(H)/PIP(1,H)

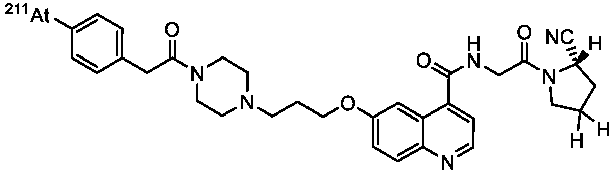

A 0.1% aqueous solution (1 µL or 10 µL, mass 1 pg or 10 pg) of Compound 44 (Borono(C1)-Pip-6Qui-FAPI(H)) synthesized in Synthesis Example 9, 7% Meylon (10 µL), water (90 µL), ²¹¹At aqueous solution (2 µL) and 0.1M potassium iodide solution (30 µL) were placed in a polypropylene (PP) tube. This solution was heated at 80°C for 45 min, passed through Oasis HLB and retained therein. Water (1 mL) (Fraction E1) was passed through the HLB cartridge for washing, followed by 40% ethanol (0.5 mL) (Fraction E2) and 100% ethanol (0.5 mL) (Fraction E3) for elution. The target compound was eluted in Fraction E3. The RCY was 14.1% when the mass of the raw material was 1 pg, and the RCY was 98.0% when the mass of the raw material was 10 pg, and the RCP was 99.9%. The TLC result of the reaction solution is shown in Fig. 3.

### Example 3 Synthesis of ²¹¹At(C1)-Pip-8Qui-FAPI(H)/PIP(1,H)-8Qui

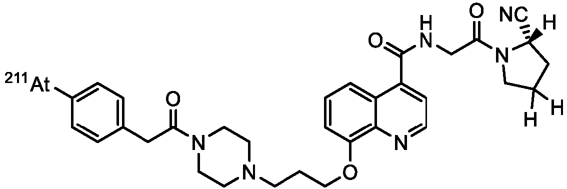

A 0.1% aqueous solution (10 µL, mass 10 pg) of Compound 49 (Borono(C1)-Pip-8Qui-FAPI(H)) synthesized in Synthesis Example 10, 7% Meylon (10 µL), water (90 µL), ²¹¹At aqueous solution (4 µL) and 0.1M potassium iodide solution (30 µL) were placed in a polypropylene (PP) tube. This solution was heated at 80°C for 45 min, passed through Oasis HLB and retained therein. Water (1 mL) (Fraction E1) was passed through the HLB cartridge for washing, followed by 40% ethanol (0.5 mL) (Fraction E2) and 100% ethanol (0.5 mL) (Fraction E3) for elution. The target compound was eluted in Fraction E3. The RCY was 91.8%, and the RCP was 99.8%. The TLC result of the reaction solution is shown in Fig. 4.

### Example 4 Synthesis of ²¹¹At(C0)-Pip-6Qui-FAPI(H)/PIP(0,H)

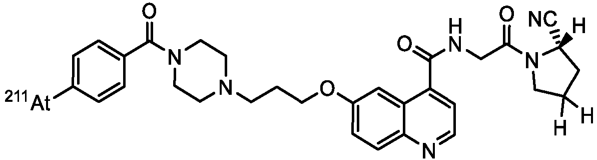

A 0.1% aqueous solution (1 µL) or 1.0% aqueous solution (10 µL) (mass 1 pg or 100 pg) of Compound 51 (Borono(C0)-Pip-6Qui-FAPI(H)) synthesized in Synthesis Example 11, 7% Meylon (10 µL), water (90 µL), ²¹¹At aqueous solution (2 µL) and 0.1M potassium iodide solution (30 µL) were placed in a polypropylene (PP) tube. This solution was heated at 80°C for 45 min, passed through Oasis HLB and retained therein. Water (1 mL) (Fraction E1) was passed through the HLB cartridge for washing, followed by 40% ethanol (0.5 mL) (Fraction E2) and 100% ethanol (0.5 mL) (Fraction E3) for elution. The target compound was eluted in Fraction E3. The RCY was 1.6% when the mass of the raw material was 1 pg, and the RCY was 77.8% when the mass of the raw material was 100 pg, and the RCP was 91.7%. The TLC result of the reaction solution is shown in Fig. 5.

### Example 5 Synthesis of ²¹¹At(C1)-PEG2-6Qui-FAPI(F)/PEG(1,F)

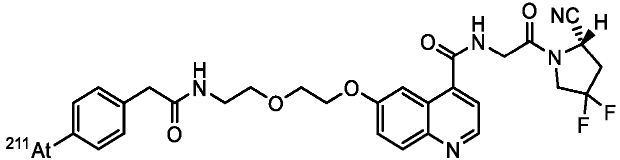

A 0.1% aqueous solution (10 µL, mass 10 pg) of Compound 13 (Borono(C1)-PEG2-6Qui-FAPI(F)) synthesized in Synthesis Example 2, 7% Meylon (10 µL), water (90 µL), ²¹¹At aqueous solution (2 µL) and 0.1M potassium iodide solution (30 µL) were placed in a polypropylene (PP) tube. This solution was heated at 80°C for 45 min, passed through Oasis HLB and retained therein. Water (1 mL) (Fraction E1) was passed through the HLB cartridge for washing, followed by 40% ethanol (0.5 mL) (Fraction E2) and 100% ethanol (0.5 mL) (Fraction E3) for elution. The target compound was eluted in Fraction E3. The RCY was 98.0%, and the RCP was 99.9%. The TLC result of the reaction solution is shown in Fig. 6.

### Example 6 Synthesis of ²¹¹At(C1)-PEG2-6Qui-FAPI(H)/PEG(1,H)

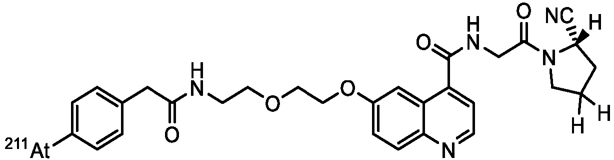

A 0.1% aqueous solution (1 µL or 10 µL, mass 1 pg or 10 pg) of Compound 39 (Borono(C1)-PEG2-6Qui-FAPI(H)) synthesized in Synthesis Example 7, 7% Meylon (10 µL), water (90 µL), ²¹¹At aqueous solution (2 µL) and 0.1M potassium iodide solution (30 µL) were placed in a polypropylene (PP) tube. This solution was heated at 80°C for 45 min, passed through Oasis HLB and retained therein. Water (1 mL) (Fraction E1) was passed through the HLB cartridge for washing, followed by 40% ethanol (0.5 mL) (Fraction E2) and 100% ethanol (0.5 mL) (Fraction E3) for elution. The target compound was eluted in Fraction E3. The RCY was 58.7% when the mass of the raw material was 1 µg, and the RCY was 98.8% when the mass of the raw material was 10 µg, and the RCP was 99.5%. The TLC result of the reaction solution is shown in Fig. 7.

### Example 7 Synthesis of ²¹¹At(C1)-PEG4-6Qui-FAPI(H)/ PEG4(1,H)

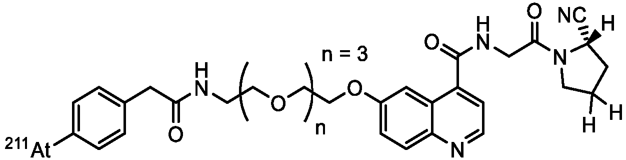

A 0.1% aqueous solution (10 µL, mass 10 pg) of Compound 42 (Borono(C1)-PEG4-6Qui-FAPI(H)) synthesized in Synthesis Example 8, 7% Meylon (10 µL), water (90 µL), ²¹¹At aqueous solution (2 µL) and 0.1M potassium iodide solution (30 µL) were placed in a polypropylene (PP) tube. This solution was heated at 80°C for 45 min, passed through Oasis HLB and retained therein. Water (1 mL) (Fraction E1) was passed through the HLB cartridge for washing, followed by 40% ethanol (0.5 mL) (Fraction E2) and 100% ethanol (0.5 mL) (Fraction E3) for elution. The target compound was eluted in Fraction E3. The RCY was 98.7%, and the RCP was 99.9%. The TLC result of the reaction solution is shown in Fig. 8.

### Example 8 Synthesis of ²¹¹At(C1)-Gly(1)-8Qui-FAPI(F)/Gly(1,F)

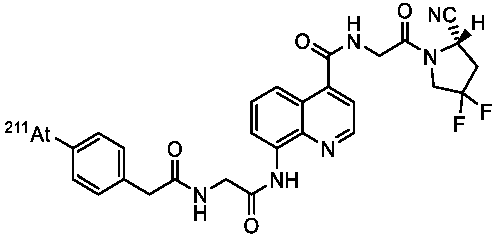

A 0.1% aqueous solution (10 µL, mass 10 pg) of Compound 19 (Borono(C1)-Gly(1)-8Qui-FAPI(F)) synthesized in Synthesis Example 3, 7% Meylon (10 µL), water (90 µL), ²¹¹At aqueous solution (2 µL) and 0.1M potassium iodide solution (30 µL) were placed in a polypropylene (PP) tube. This solution was heated at 50°C for 45 min, passed through Oasis HLB and retained therein. Water (1 mL) (Fraction E1) was passed through the HLB cartridge for washing, followed by 40% ethanol (0.5 mL) (Fraction E2) and 100% ethanol (0.5 mL) (Fraction E3) for elution. The target compound was eluted in Fraction E3. The RCY was 95.7%, and the RCP was 97.4%. The TLC result of the reaction solution is shown in Fig. 9.

### Example 9 Synthesis of ²¹¹At(C0)-GlcN-Pip-6Qui-FAPI(F)/GlcN-PIP(0,F)

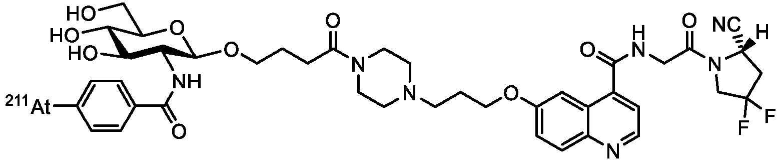

A 0.1% aqueous solution (10 µL, mass 10 pg) of Compound 29 (Borono(C0)-GlcN-Pip-6Qui-FAPI(F)) synthesized in Synthesis Example 4, 7% Meylon (10 µL), water (90 µL), ²¹¹At aqueous solution (2 µL) and 0.1M potassium iodide solution (30 µL) were placed in a polypropylene (PP) tube. This solution was heated at 80°C for 45 min, passed through Oasis HLB and retained therein. Water (1 mL) (Fraction E1) was passed through the HLB cartridge for washing, followed by 40% ethanol (0.5 mL) (Fraction E2) and 100% ethanol (0.5 mL) (Fraction E3) for elution. The target compound was eluted in Fraction E3. The RCY was 70.3%, and the RCP was 90.9%. The TLC result of the reaction solution is shown in Fig. 10.

### Example 10 Synthesis of ²¹¹At(C0)-GlcN-Pip-6Qui-FAPI(H)/GlcN-PIP(0,H)

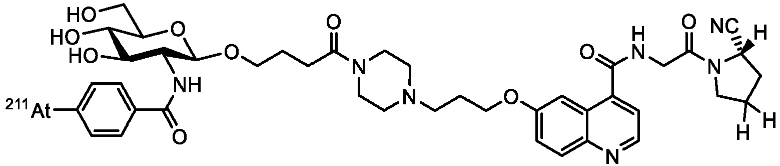

A 0.1% or 1.0% aqueous solution (10 µL, mass 10 pg or 100 pg) of Compound 32 (Borono(C0)-GlcN-Pip-6Qui-FAPI(H)) synthesized in Synthesis Example 5, 7% Meylon (10 µL), water (90 µL), ²¹¹At aqueous solution (2 µL) and 0.1M potassium iodide solution (30 µL) were placed in a polypropylene (PP) tube. This solution was heated at 80°C for 45 min, passed through Oasis HLB and retained therein. Water (1 mL) (Fraction E1) was passed through the HLB cartridge for washing, followed by 40% ethanol (0.5 mL) (Fraction E2) and 100% ethanol (0.5 mL) (Fraction E3) for elution. The target compound was eluted in Fraction E3. The RCY was 55.9% when 10 µg of the raw material was used, and the RCY was 62.0% when 100 µg of the raw material was used, and the RCP was 70.4%. The TLC result of the reaction solution is shown in Fig. 11.

### Example 11 Synthesis of ²¹¹At(C1)-GlcN-PEG-6Qui-FAPI(F)/GlcN-PEG(1,F)

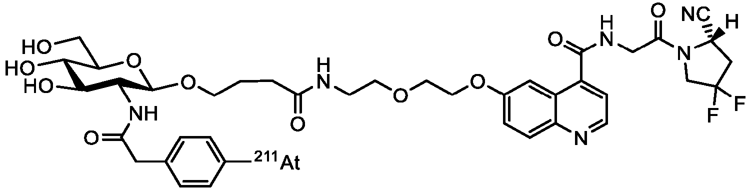

A 0.1% aqueous solution (10 µL, mass 10 pg) of Compound 35 (Borono(C1)-GlcN-PEG-6Qui-FAPI(F)) synthesized in Synthesis Example 6, 7% Meylon (10 µL), water (90 µL), ²¹¹At aqueous solution (2 µL) and 0.1M potassium iodide solution (30 µL) were placed in a polypropylene (PP) tube. This solution was heated at 80°C for 45 min, passed through Oasis HLB and retained therein. Water (1 mL) (Fraction E1) was passed through the HLB cartridge for washing, followed by 40% ethanol (0.5 mL) (Fraction E2) and 100% ethanol (0.5 mL) (Fraction E3) for elution. The target compound was eluted in Fraction E3. The RCY was 97.2%, and the RCP was 99.9%. The TLC result of the reaction solution is shown in Fig. 12.

### Example 12 Synthesis of Qui-Gly-Pro-(²¹¹At)Phe/Qui-Phe

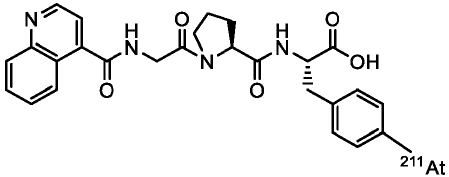

A 0.1% aqueous solution (1 µL or 10 µL, mass 1 pg or 10 pg) of Compound 53 (Qui-Gly-Pro(B)Phe) synthesized in Synthesis Example 12, 7% Meylon (10 µL), water (90 µL), ²¹¹At aqueous solution (2 µL) and 0.1M potassium iodide solution (30 µL) were placed in a polypropylene (PP) tube. This solution was heated at 80°C for 45 min, passed through Oasis HLB and retained therein. Water (1 mL) (Fraction E1) was passed through the HLB cartridge for washing, followed by 40% ethanol (0.5 mL) (Fraction E2) and 100% ethanol (0.5 mL) (Fraction E3) for elution. The target compound was eluted in Fraction E2. The RCY was 96.3% when 1 pg of the raw material was used, and the RCY was 98.6% when 10 pg of the raw material was used. The TLC result of the reaction solution is shown in Fig. 13.

### Example 13 Synthesis of Ac-Pip-6Qui-Gly-Pro-(²¹¹At)Phe/Ac-PIP

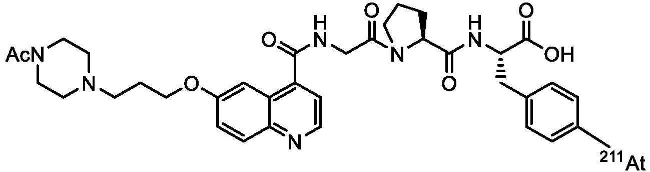

A 0.1% aqueous solution (10 µL, mass 10 pg) of Compound 57 (Ac-Pip-6Qui-Gly-Pro-(B)Phe) synthesized in Synthesis Example 13, 7% Meylon (10 µL), water (90 µL), ²¹¹At aqueous solution (2 µL) and 0.1M potassium iodide solution (30 µL) were placed in a polypropylene (PP) tube. This solution was heated at 80°C for 45 min, passed through Oasis HLB and retained therein. Water (1 mL) (Fraction E1) was passed through the HLB cartridge for washing, followed by 40% ethanol (0.5 mL) (Fraction E2) and 100% ethanol (0.5 mL) (Fraction E3) for elution. The target compound was eluted in Fraction E2. The RCY was 95.7%, and the RCP was 99.2%. The TLC result of the reaction solution is shown in Fig. 14.

### Example 14 Synthesis of ²¹¹At(C1)-Pip-amido-6Qui-FAPI(H)/PIP-amide (1,H)

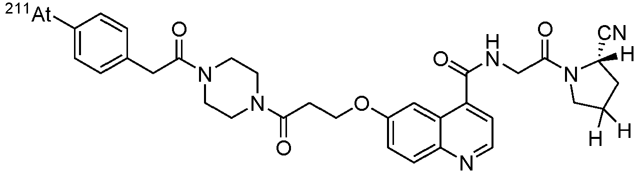

A 0.1% aqueous solution (10 µL, mass 10 pg) of Compound 61 (Borono(C1)-Pip-amido-6Qui-FAPI(H)) synthesized in Synthesis Example 14, 7% Meylon (10 µL), water (90 µL), ²¹¹At aqueous solution (2 µL) and 0.1M potassium iodide solution (30 µL) were placed in a polypropylene (PP) tube. This solution was heated at 80°C for 45 min, passed through Oasis HLB and retained therein. Water (1 mL) (Fraction E1) was passed through the HLB cartridge for washing, followed by 40% ethanol (0.5 mL) (Fraction E2) and 100% ethanol (0.5 mL) (Fraction E3) for elution. The target compound was eluted in Fraction E3. The RCY was 97.1%, and the RCP was 97.7%. The TLC result of the reaction solution is shown in Fig. 15.

### Example 15 Synthesis of ²¹¹At(C2)-Pip-6Qui-FAPI(H)/PIP(2,H)

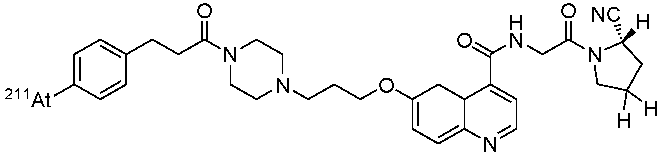

A 0.1% aqueous solution (10 µL, mass 10 pg) of Compound 62 (Borono(C2)-Pip-6Qui-FAPI(H)) synthesized in Synthesis Example 15, 7% Meylon (10 µL), water (90 µL), ²¹¹At aqueous solution (2 µL) and 0.1M potassium iodide solution (30 µL) were placed in a polypropylene (PP) tube. This solution was heated at 80°C for 45 min, passed through Oasis HLB and retained therein. Water (1 mL) (Fraction E1) was passed through the HLB cartridge for washing, followed by 40% ethanol (0.5 mL) (Fraction E2) and 100% ethanol (0.5 mL) (Fraction E3) for elution. The target compound was eluted in Fraction E3. The RCY was 98.1%, and the RCP was 98.2%. The TLC result of the reaction solution is shown in Fig. 16.

The RCY% and RCP% of the radiolabeled products obtained in Examples are summarized in Table 1. For Examples 1 to 11, 14 and 15, C0, C1 and C2 in parentheses indicate the alkylene chain length between the phenyl group having a borono group and the carbonyl group.

When the alkylene chain length was 1 or 2 (Examples 1-3, 5-8, 11, 14 and 15), a high reaction yield of RCY>90% was achieved with 10 pg of the raw material.

On the other hand, when the alkylene chain length was 0 (Examples 4, 9 and 10), the reaction yield was low, and the reaction yield did not reach 90% even with 100 pg of the raw material (Examples 4 and 10). In these cases, it was found that the purity after purification was low (RCP<92%) and the stability of the product was also low.

From the above, it was shown that in the synthesis of Radiolabeled Compound (I-1) from Boronic Acid Compound (II-1), the alkylene chain length affects the reaction yield, and that in order to achieve a reaction yield of 90% or more, the number of carbon atoms is preferably 1 or more.

### [Table 1]

**Table 1**

| List of names/abbreviations, radiochemical yields and radiochemical purities for each compound | | | | | | |
|---|---|---|---|---|---|---|
| | ²¹¹At-labeled compound name | abbreviation | RCY% | | | RCP% |
| | | | Use of raw material | | | |
| | | | 1 µg | 10 µg | 100 µg | |
| Example 1 | ²¹¹At (C1) -Pip-6Qui-FAPI (F) | PIP (1, F) | --- | 91.0 | --- | 99.9 |
| Example 2 | ²¹¹At (C1) -Pip-6Qui-FAPI (H) | PIP (1, H) | 14.1 | 98.0 | --- | 99.9 |
| Example 3 | ²¹¹At (C1) -Pip-8Qui-FAPI (H) | PIP (1, H)-8Qui | --- | 91.8 | --- | 99.8 |
| Example 4 | ²¹¹At (C0) -Pip-6Qui-FAPI (H) | PIP (0, H) | 1.6 | --- | 77.8 | 91.7 |
| Example 5 | ²¹¹At (C1) -PEG2-6Qui-FAPI (F) | PEG (1, F) | --- | 98.0 | --- | 99.9 |
| Example 6 | ²¹¹At (C1) -PEG2-6Qui-FAPI (H) | PEG (1, H) | 58.7 | 98.8 | --- | 99.5 |
| Example 7 | ²¹¹At (C1) -PEG4-6Qui-FAPI (H) | PEG4 (1, H) | --- | 98.7 | --- | 99.9 |
| Example 8 | ²¹¹At (C1) -Gly(1)-8Qui-FAPI (F) | Gly (1, F) | --- | 95.7 | --- | 97.4 |
| Example 9 | ²¹¹At (C0) -GlcN-Pip-6Qui-FAPI (F) | GlcN-PIP (0, F) | --- | 70.3 | --- | 90.9 |
| Example 10 | ²¹¹At (C0) -GlcN-Pip-6Qui-FAPI (H) | GlcN-PIP (0, H) | --- | 55.9 | 62.0 | 70.4 |
| Example 11 | ²¹¹At (C1) -GlcN-PEG-6Qui-FAPI (F) | GlcN-PEG (1, F) | --- | 97.2 | --- | 99.9 |
| Example 12 | Qui-Gly-Pro-²¹¹AtPhe | Qui-Phe | 96.3 | 98.6 | --- | --- |
| Example 13 | Ac-Pip-6Qui-Gly-Pro-²¹¹AtPhe | Ac-PIP | --- | 95.7 | --- | 99.2 |
| Example 14 | ²¹¹At (C1) -Pip-amido-6Qui-FAPI (H) | PIP-amide (1, H) | | 97.1 | | 97.7 |
| Example 15 | ²¹¹At (C2) -Pip-6Qui-FAPI (H) | PIP (2, H) | | 98.1 | | 98.2 |

### Example 16 Synthesis of ¹³¹I(C1)-Pip-6Qui-FAPI(H)

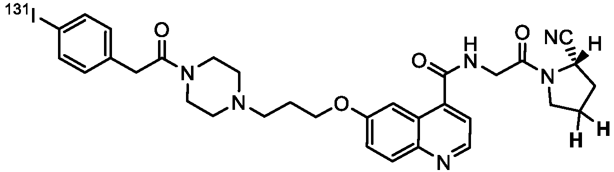

In this Example, ¹³¹I-labeling was conducted according to the basic procedures for ²¹¹At-labeling.

Compound 44 (Borono(C1)-Pip-6Qui-FAPI(H)) synthesized in Synthesis Example 9 (1 mg), water (80 µL), ¹³¹I-NaI aqueous solution (30 µL) and 0.4% N-bromosuccinimide (30 µL) were placed in a PP tube. The solution was heated at 80°C for 30 min, 4% ascorbic acid (10 µL) was added to the reaction solution, and the mixture was allowed to stand at room temperature for 15 min. This reaction solution was passed through Oasis HLB cartridge and retained therein. Water (1 mL) (Fraction E1) was passed through the HLB cartridge for washing, followed by 40% ethanol (0.5 mL) (Fraction E2) and 100% ethanol (0.5 mL) (Fraction E3) for elution. The target compound was eluted in Fraction E3. The RCY was 50.0%, and the RCP was 94.6%. The TLC results of the reaction solution and eluate are shown in Fig. 17. Borono(C1)-Pip-6Qui-FAPI(H) was also found to be useful for the synthesis of iodine-labeled products.

### Experimental Example 1 Uptake of ²¹¹At-labeled FAPI derivatives into FAPα/293 cells

Human embryonic kidney 293 cells (HEK293), and 293 cells transfected with the human fibroblast activation protein (FAPα) gene (FAPα/293) were used in the experiment as FAPα-negative and FAPα-positive cells, respectively. For each type, the cells were seeded in a 24-well plate at 2×10⁴ cells per well and cultured for 2 days. An appropriate amount of eight ²¹¹At-labeled FAPI derivatives (Gly(1,F) (Example 8), PEG(1,F) (Example 5), PIP(1,F) (Example 1), PIP(1,H)-8Qui (Example 3), Ac-PIP (Example 13), PEG4(1,H) (Example 7), GlcN-PEG(1,F) (Example 11), PEG(1,H) (Example 6)) were added to each type of the above cells, respectively, and cultured for 30 min. The supernatant was removed by aspiration, and then 0.1N sodium hydroxide solution was added to lyse the cells, and the intracellular radioactivity was measured by a gamma counter. The radioactivity count was corrected per unit protein amount (counts/pg protein). The results are shown in Figure 18. The three compounds with the highest uptake levels into FAPα-positive cells were PIP(1,F)>PIP(1,H)-8Qui>PEG(1,H), in order of increasing level. Furthermore, all of these compounds were found to be taken up in greater amounts into FAPα-positive cells than into FAPα-negative cells, thus indicating that they were specifically taken up into FAPα.

### Experimental Example 2 Uptake of ²¹¹At-labeled FAPI derivatives into FAPα/A549 or FAPα/MDA-MB-231 cells

Human non-small cell lung cancer cell lines (A549), human triple-negative (estrogen and progesterone receptors which are hormone receptors, as well as HER2 protein, are all absent) breast cancer cell lines (MDA-MB-231), and A549 cells and MDA-MB-231 cells, each transfected with the human fibroblast activation protein (FAPα) gene (FAPα/A549, FAPα/MDA-MB-231) were used in the experiments as cancer cells with low FAPα expression and cancer cells with high FAPα expression (mimic after high-order histogenesis), respectively. For each type, the cells were seeded in a 24-well plate at 2×10⁴ cells per well and cultured for 2 days. An appropriate amount of two ²¹¹At-labeled FAPI derivatives (PIP(1,H) (Example 1), PIP-amide(1,H) (Example 14)) were added to each type of the above cells, respectively, and cultured for 30 min. The supernatant was removed by aspiration, and the cells were washed with PBS(-), and then 0.1N sodium hydroxide solution was added to lyse the cells, and the intracellular radioactivity was measured by a gamma counter. The radioactivity count was corrected per unit protein amount (counts/pg protein). It was confirmed that the uptake level increased as FAPα expression increased, and the uptake level of PIP(1,H) was slightly higher than that of PIP-amide(1,H). Furthermore, the degree of competitive inhibition was also confirmed by the addition of the corresponding unlabeled form, and it was found that FAPα expression could be suppressed to a low level. Considering that the uptake level into human embryonic kidney-derived cells (HEK293) was about 20 counts/µg protein at most, based on the results of PIP(1,F) and the like in Experimental Example 1, it can be said that cancer cells take up more FAPI derivatives than non-cancer cells. The above results are shown in Fig. 19(a) and (b).

### Experimental Example 3 Inhibitory effect on tumor growth in human pancreatic cancer-transplanted mice

Human pancreatic cancer cells (PANC-1) were subcutaneously administered at 1×10⁷ cells to nude mice (male, 6-weeks old) to generate pancreatic cancer-transplanted mice. The mice were intravenously injected with ²¹¹At(C1)-Pip-6Qui-FAPI(H) (PIP(1,H), Example 2) or ²¹¹At (C1)-PEG2-6Qui-FAPI(H) (PEG(1,H), Example 6) at 1 MBq per mouse (N=4). Physiological saline was administered to the control group (Control (Saline)) (N=4). The tumor size and body weight of the mice were measured over a period of three weeks, and the results are shown in Fig. 20. The PIP(1,H) and PEG(1,H)-treated groups showed superior inhibitory effects on tumor growth compared to the control group. The changes in body weight of both groups were comparable to that of the control group, suggesting that these drugs have low toxicity. [Industrial Applicability]

According to the present invention, it is possible to provide radiolabeled compounds that bind specifically to FAPα, are effective in the treatment and diagnosis of tumors or cancers expressing FAPα, for example, in the treatment and diagnosis of solid cancers such as pancreatic cancer, sarcoma, esophageal cancer, lung cancer, breast cancer, prostate cancer, head and neck cancer, ovarian cancer, colon cancer, neuroendocrine tumor, thyroid cancer, uterine cancer, liver cancer and the like (particularly, pancreatic cancer), and have a lower risk of prolonged side effects.

This application is based on patent application No. 2022-026194 filed on February 22, 2022 in Japan, the contents of which are encompassed in full herein.

## Claims

1. A conjugate comprising
a radioactive moiety comprising an aryl group substituted with a radionuclide selected from ²¹¹At, ²¹⁰At, ¹³¹I, ¹²⁵I, ¹²⁴I, ¹²³I, ⁷⁷Br and ⁷⁶Br, and
a bioactive moiety having an affinity for fibroblast activation protein α (FAPα).

2. The conjugate according to Claim 1, wherein the bioactive moiety having an affinity for fibroblast activation protein α comprises a structure represented by the following formula:

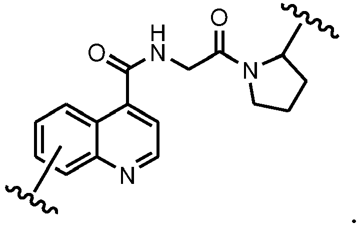

3. The conjugate according to Claim 1 or 2, wherein the radioactive moiety comprises an aryl-C₁₋₃ alkyl group, wherein the aryl is substituted with a radionuclide selected from ²¹¹At, ²¹⁰At, ¹³¹I, ¹²⁵I, ¹²⁴I, ¹²³I, ⁷⁷Br and ⁷⁶Br.

4. A compound radiolabeled represented by Formula (I-1) or a pharmaceutically acceptable salt thereof: 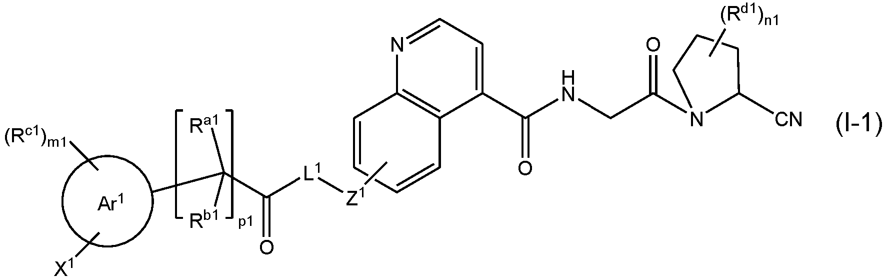 wherein
X¹ is a radionuclide selected from ²¹¹At, ²¹⁰At, ¹³¹I, ¹²⁵I, ¹²⁴I, ¹²³I, ⁷⁷Br and ⁷⁶Br;
Ar¹ is a C₆₋₁₄ aryl group;
R^{a1} and R^{b1} in the number of p1 are each independently a hydrogen atom or a C₁₋₆ alkyl group;
R^{c1} in the number of m1 are each independently a C₁₋₆ alkyl group or
a hydroxy group;
R^{d1} in the number of n1 are each independently a halogen atom;
Z¹ is an oxygen atom, a sulfur atom or NR^{f1} wherein R^{f1} is a hydrogen atom or a C₁₋₃ alkyl group;
L¹ is
(1) a linker represented by *-L^{d1}-L^{c1}-L^{b1}-L^{a1}-**
wherein
* indicates a binding site to CO,
** indicates a binding site to Z¹,
L^{a1} is
(i) a C₁₋₆ alkylene group, or
(ii) -CH₂-(CH₂-O-CH₂)_{q1}-CH₂- wherein q1 is an integer of 0 to 5, L^{b1} is a bond or -CO-,
L^{c1} is
(i) NR^{g1} wherein R^{g1} is a hydrogen atom or a C₁₋₃ alkyl group,
(ii) a divalent cyclic amino group,
(iii) an oxygen atom, or
(iv) a sulfur atom, and
L^{d1} is
(i) a bond,
(ii) *- (NH-A^{a1}-CO)ᵣ₁-***wherein NH-A^{a1}-CO in the number of r1 are each independently an amino acid residue, r1 is an integer of 1 to 3, * indicates a binding site to CO, and *** indicates a binding site to L^{c1}, or
(iii) *-NH-B^{a1}-O-B^{b1}-CO-*** wherein -NH-B^{a1}-O- is a divalent residue derived from an aminosaccharide or a derivative thereof, B^{b1} is a C₁₋₆ alkylene group, * indicates a binding site to CO, and *** indicates a binding site to L^{c1}, or
(2) a linker represented by *- (NH-A^{b1}-CO)ₛ₁-**
wherein
* indicates a binding site to CO,
** indicates a binding site to Z¹,
NH-A^{b1}-CO in the number of s1 are each independently an amino acid residue, and
s1 is an integer of 1 to 3;
p1 is an integer of 1 to 3;
m1 is an integer of 0 to 3; and
n1 is an integer of 0 to 3.

5. The compound or pharmaceutically acceptable salt thereof according to Claim 4, wherein
Z¹ is an oxygen atom or a sulfur atom, and
L¹ is a linker represented by *-L^{dl}1L^{c1}-L^{b1}-L^{a1}-** wherein each symbol is as defined above.

6. The compound or pharmaceutically acceptable salt thereof according to Claim 4, wherein
Z¹ is NR^{f1} wherein the symbol is as defined above, and
L¹ is a linker represented by *- (NH-A^{b1}-CO)ₛ₁-** wherein the symbols are as defined above.

7. The compound or pharmaceutically acceptable salt thereof according to Claim 5, wherein L¹ is a linker represented by *-L^{d1}-L^{c1}-L^{b1}- L^{a1}-**
wherein
* indicates a binding site to CO,
** indicates a binding site to Z¹,
L^{a1} is a C₁₋₆ alkylene group,
L^{b1} is a bond or -CO-,
L^{c1} is a divalent cyclic amino group, and
L^{d1} is
(i) a bond,
(ii) *- (NH-A^{a1}-CO)ᵣ₁-*** wherein each symbol is as defined above, or
(iii) *-NH-B^{a1}-O-B^{b1}-CO-*** wherein each symbol is as defined above.

8. The compound or pharmaceutically acceptable salt thereof according to any one of Claims 4, 5 and 7, wherein the divalent cyclic amino group in L^{c1} is a divalent 3- to 8-membered cyclic diamino group.

9. The compound or pharmaceutically acceptable salt thereof according to Claim 5, wherein L¹ is a linker represented by *-L^{d1}-L^{c1}-L^{b1}- L^{a1}-**
wherein
* indicates a binding site to CO,
** indicates a binding site to Z¹,
L^{a1} is -CH₂-(CH₂-O-CH₂)_{q1}-CH₂- wherein the symbol is as defined above,
L^{b1} is a bond,
L^{c1} is NR^{g1} wherein the symbol is as defined above, an oxygen atom or a sulfur atom, and
L^{d1} is a bond.

10. The compound or pharmaceutically acceptable salt thereof according to Claim 4 or 6, wherein s1 is 1.

11. A compound radiolabeled represented by Formula (I-2) or a pharmaceutically acceptable salt thereof: 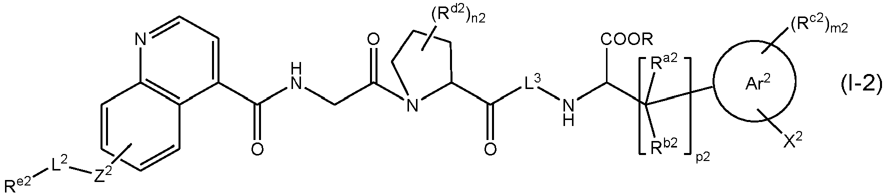 wherein
X² is a radionuclide selected from ²¹¹At, ²¹⁰At, ¹³¹I, ¹²⁵I, ¹²⁴I, ¹²³I, ⁷⁷Br and ⁷⁶Br;
Ar² is a C₆₋₁₄ aryl group;
R^{a2} and R^{b2} in the number of p2 are each independently a hydrogen atom or a C₁₋₆ alkyl group;
R^{c2} in the number of m2 are each independently a C₁₋₆ alkyl group or a hydroxy group;
R^{d2} in the number of n2 are each independently a halogen atom;
Z² is an oxygen atom, a sulfur atom or NR^{f2} wherein R^{f2} is a hydrogen atom or a C₁₋₃ alkyl group;
L² is
(1) a linker represented by *-L^{c2}-L^{b2}-L^{a2}-**
wherein
* indicates a binding site to R^{e2},
** indicates a binding site to Z²,
L^{a2} is
(i) a C₁₋₆ alkylene group, or
(ii) -CH₂- (CH₂-O-CH₂)_{q2}-CH₂- wherein q2 is an integer of 0 to 5, L^{b2} is a bond or -CO-, and
L^{c2} is
(i) NR^{g2} wherein R^{g2} is a hydrogen atom or a C₁₋₃ alkyl group,
(ii) a divalent cyclic amino group,
(iii) an oxygen atom, or
(iv) a sulfur atom, or
(2) a linker represented by *- (NH-A^{a2}-CO)ᵣ₂-**
wherein
* indicates a binding site to R^{e2},
** indicates a binding site to Z²,
NH-A^{a2}-CO in the number of r2 are each independently an amino acid residue, and
r2 is an integer of 1 to 3;
R^{e2} is a C₁₋₆ alkyl-carbonyl group; or
the group R^{e2}-L²-Z²- is a hydrogen atom;
L³ is a linker represented by ***- (NH-A^{b2}-CO)ₛ₂-****
wherein
*** indicates a binding site to CO,
**** indicates a binding site to NH,
NH-A^{b2}-CO in the number of s2 are each independently an amino acid residue, and
s2 is an integer of 0 to 3;
R is a hydrogen atom or a C₁₋₃ alkyl group;
p2 is an integer of 0 to 3;
m2 is an integer of 0 to 3; and
n2 is an integer of 0 to 3.

12. The compound or pharmaceutically acceptable salt thereof according to Claim 11, wherein
Z² is an oxygen atom or a sulfur atom, and
L² is a linker represented by *-L^{c2}-L^{b2}-L^{a2}-** wherein each symbol is as defined above.

13. The compound or pharmaceutically acceptable salt thereof according to Claim 11, wherein
Z² is NR^{f2} wherein the symbol is as defined above, and
L² is a linker represented by *-(NH-A^{a2}-CO)ᵣ₂-** wherein each symbol is as defined above.

14. The compound or pharmaceutically acceptable salt thereof according to any one of Claims 11 to 13, wherein p2 is an integer of 1 to 3.

15. The compound or pharmaceutically acceptable salt thereof according to any one of Claims 11 to 14, wherein s2 is 0.

16. A pharmaceutical composition comprising a compound or pharmaceutically acceptable salt thereof as defined in any one of Claims 1 to 15, and a pharmaceutically acceptable carrier.

17. A therapeutic agent for a tumor or cancer expressing fibroblast activation protein α (FAPα), comprising a compound or pharmaceutically acceptable salt thereof as defined in any one of Claims 1 to 15.

18. The therapeutic agent according to Claim 17, wherein the tumor or cancer expressing fibroblast activation protein α is pancreatic cancer, sarcoma, esophageal cancer, lung cancer, breast cancer, prostate cancer, head and neck cancer, ovarian cancer, colon cancer, neuroendocrine tumor, thyroid cancer, uterine cancer or liver cancer.

19. A compound or pharmaceutically acceptable salt thereof as defined in any one of Claims 1 to 15, for use in the treatment of a tumor or cancer expressing fibroblast activation protein α (FAPα).

20. The compound or pharmaceutically acceptable salt thereof according to Claim 19, wherein the tumor or cancer expressing fibroblast activation protein α is pancreatic cancer, sarcoma, esophageal cancer, lung cancer, breast cancer, prostate cancer, head and neck cancer, ovarian cancer, colon cancer, neuroendocrine tumor, thyroid cancer, uterine cancer or liver cancer.

21. A method for treating a tumor or cancer expressing fibroblast activation protein α (FAPα) in a mammal, comprising administering to the mammal a therapeutically effective amount of a compound or pharmaceutically acceptable salt thereof as defined in any one of Claims 1 to 15.

22. The method according to Claim 21, wherein the tumor or cancer expressing fibroblast activation protein α is pancreatic cancer, sarcoma, esophageal cancer, lung cancer, breast cancer, prostate cancer, head and neck cancer, ovarian cancer, colon cancer, neuroendocrine tumor, thyroid cancer, uterine cancer or liver cancer.

23. Use of a compound or pharmaceutically acceptable salt thereof as defined in any one of Claims 1 to 15, for the manufacture of a therapeutic agent for a tumor or cancer expressing fibroblast activation protein α (FAPα).

24. The use according to Claim 23, wherein the tumor or cancer expressing fibroblast activation protein α is pancreatic cancer, sarcoma, esophageal cancer, lung cancer, breast cancer, prostate cancer, head and neck cancer, ovarian cancer, colon cancer, neuroendocrine tumor, thyroid cancer, uterine cancer or liver cancer.

25. A compound represented by Formula (II-1) or a salt thereof: 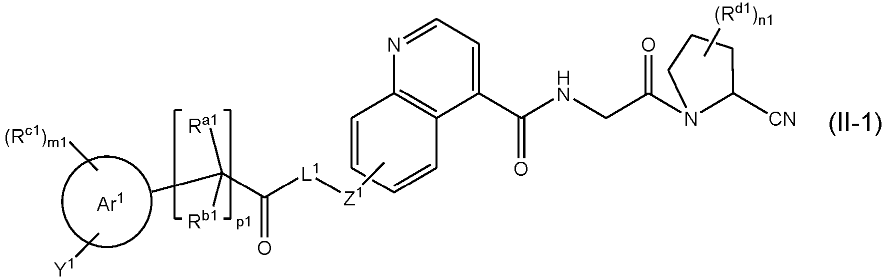 wherein
Y¹ is a boryl group (-B(OH)₂) or its ester group;
Ar¹ is a C₆₋₁₄ aryl group;
R^{a1} and R^{b1} in the number of p1 are each independently a hydrogen atom or a C₂₋₆ alkyl group;
R^{c1} in the number of m1 are each independently a C₁₋₆ alkyl group or a hydroxy group;
R^{d1} in the number of n1 are each independently a halogen atom;
Z¹ is an oxygen atom, a sulfur atom or NR^{f1} wherein R^{f1} is a hydrogen atom or a C₁₋₃ alkyl group;
L¹ is
(1) a linker represented by *-L^{d1}-L^{c1}-L^{b1}-L^{a1}-**
wherein
* indicates a binding site to CO,
** indicates a binding site to Z¹,
L^{a1} is
(i) a C₁₋₆ alkylene group, or
(ii) -CH₂-(CH₂-O-CH₂)_{q1}-CH₂- wherein q1 is an integer of 0 to 5, L^{b1} is a bond or -CO-,
L^{c1} is
(i) NR^{g1} wherein R^{g1} is a hydrogen atom or a C₁₋₃ alkyl group,
(ii) a divalent cyclic amino group,
(iii) an oxygen atom, or
(iv) a sulfur atom, and
L^{d1} is
(i) a bond,
(ii) *- (NH-A^{a1}-CO)ᵣ₁-*** wherein NH-A^{a1}-CO in the number of r1 are each independently an amino acid residue, r1 is an integer of 1 to 3, * indicates a binding site to CO, and *** indicates a binding site to L^{c1}, or
(iii) *-NH-B^{a1}-O-B^{b1}-CO-*** wherein -NH-B^{a1}-O- is a divalent residue derived from an aminosaccharide or a derivative thereof, B^{b1} is a C₁₋₆ alkylene group, * indicates a binding site to CO, and *** indicates a binding site to L^{c1}, or
(2) a linker represented by *- (NH-A^{b1}-CO)ₛ₁-**
wherein
* indicates a binding site to CO,
** indicates a binding site to Z¹,
NH-A^{b1}-CO in the number of s1 are each independently an amino acid residue, and
s1 is an integer of 1 to 3;
p1 is an integer of 1 to 3;
m1 is an integer of 0 to 3; and
n1 is an integer of 0 to 3.

26. A compound radiolabeled represented by Formula (II-2) or a pharmaceutically acceptable salt thereof: 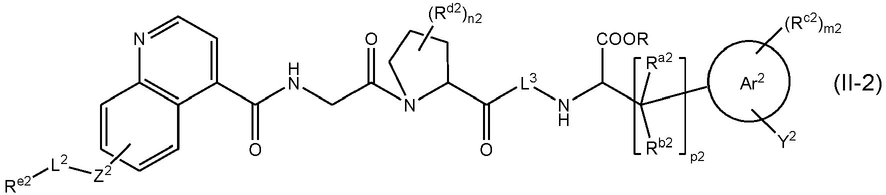 wherein
Y² is a boryl group (-B(OH)₂) or its ester group;
Ar² is a C₆₋₁₄ aryl group;
R^{a2} and R^{b2} in the number of p2 are each independently a hydrogen atom or a C₁₋₆ alkyl group;
R^{c2} in the number of m2 are each independently a C₁₋₆ alkyl group or a hydroxy group;
R^{d2} in the number of n2 are each independently a halogen atom;
Z² is an oxygen atom, a sulfur atom or NR^{f2} wherein R^{f2} is a hydrogen atom or a C₁₋₃ alkyl group;
L² is
(1) a linker represented by *-L^{c2}-L^{b2}-L^{a2}-**
wherein
* indicates a binding site to R^{e2},
** indicates a binding site to Z²,
L^{a2} is
(i) a C₁₋₆ alkylene group, or
(ii) -CH₂-(CH₂-O-CH₂)_{q2}-CH₂- wherein q2 is an integer of 0 to 5, L^{b2} is a bond or -CO-, and
L^{c2} is
(i) NR^{g2} wherein R^{g2} is a hydrogen atom or a C₁₋₃ alkyl group,
(ii) a divalent cyclic amino group,
(iii) an oxygen atom, or
(iv) a sulfur atom, or
(2) a linker represented by *- (NH-A^{a2}-CO)ᵣ₂-**
wherein
* indicates a binding site to R^{e2},
** indicates a binding site to Z²,
NH-A^{a2}-CO in the number of r2 are each independently an amino acid residue, and
r2 is an integer of 1 to 3;
R^{e2} is a C₁₋₆ alkyl-carbonyl group; or
the group R^{e2}-L²-Z²- is a hydrogen atom;
L³ is a linker represented by ***- (NH-A^{b2}-CO)ₛ₂-****
wherein
*** indicates a binding site to CO,
**** indicates a binding site to NH,
NH-A^{b2}-CO in the number of s2 are each independently an amino acid residue, and
s2 is an integer of 0 to 3;
R is a hydrogen atom or a C₁₋₃ alkyl group;
p2 is an integer of 0 to 3;
m2 is an integer of 0 to 3; and
n2 is an integer of 0 to 3.

27. A method for producing a compound radiolabeled represented by Formula (I-1) or a pharmaceutically acceptable salt thereof, comprising the following step; 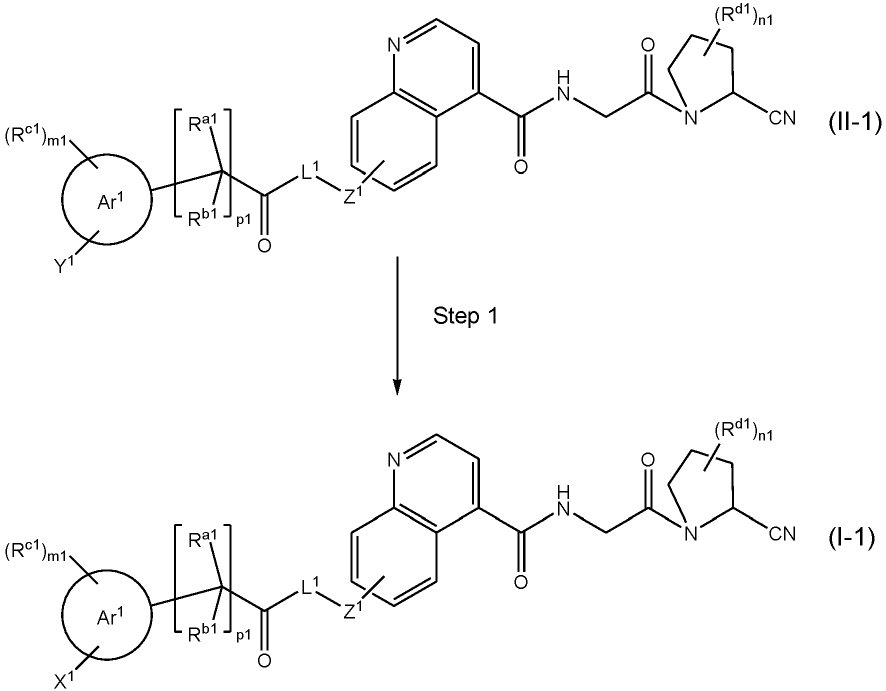 wherein
X¹ is a radionuclide selected from ²¹¹At, ²¹⁰At, ¹³¹I, ¹²⁵I, ¹²⁴I, ¹²³I, ⁷⁷Br and ⁷⁶Br;
Y¹ is a boryl group (-B(OH)₂) or its ester group;
Ar¹ is a C₆₋₁₄ aryl group;
R^{a1} and R^{b1} in the number of p1 are each independently a hydrogen atom or a C₁₋₆ alkyl group;
R^{c1} in the number of m1 are each independently a C₁₋₆ alkyl group or
a hydroxy group;
R^{d1} in the number of n1 are each independently a halogen atom;
Z¹ is an oxygen atom, a sulfur atom or NR^{f1} wherein R^{f1} is a hydrogen atom or a C₁₋₃ alkyl group;
L¹ is
(1) a linker represented by *-L^{d1}-L^{c1}-L^{b1}-L^{a1}-**
wherein
* indicates a binding site to CO,
** indicates a binding site to Z¹,
L^{a1} is
(i) a C₁₋₆ alkylene group, or
(ii) -CH₂- (CH₂-O-CH₂)_{q1}-CH₂- wherein q1 is an integer of 0 to 5, L^{b1} is a bond or -CO-,
L^{c1} is
(i) NR^{g1} wherein R^{g1} is a hydrogen atom or a C₁₋₃ alkyl group,
(ii) a divalent cyclic amino group,
(iii) an oxygen atom, or
(iv) a sulfur atom, and
L^{d1} is
(i) a bond,
(ii) *- (NH-A^{a1}-CO)ᵣ₁-*** wherein NH-A^{a1}-CO in the number of r1 are each independently an amino acid residue, r1 is an integer of 1 to 3, * indicates a binding site to CO, and *** indicates a binding site to L^{c1}, or
(iii) *-NH-B^{a1}-O-B^{b1}-CO-*** wherein -NH-B^{a1}-O- is a divalent residue derived from an aminosaccharide or a derivative thereof, B^{b1} is a C₁₋₆ alkylene group, * indicates a binding site to CO, and *** indicates a binding site to L^{c1}, or
(2) a linker represented by *- (NH-A^{b1}-CO)ₛ₁-**
wherein
* indicates a binding site to CO,
** indicates a binding site to Z¹,
NH-A^{b1}-CO in the number of s1 are each independently an amino acid residue, and
s1 is an integer of 1 to 3;
p1 is an integer of 1 to 3;
m1 is an integer of 0 to 3; and
n1 is an integer of 0 to 3,
Step 1: a step of reacting a compound represented by Formula (II-1) or a salt thereof with a radionuclide selected from ²¹¹At, ²¹⁰At, ¹³¹I, ¹²⁵I, ¹²⁴I, ¹²³I, ⁷⁷Br and ⁷⁶Br in the presence of a reagent selected from an alkali metal iodide, an alkali metal bromide, N-bromosuccinimide, N-chlorosuccinimide, N-iodosuccinimide and
hydrogen peroxide, in water to obtain a compound radiolabeled represented by Formula (I-1) or a pharmaceutically acceptable salt thereof.

28. A method for producing a compound radiolabeled represented by Formula (I-2) or a pharmaceutically acceptable salt thereof, comprising the following step; 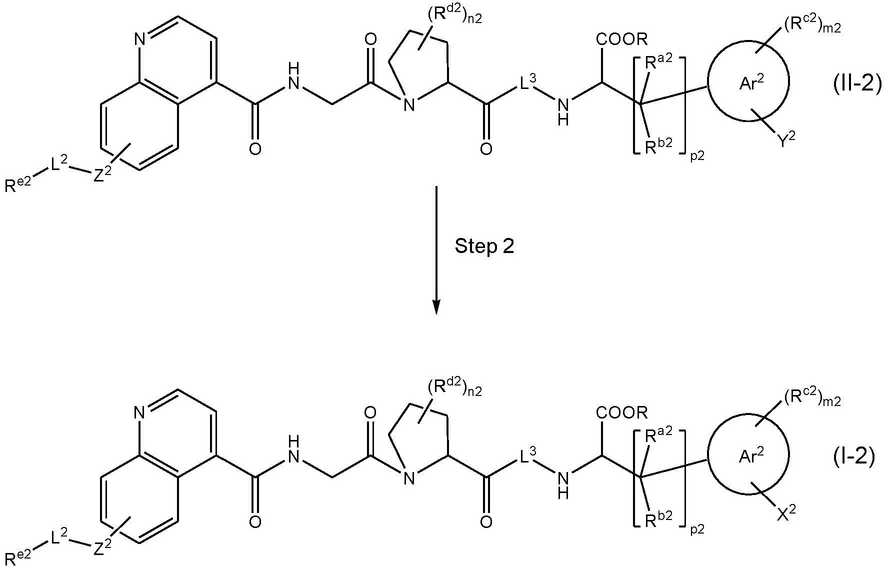 wherein
X² is a radionuclide selected from ²¹¹At, ²¹⁰At, ¹³¹I, ¹²⁵I, ¹²⁴I, ¹²³I, ⁷⁷Br and ⁷⁶Br;
Y² is a boryl group (-B(OH)₂) or its ester group;
Ar² is a C₆₋₁₄ aryl group;
R^{a2} and R^{b2} in the number of p2 are each independently a hydrogen atom or a C₁₋₆ alkyl group;
R^{c2} in the number of m2 are each independently a C₁₋₆ alkyl group or a hydroxy group;
R^{d2} in the number of n2 are each independently a halogen atom;
Z² is an oxygen atom, a sulfur atom or NR^{f2} wherein R^{f2} is a hydrogen atom or a C₁₋₃ alkyl group;
L² is
(1) a linker represented by *-L^{c2}-L^{b2}-L^{a2}-**
wherein
* indicates a binding site to R^{e2},
** indicates a binding site to Z²,
L^{a2} is
(i) a C₁₋₆ alkylene group, or
(ii) -CH₂-(CH₂-O-CH₂)_{q2}-CH₂- wherein q2 is an integer of 0 to 5, L^{b2} is a bond or -CO-, and
L^{c2} is
(i) NR^{g2} wherein R^{g2} is a hydrogen atom or a C₁₋₃ alkyl group,
(ii) a divalent cyclic amino group,
(iii) an oxygen atom, or
(iv) a sulfur atom, or
(2) a linker represented by *- (NH-A^{a2}-CO)ᵣ₂-**
wherein
* indicates a binding site to R^{e2},
** indicates a binding site to Z²,
NH-A^{a2}-CO in the number of r2 are each independently an amino acid residue, and
r2 is an integer of 1 to 3;
R^{e2} is a C₁₋₆ alkyl-carbonyl group; or
the group R^{e2}-L²-Z²- is a hydrogen atom;
L³ is a linker represented by ***- (NH-A^{b2}-CO)ₛ₂-****
wherein
*** indicates a binding site to CO,
**** indicates a binding site to NH,
NH-A^{b2}-CO in the number of s2 are each independently an amino acid residue, and
s2 is an integer of 0 to 3;
R is a hydrogen atom or a C₁₋₃ alkyl group;
p2 is an integer of 0 to 3;
m2 is an integer of 0 to 3; and
n2 is an integer of 0 to 3,
Step 2: a step of reacting a compound represented by Formula (II-2) or a salt thereof with a radionuclide selected from ²¹¹At, ²¹⁰At, ¹³¹I, ¹²⁵I, ¹²⁴I, ¹²³I, ⁷⁷Br and ⁷⁶Br in the presence of a reagent selected from an alkali metal iodide, an alkali metal bromide, N-bromosuccinimide, N-chlorosuccinimide, N-iodosuccinimide and hydrogen peroxide, in water to obtain a compound radiolabeled represented by Formula (I-2) or a pharmaceutically acceptable salt thereof.
